# EUROPEAN PATENT SPECIFICATION

(11) **EP 1 871 391 B1**
(45) Date of publication and mention of the grant of the patent: **28.12.2011**
(21) Application number: 06727501.6
(22) Date of filing: 30.03.2006
(51) Int. Cl.: A61K 31/713, A61K 39/21, A61K 38/16

(54) **ENDOGENOUS RETROVIRUS AND PROTEINS ENCODED BY ENV AS A TARGET FOR CANCER TREATMENT**
ENDOGENER RETROVIRUS UND PROTEINE, KODIERT DURCH ENV-GEN, ALS TARGET FÜR DIE KREBSBEHANDLUNG
RÉTROVIRUS ENDOGÈNE ET PROTÉINES CODÉES PAR UN GÈNE ENV EN TANT QUE CIBLE POUR LE TRAITEMENT DU CANCER

(30) Priority: 30.03.2005 US 666178 P
(43) Date of publication of application: 02.01.2008
(60) Divisional application: 10183796.1
(73) Proprietor: Viroxis, 75017 Paris (FR)
(72) Inventor: MANGENEY, Marianne, 75014 Paris (FR); RENARD, Martial, 75012 Paris (FR); HEIDMANN, Therry, 75017 Paris (FR); POTHLICHET, Julien, 94200 Ivry (FR); DEWANNIEUX, Marie, 56000 Vannes (FR)
(74) Representative: Grosset-Fournier, Chantal Catherine
(86) International application number: PCT/IB2006/000921
(87) International publication number: WO 2006/103562

(56) References cited:
- WO-A-01/16171
- WO-A-01/31021
- WO-A-03/050258
- US-A1- 2004 241 642
- US-B1- 6 770 456
- SCHIAVETTI FRANCESCA ET AL: "A human endogenous retroviral sequence encoding an antigen recognized on melanoma by cytolytic T lymphocytes." CANCER RESEARCH. 1 OCT 2002, vol. 62, no. 19, 1 October 2002 (2002-10-01), pages 5510-5516, XP002394541 ISSN: 0008-5472
- WANG-JOHANNING FENG ET AL: "Quantitation of HERV-K env gene expression and splicing in human breast cancer." ONCOGENE, vol. 22, no. 10, 13 March 2003 (2003-03-13), pages 1528-1535, XP002394542 ISSN: 0950-9232
- MANGENEY M ET AL: "The full-length envelope of an HERV-H human endogenous retrovirus has immunosuppressive properties." THE JOURNAL OF GENERAL VIROLOGY. OCT 2001, vol. 82, no. Pt 10, October 2001 (2001-10), pages 2515-2518, XP002394543 ISSN: 0022-1317
- ARMBRUESTER VIVIENNE ET AL: "Np9 protein of human endogenous retrovirus K interacts with ligand of numb protein X." JOURNAL OF VIROLOGY. OCT 2004, vol. 78, no. 19, October 2004 (2004-10), pages 10310-10319, XP002394544 ISSN: 0022-538X
- SERAFINO A ET AL: "The activation of human endogenous retrovirus K (HERV-K) is implicated in melanoma cell malignant transformation.", EXPERIMENTAL CELL RESEARCH 10 MAR 2009, vol. 315, no. 5, 10 March 2009 (2009-03-10), pages 849-862, ISSN: 1090-2422
- ARMBRUESTER VIVIENNE ET AL: "A novel gene from the human endogenous retrovirus K expressed in transformed cells", CLINICAL CANCER RESEARCH, THE AMERICAN ASSOCIATION FOR CANCER RESEARCH, US, vol. 8, no. 6, 1 June 2002 (2002-06-01), pages 1800-1807, XP002418243, ISSN: 1078-0432

## Description

The present invention is directed to a method for the prevention and/or the treatment of cancer in a mammal, wherein the cancerous cells express at least one protein of endogenous retrovirus, particularly immunosuppressive protein such as *env* gene encoded protein from HERV-K, particularly the Np9 protein, said method comprising inhibiting the expression and/or the activity of said endogenous retrovirus and/or said env gene encoded immunosuppressive protein. The invention also relates to pharmaceutical compositions comprising nucleic acid capable of inhibiting said expression, ligand such as antibodies capable of inhibiting the activity of said env gene encoded protein, or immunogen or vaccinal composition capable of inducing an immune response, cellular or humoral response directed against said env gene encoded protein. The invention is further directed to the use of such env gene encoded protein, mutated or not, for the manufacture of a medicament or a vaccine composition intended for the prevention or the treatment of cancers.

The human genome contains a large fraction (ca. 8%) of elements of retroviral origin, with thousands of sequences close to the integrated proviral form of infectious retroviruses, with two long terminal repeats (LTR) bordering internal regions homologous to the gag, pol and env genes (Lower et al. (1996) Proc. Natl. Acad. Sci. USA 93, 5177-5184; Boeke et al. (1997) in Retroviruses, eds. Coffin, J. M., Hughes, S. H. & Varmus, H. E. (Cold Spring Harbor Laboratory Press, New York), pp. 343-436; Lander et al. (2001) Nature 409, 860-921). These elements, named Human Endogenous RetroViruses (HERV), are most probably the proviral remnants of ancestral germline infections by active retroviruses, which have thereafter been transmitted in a mendelian manner. HERV can be grouped according to sequence homologies into approximately 100 distinct families, each containing a few to several hundreds elements.

Strong similarities between HERV and present-day retroviruses can be inferred from phylogenetic analyses on the Reverse Transcriptase domain of the pol gene or the TransMembrane moiety (TM) of the env gene, which disclose interspersion of both classes of elements and suggest a common history and shared ancestors (Tristem, M. (2000) J. Virol. 74, 3715-3730; Benit et al. (2001) J. Virol. 75 (11709-11719). Similarities are also observed at the functional level.

As a consequence of the close relationship between HERVs and infectious retroviruses, and despite the fact that most HERVs have accumulated mutations, deletions and/or truncations, it remains plausible that some elements still possess functions of infectious retroviruses, that may have been diverted by the host to its benefit. Along this line, it has been proposed (reviewed in ref. Venables et al.(1995) Virology 211, 589-592, Harris, J. R. (1998) BioEssays 20, 307-316) that the HERV envelopes could play a role in several processes including i) protection against infection by present-day retroviruses through receptor interference (Best et al. (1997) Trends Microbiol. 5, 313-318), ii) protection of the fetus against the maternal immune system via an immunosuppressive domain located in the envelope TM subunit (Cianciolo et al. (1985) Science 230, 453-455; Mangeney et al. (1998) Proc. Natl. Acad. Sci. USA 95, 14920-14925), and iii) placenta morphogenesis through fusogenic effects allowing differentiation of cytotrophoblast cells into the syncytiotrophoblast (Blond et al. (2000) J. Virol. 74, 3321-3329; Mi et al. (2000) Nature 403, 785-788).

Thus, the envelope protein of a HERV-W family member, Syncytin, has retained the ability to interact with the receptor of the D-type retroviruses (Blond et al. (2000) J. Virol. 74, 3321-3329; Lavillette et al. (2002) J. Virol. 76, 6442-6452) and can confer infectivity on pseudotyped retroviral particles (Lavillette et al. (2002); An et al. (2001) J. Virol. 75, 3488-3489).

In accordance with a symbiotic role for HERVs, WO 01/16171 discloses that the HERV- W envelope gene product (Syncytin) is a highly fusogenic glycoprotein which is specifically expressed in the placenta and can mediate cell-cell fusion ex vivo. This HERV-W envelope could be used in cancer treatment by promoting the formation a syncytia by cancerous cells, thus leading to their death. Disorders in the expression of the HERV-W envelope could impair placental morphogenesis.

WO 2004/087748 discloses that the HERV- FRD envelope gene product (named Syncytin 2) is also a fusogenic glycoprotein which is essentially expressed in the placenta and can mediate cell-cell fusion *ex vivo.* It also discloses that this HERV-FRD envelope is immunosuppressive and may be used for the immunosuppressive treatment of patients, such as patients afflicted with allergy, autoimmune disease or graft rejection.

HERVs thus appear to contain still active genes, which most probably have been subverted by the host for its benefit and should be considered as bona fide human genes. Some of their characteristic features and possible physiological roles, as well as potential pathological effects inherited from their retroviral ancestors have been also reviewed (de Parseval et al., Cytogenet Genome Res. 2005;110(1-4):318-32).

Nevertheless, a comprehensive analysis of such genes has to be performed, in order to assess their possible physiopathological role in human diseases including autoimmune diseases or cancer diseases. Indeed, recent studies demonstrating the expression of HERV mRNAs in cancer cells (Wang-Johanning et al., Cancer, 2003, 98(1), 187-197; Yi et al., Journal of General Virology, 2004, 85, 1203-1210; Armbruester et al., Clinical Cancer Research, 2002, 8, 1800-1807, J. Virol. 2004 Oct;78(19):10310-10319), suggest that these HERV encoded proteins may possess a function in tumorigenesis and be used as markers.

Because there currently is a need for new methods in the fight against cancer, it would therefore be beneficial to provide specific methods.

The inventors have demonstrated that tumors have the capacity to escape immune surveillance and are able to overwhelm the immune system by expressing the envelope of an ERV, the blocking of ERV expression resulting in enhanced tumor rejection and the expression of the sole ERV envelope protein partially restoring the initial extent of tumor progression. They have also demonstrated that the expression of env gene encoded protein of HERV-K such as Np9 protein induces down regulation of MHC class I antigens, a characteristic feature of tumor cells allowing them to escape immune surveillance.

So, targeting such genes by any available mean will contribute to tumor regression by restoring the natural ability of the immune system to reject tumor cells, and therefore contribute to cancer treatment.

This is the object of the present invention. The present invention provides the use of a composition comprising:
- a env gene endogenous retrovirus encoded immunosuppressive protein, mutated or not, or a fragment thereof comprising an immunosuppressive domain of said env gene endogenous retrovirus encoded protein, or
- a nucleic acid encoding said env gene endogenous retrovirus encoded protein, or fragment of said env gene endogenous retrovirus encoded protein, or
- a ligand such as antibodies, said ligand being capable of specifically binding to said env gene encoded immunosuppressive protein, or
- a double stranded RNA, siRNA, shRNA, miRNA or an antisense nucleic acid targeting nucleic acid sequences of the endogenous retrovirus and/or of a protein expressed by said endogenous retrovirus,
said composition
- inhibiting the expression of said endogenous retrovirus and/or
- inhibiting the expression, and/or lowering the concentration, and/or inhibiting the activity of said expressed protein, and/or
- stimulating an immune response against said env gene endogenous retrovirus encoded protein
for the preparation of a drug intended for the prevention and/or the treatment of cancer in a mammal, wherein the cancerous cells of said mammal express said env gene endogenous retrovirus encoded protein.

All the cancers induced by the expression of endogenous retrovirus or endogenous protein thereof can be prevent and/or treated by the method of the present invention by inhibiting the expression of said endogenous retrovirus and/or by inhibiting the expression, and/or lowering the concentration, and/or inhibiting the activity of said protein expressed by the cancererous cells. Among these cancers, melanoma, neuroblastoma, germ-cell tumors, breast tumors and leukemia are preferred, melanoma and neuroblastoma being the more preferred.

In one embodiment, the method according to the present invention is characterized in that the protein of endogenous retrovirus is immunosuppressive, an env gene encoded protein of endogenous retrovirus is particularly preferred. Among these env gene encoded proteins of endogenous retrovirus:
- the ENV proteins or analog thereof; or
- the proteins which are encoded by the env gene but which are not ENV protein (envelope protein), such as Np9 protein of HERV-K or analog thereof,
are particularly preferred.

By "ENV analog", it is intended to designate a protein having at least 60 %, preferably 70 %, 75 %, 80 %, 85 %, 90 %, 95 %, 98 %, 99 %, or higher identity with an endogenous retrovirus ENV protein, preferably HERV. More preferably, this ENV analog protein is immunosuppressive and/or capable of inducing cancerous cell proliferation when express in these cells.

By "Np9 analog", it is intended to designate a protein having at least 60 %, preferably 70 %, 75 %, 80 %, 85 %, 90 %, 95 %, 98 %, 99 %, or higher identity with a Np9 protein having the sequence SEQ ID NO: 3 or a Np9 amino acid sequence as depicted in Genbank accession Number P61580, P61581, P61582 and P61583. More preferably, this Np9 analog protein is immunosuppressive and/or capable of inducing cancerous cell proliferation when express in these cells.

In a preferred embodiment, the invention relates to the use according to the present invention is wherein said endogenous retrovirus is a human endogenous retrovirus (HERV),
selected from the group of HERV consisting of HERV-K, HERV-FRD, HERV-H, HERV-T, HERV-R,HERV-V, HERV-P(b), HERV-E, HERV-F(c)1, HERV-F(c)2, and HERV-R(b).

The sequence of these HERV can be found in the documents Blaise et al. (Proc. Natl. Acad. Sci. USA., 2003, 100(22):13013-8), de Parseval et al. (J. Virol. 2003 Oct;77(19):10414-22), Blaise et al. (Retrovirology 2005, 2(1):19); de Parseval et al. (Cytogenet Genome Res. 2005, 110(1-4):318-32 (Review)).

In a more preferred embodiment, the human endogenous retrovirus is a HERV-K.

In another more preferred embodiment, the human endogenous retrovirus is a HERV-K sub-type characterized in that the open reading frame encoding the env viral protein encodes a Np9 protein, preferably selected from the group consisting of the HERV-K sub-type HERV-K101, HERV-K102, HERV-K107 and HERV-K(II).

In another more preferred embodiment, said Np9 protein has an amino acid sequence selected from the group consisting of:
- a) the amino acid sequence SEQ ID NO: 3 or of a HERV-K sub-type Np9 protein as depicted in Genbank (GenPept) under the Accession Number P61580, P61581, P61582 and P61583; and
- b) an amino acid sequence having at least 75 % identity with an amino acid sequence of a) and having an immunosuppressive activity.

The terms "identical" or percent "identity," in the context of two or more polypeptide sequences, refer to two or more sequences or subsequences that are the same or have a specified percentage of amino acid residues that are the same (i.e., about 75 % identity, preferably 80 %, 85 %, 90 %, 95 %, 98 %, 99 %, or higher identity over a specified region when compared and aligned for maximum correspondence over a comparison window or designated region) as measured using a BLAST or BLAST 2.0 sequence comparison algorithms with default parameters, or by manual alignment and visual inspection (see, e.g., NCBI web site). The definition also includes sequences that have deletions and/or additions, as well as those that have substitutions. As described below, the preferred algorithms can account for gaps and the like. Preferably, identity exists over a region that is at least about 25 amino acids in length, or more preferably over a region that is 25-75 amino acids in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

Methods of alignment of sequences for comparison are well-known in the art. A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990).

In a particular aspect, the use according to the present invention is characterized in that the expression of the endogenous retrovirus and/or of the endogenous retrovirus immunosuppressive protein, is inhibited by a nucleic acid, such as a double stranded RNA, a siRNA (small interfering RNA), a shRNA (short RNA), miRNA (microRNA) or an antisense nucleic acid targeting nucleic acid sequences of said endogenous retrovirus and/or of said endogenous retrovirus immunosuppressive protein.

"Nucleic acid" refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Said nucleic acid, such as a double stranded RNA, siRNA, shRNA, miRNA or an antisense nucleic acid refers to a nucleic acid that has the ability to reduce or inhibit expression (transcription and/or translation) of a target nucleic acid sequences of said endogenous retrovirus and/or of said protein of endogenous retrovirus when said nucleic acid is introduced or expressed in the same cell as the target nucleic acid. Said nucleic acid has substantial or complete identity to the complementary sequence of the target gene and can so hybridizes with it. Typically, said nucleic acid s is at least about 15-50 nucleotides in length, preferably about 20-25 nucleotides in length.

By antisense nucleic acid molecules it is intended to designate molecules which are complementary to the sense targeting nucleic acid or to the sense nucleic encoding the target protein such as the target env gene encoded protein, e.g., complementary to the coding strand of a double stranded cDNA molecule or complementary to an mRNA sequence. Accordingly, an antisense nucleic acid can hydrogen bond to a sense nucleic acid. The antisense nucleic acid can be complementary to an entire protein coding strand, or to only a portion thereof, e.g., all or part of the protein coding region (or open reading frame). An antisense nucleic acid molecule can be antisense to a non-coding region of the coding strand of a nucleotide sequence encoding the target protein. An antisense oligonucleotide can be, for example, about 5, 10, 15, 20, 25, 30, 35, 40, 45 or 50 nucleotides in length. A siRNA, shRNA, miRNA or antisense nucleic acid in the method or in the use of the present the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives. Alternatively, the nucleic acid can be produced biologically using an expression vector into which a nucleic acid has been subcloned.

The nucleic acid molecules in the method or use of the present invention are typically administered to a subject or generated *in situ* such that they hybridize with or bind to cellular target mRNA and/or genomic DNA encoding the target protein to thereby inhibit expression of the protein, e.g., by inhibiting transcription and/or translation. An example of a route of administration of nucleic acid molecules for the present invention includes direct injection at a tissue site. Alternatively, nucleic acid molecules can be modified to target selected cells and then administered systemically. For example, for systemic administration, antisense molecules can be modified such that they specifically bind to receptors or antigens expressed on a selected cell surface, e.g., by linking the nucleic acid molecules to peptides or antibodies which bind to cell surface receptors or antigens. The nucleic acid molecules can also be delivered to cells using vectors well known by the skilled person.

In embodiments, the nucleic acid molecules used in the present invention can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic ("PNAs").

In another embodiment, the nucleic acids used in the present invention can be modified to enhance their cellular uptake, by attaching lipophilic or other helper groups or by the use of liposomes or other techniques of drug delivery known in the art.

In one embodiment, the method according to the present invention is characterized in that said nucleic acid, such as a the double stranded RNA, the siRNA, the shRNA, miRNA or the antisense nucleic acid are administered to said mammal.

In another embodiment, the method according to the present invention is characterized in that vectors, in particular viral vectors, which encode said nucleic acid such as the double stranded RNA, the siRNA, the shRNA, miRNA or the antisense nucleic acid are administered to said mammal.

In another aspect, the method according to the present invention is characterized in that the activity of the protein of endogenous retrovirus is inhibited by ligands of said protein of endogenous retrovirus.

In a preferred embodiment, the method according to the present invention is characterized in that the protein of endogenous retrovirus is a env gene encoded protein and said ligands is capable of specifically binding to said env gene encoded protein, particularly to the immunosuppressive domain of said env gene encoded protein.

Advantageously, the ligands are antibodies, in particular monoclonal antibodies, antibody fragments, such as Fab or F(ab)'₂ fragments, scFv polypeptides, chimeric antibodies, humanized antibodies, aptamers or binding peptides.

Useful antibodies include antibodies which bind to a domain or subdomain of the target protein such as said env gene encoded protein described above (e.g., the immunosuppressive domain).

Preferred epitopes encompassed by the antigenic peptide for the preparation of the antibodies are regions of the immunosuppressive domain of the env gene encoded protein, preferably mutated immunosuppressive domain which are able to induce antibodies capable of recognizing the wild type immunosuppressive region.

Typically, an immunogen comprising an env gene encoded protein, mutated or not, is used to prepare antibodies by immunizing a suitable subject, (e.g., rabbit, goat, mouse or other mammal) with the immunogen. An appropriate immunogenic preparation can contain, for example, recombinantly expressed env gene encoded protein (or chemically synthesized). The preparation can further include an adjuvant, such as Freund's complete or incomplete adjuvant, or similar immunostimulatory agent. Immunization of a suitable subject with an immunogenic env gene encoded protein preparation induces a polyclonal antibody response. Examples of immunologically active portions of immunoglobulin molecules include F(ab) and F(ab')2 fragments which can be generated by treating the antibody with an enzyme such as pepsin.

In the method of the present invention monoclonal antibodies that bind said env gene encoded protein can be used. Additionally, recombinant antibodies, such as chimeric and humanized monoclonal antibodies, comprising both human and non-human portions, which can be made using standard recombinant DNA techniques, are within the scope of the method or the use of the present invention. Such chimeric and humanized monoclonal antibodies can be produced by recombinant DNA techniques known in the art.

In another aspect, the use according to the present invention is characterized in that the protein of endogenous retrovirus as defined above, preferably an env gene encoded protein, is immunosuppressive and the activity of said protein of endogenous retrovirus is :
- said immunosuppressive endogenous retrovirus protein, or a fragment thereof comprising its immunosuppressive domain, or a nucleic acid encoding said protein or encoding said fragment thereof, or
- a mutated form of said immunosuppressive endogenous retrovirus protein lacking immunosuppressive activity, or fragments thereof comprising a mutated immunosuppressive domain of said immunosuppressive endogenous retrovirus protein, or a nucleic acid encoding said protein or encoding said fragment thereof.

In another aspect, the use according to the present invention is characterized in that the protein of endogenous retrovirus, preferably an env gene encoded protein, is immunosuppressive and the activity of said protein of endogenous retrovirus, which results in an immunosurveillance escape inducing tumor cell proliferation, is inhibited by a cellular and/or humoral immune response to the administration to the mammal:
- of an endogenous retrovirus protein (such as the Gag, Pol, Pro, Env, Rec or Np9 protein encoded by said ERV), or a fragment thereof, preferably an env gene encoded immunosuppressive protein or a fragment thereof, said fragment comprising its immunosuppressive domain, or a nucleic acid encoding said endogenous retrovirus protein or a fragment thereof; or
- of a mutated form of said immunosuppressive endogenous retrovirus protein lacking immunosuppressive activity, preferably an env gene encoded protein, such as ENV or Np9 analog, or a fragment thereof comprising a mutated immunosuppressive domain of said immunosuppressive endogenous retrovirus protein, or a nucleic acid encoding said protein or encoding a fragment thereof.

The immunosuppressive effects and the immunosuppressive domain of protein of endogenous retrovirus, such as env gene encoded protein, can be for example determined by using the method disclosed in the document WO 2005/095442 (Example 1, Methods) and in Example 3 of the present application.

The strategy for the identification of mutated ENV protein, with decreased or lacked immunosuppressive activity, can be for example the stategy used in in the document WO 2005/095442 (by site-directed mutagenesis and study of the immunosuppressive effect of the mutated protein obtained compared to the wild type protein) (see Example 1, Methods and paragraphs 3 and 4 of the document WO 2005/095442).

For the env gene encoded Np9 type protein, the epitope mapping type method wherein certain parts of the full length Np9 protein are tested for their ability to induce down regulation of MHC class I antigens (as shown in Example 3) can be used.

The identification of possible deletion or substitution of the env gene encoded Np9 type protein in order to determine mutated Np9 type protein having decreased or lacked immunosuppressive activity and conserved epitopic determinant capable of eliciting antibodies specifically recognizing the wild type Np9 protein, can be carried out by using the site-directed mutagenesis associated to the study of the lack of down regulation of MHC class I antigens effect compared to the wild type protein.

In a preferred embodiment, the present invention relates to a method, wherein the activity of said protein of endogenous retrovirus is inhibited by a cellular immune response cellular and/or a humoral (antibodies) immune response elicited in response to the administration to the mammal, according to the invention, characterized in that the protein of endogenous retrovirus is an env gene encoded protein, such as an ENV protein or a Np9 protein.

In said method according to the invention, the use of a mutated form of an env gene encoded protein, such as an ENV protein or a Np9 protein, deprived of immunosuppressive activity, or a nucleic acid encoding said protein, is more preferred.

In this above method using a mutated form of an env gene encoded protein, is more preferred a mutated form of an env gene HERV, preferably HERV-K, encoded protein deprived of immunosuppressive activity and wherein said mutated form is capable of eliciting an immune response, cellular and/or humoral (antibodies) immune response, specifically recognizing the non mutated form of said env gene HERV encoded protein.

In this above method using of a mutated form of an env gene encoded protein, is also more preferred a mutated form of HERV-K Np9 protein.

In said method according to the invention, the use of a mutated form of HERV-K Np9 deprived of immunosuppressive activity is particularly preferred when the non mutated form of Np9 protein has an amino acid sequence selected from the group consisting of:
- a) the amino acid sequence of HERV-K sub-type Np9 protein as depicted in Genbank (GenPept) under the Accession Number, P61580, P61581, P61582 and P61583; and
- b) an amino acid sequence having at least 75 % identity with an amino acid sequence of a) and having an immunosuppressive activity.

The present invention is also directed to **the use** according to the present invention, wherein the protein of endogenous retrovirus is immunosuppressive and fragments of said protein of endogenous retrovirus, which comprise an immunosuppressive domain, mutated or not, or a or nucleic acid encoding said protein or encoding said fragment thereof, are administered to said mammal to antagonize the interaction of said protein of endogenous retrovirus with its ligands in the organism of said mammal.

Is preferred for said method when the endogenous retrovirus is a HERV ENV protein or a HERV-K Np9 protein.

In another aspect, the present invention encompasses a pharmaceutical composition comprising as active substance a nucleic acid, such as a double stranded RNA, siRNA, shRNA, miRNA or antisense nucleic acid targeting nucleic acid sequences of an endogenous retrovirus and/or of **an immunosuppressive** protein expressed by an endogenous retrovirus (such as the Gag, Pol, Pro, Env, Rec or Np9 protein encoded by said ERV) in association with a pharmaceutically acceptable vehicle said endogenous retrovirus is a human endogenous retrovirus (HERV), selected from the group of HERV consisting of HERV-K, HERV-FRD, HERV-H, HERV-T, HERV-R, HERV-V, HERV-P(b), HERV-E, HERV-F(c)1, HERV-F(c)2 and HERV-R(b).

Vector DNA or RNA or an isolated nucleic acid molecule used in the present invention can be introduced *in vivo* into the target cell via conventional techniques or routes of administering.

Preferably, the pharmaceutical composition according to the present invention is characterized in that said endogenous retrovirus is HERV-K.

Preferably, the pharmaceutical composition according to the present invention is characterized in that said expressed protein is an env gene HERV encoded protein such as an ENV protein or a Np9 protein, preferably an env gene HERV encoded protein which is immunosuppressive.

In another aspect, the present invention is directed to a pharmaceutical composition comprising as active substance an endogenous retrovirus protein, such as the Gag, Pol, Pro, Env, Rec or Np9 protein encoded by said ERV), or a fragment thereof said endogenous retrovirus is a human endogenous retrovirus (HERV), selected from the group of HERV consisting of HERV-K, HERV-FRD, HERV-H, HERV-T, HERV-R, HERV-V, HERV-P(b), HERV-E, HERV-F(c)1, HERV-F(c)2, and HERV-R(b).

By fragment thereof, it is intended here to designate particularly, the fragment of at least 10, 12, 15, 20, 25, 30, 35, 40, 50, 75, or 100 amino acids capable, when administering to the host animal or human, to induce a cellular or humoral immune response resulting in the restoration of the immunosurveillance and/or in the decrease or the stop of the proliferation of the cancerous cells.

In a preferred embodiment, the present invention is directed to a pharmaceutical composition comprising as active substance an immunosuppressive env gene HERV encoded protein, mutated or not, or fragment thereof comprising the immunosuppressive domain of said env gene HERV encoded protein, or a nucleic acid encoding said protein or encoding a fragment thereof, in association with a pharmaceutically acceptable vehicle.

By mutated endogenous retrovirus protein, or mutated fragment thereof, it is intended to designate a protein, or fragment thereof which when compared to the wild type protein, or fragment thereof, contains at least one mutation point (deletion, insertion or modification of one amino acid residue), preferably at least 2, 3, 4, 5, 10, or 20 mutation points and at most 35 % mutation, preferably 30 %, 25 %, 20 %, 15 %, 10%, 9%, 8%, 7%, 6%, 5%, 4%, 3%, 2% and 1 % mutation.

In an also preferred embodiment, the pharmaceutical compositions according to the present invention comprising as active substance an endogenous retrovirus HERV-K protein, more preferably a HERV-K sub-type env protein characterized in that the open reading frame encoding the env HERV-K protein encodes a Np9 protein, or a nucleic acid encoding said endogenous retrovirus HERV-K protein.

Advantageously, the HERV-K is a HERV-K sub-type selected from the group consisting of the HERV-K sub-type HERV-K101, HERV-K102, HERV-K107 and HERV-K(II).

More preferred are also the pharmaceutical compositions according to the present invention is characterized in that the protein is a Np9 protein having an amino acid sequence selected from the group consisting of:
- a) the amino acid sequence of HERV-K sub-type Np9 protein as depicted in Genbank (GenPept) under the Accession Number, P61580, P61581, P61582 and P61583; and
- b) an amino acid sequence having at least 75 % identity with an amino acid sequence of a) and having an immunosuppressive activity.

In another aspect, the present invention relates to a pharmaceutical composition according to the present invention, characterized in that said composition further comprises at least another or preferably at least 2, 3 or all the proteins encoded by said HERV provirus, or a nucleic sequence encoding said other(s) protein(s) encoded by said HERV provirus, in order to enhance the immune response against cells expressing proteins of this HERV provirus.

Is preferred, a pharmaceutical composition according to the present invention, characterized in that said composition further comprises at least another or preferably at least 2, 3 or all the proteins encoded by the nucleic sequence of the consensus HERV-K(HML2) provirus ("Phoenix") having the sequence SEQ ID NO: 4, or a nucleic sequence encoding said other(s) protein(s) encoded by said nucleic sequence having the sequence SEQ ID NO: 4.

In another aspect, the present invention relates to active substance, such as env gene encoded protein, mutated or not, or nucleic acid encoding said active protein substances, as defined in the pharmaceutical compositions of the present invention, as an immunogen or as a vaccine.

In another aspect, the present invention relates to composition for stimulating an immune response, cellular and/or humoral immune response, against env gene HERV encoded protein, said composition comprising an active substance such as env gene encoded protein, mutated or not, or an immunogenic fragment thereof, as defined in the pharmaceutical compositions according to the present invention, or a nucleic acid encoding these active protein substances.

In another aspect, the present invention encompasses the use of an active substance, such as env gene encoded protein, mutated or not, or an immunogenic fragment thereof, as defined in the pharmaceutical compositions of the present invention for the manufacture of an anti-cancer vaccine.

In another aspect, the present invention relates to the use of an active substance as defined in the pharmaceutical compositions of the present invention for the manufacture of a medicament intended for the prevention or the treatment of cancers.

All the cancers induced by the expression of endogenous retrovirus or endogenous protein thereof can be prevent and/or treated by the pharmaceutical compositions or the vaccines of the present invention. Among these cancers, melanoma, neuroblastoma, germ-cell tumors, breast tumors and leukemia are preferred, melanoma and neuroblastoma being the more preferred.

It can be especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the pharmaceutical composition of the present invention are dictated by and directly dependent on the unique characteristics of the active compound and the particular therapeutic effect to be achieved, and the limitations inherent in the art of compounding such an active compound for the treatment of individuals.

For antibodies or env gene encoded protein as an immunogen, mutated or not, the preferred dosage is 0.1 mg/kg to 100 mg/kg of body weight (generally 10 mg/kg to 20 mg/kg). lower dosages and less frequent administration are often possible. Modifications such as lipidation can be used to stabilize antibodies or the ENV protein and to enhance uptake and tissue penetration.

The nucleic acid molecules used in the method of prevention and/or treatment or in the pharmaceutical compositions of the invention can be inserted into vectors and used as therapeutical and/or vaccinal vectors. Therapeutical and/or vaccinal vectors can be delivered to a subject by, for example, intravenous injection, local administration. The pharmaceutical preparation of the therapeutical and/or vaccinal vector can include the therapeutical and/or vaccinal vector in an acceptable diluent, or can comprise a slow release matrix in which the nucleic acid delivery vehicle is imbedded. Alternatively, the complete nucleic acid delivery vector, e.g. retroviral vector, can be produced intact from recombinant cells.

Pharmaceutically acceptable vehicle is understood to mean a compound or a combination of compounds entering a pharmaceutical or vaccinal composition not causing secondary reactions and which enables for example ease of administration of active compound, an increase in its life expectancy and/or its efficacy in the organism, augmentation of its solubility in solution or again an improvement in its preservation. These pharmaceutically acceptable vehicles are well known and will be adapted by the specialist as a function of the nature and mode of administration of the selected active compound.

As for vaccinal formulations, these can comprise adjuvants of appropriate immunity which are known to the specialist, such as for example aluminum hydroxide, a representative of the family of muramyl peptides as one of the peptidic derivatives of N-acetyl-muramyl, a bacterial lysate, or even the incomplete Freund adjuvant.

Preferably, the immunogenic or vaccinal composition according to the present invention will be administered systemically, in particular intravenously, intramuscularly, intradermally or subcutaneously, or orally. In a more preferred way, the vaccinal composition of the invention comprising protein or nucleic acid will be administered in several doses, spread out over time.

In another aspect, the present invention comprises the full length nucleic acid of the consensus HERV-K(HML2) provirus (named "Phoenix" provirus) having the sequence SEQ ID NO: 4 and their fragment such as the ORF encoding the HERV-K(HML2) proteins (Gag, Pro, Pol, Env and the accessory Rec protein).

The invention also comprises the use of said full length nucleic acid of the consensus HERV-K(HML2) provirus, or fragment thereof such as the ORF encoding the HERV-K(HML2) proteins (Gag, Pro, Pol, Env and the accessory Rec protein), or at least one of the protein encoded by this consensus provirus nucleic acid sequence, as a medicament, preferably as an immunogen or as a vaccine, more preferably for the prevention or the treatment of cancer.

The invention also comprises the use of said full length nucleic acid of the consensus HERV-K(HML2) provirus, or ORF fragments thereof as or in an expression vector or a gene delivery vector, preferably for the expression in a host cell of an animal or a human of at least one, preferably 2, 3 or all the proteins encoded by this consensus provirus, preferably for the prevention or the treatment of cancer, more preferably for cancer induced by the activation of the transcription of env gene HERV-K in said animal or human.

The invention also comprises the use of specific fragment of said full length nucleic acid of the consensus HERV-K(HML2) provirus as primer or probe for the specific amplification or identification of nucleic acid present in a biological sample.

The present invention further comprises the protein Gag (SEQ ID NO: 5), Pro (SEQ ID NO: 6), Pol (SEQ ID NO: 7), Env (SEQ ID NO: 8) and the accessory Rec (SEQ ID NO: 9) protein encoded by said full length nucleic acid of the consensus HERV-K(HML2) provirus of the invention and their use for the preparation of vaccinal antigens in a composition intended to the prevention or the treatment of cancer.

Finally, the present invention is directed to an *in vivo* method for the determination of the presence of infectious HERV-K endogenous retrovirus particles in a biological fluid sample characterized in that said method comprises the step of:
a) incubating the biological fluid in presence of animal cells which do not naturally contains ERV- K in conditions suitable for infecting said animal cells with the infectious HERV-K endogenous retrovirus particles suspected to be present in the sample;
b) recovering the genomic DNA of the animal cells;
c) determining the presence of integrated HERV-K proviruses by a nucleic acid amplification method, such as PCR method, or analog thereof, using the following pair of primers:
   5'-GCCTTTCCCGCCCTTAATT-3' and 5'-GCAGGGCAGCGATCATCTA-3'.

The method according to the present invention, wherein in step c), the determination is carried out by a Taqman analysis method wherein the Taqman probe is a probe having the sequence 6FAM-ATAGTGTATGGGTACCTGGC-MGBNFQ.

The method according to the present invention, wherein in step a, the animal cells are hamster BHK21 cells.

The method according to the present invention, wherein in step a), the biological fluid is from human tissue, particularly from human cancerous tissue.

Other features and advantages of the invention will be apparent from the following legends of the figures and the examples.

### Legends of the figures

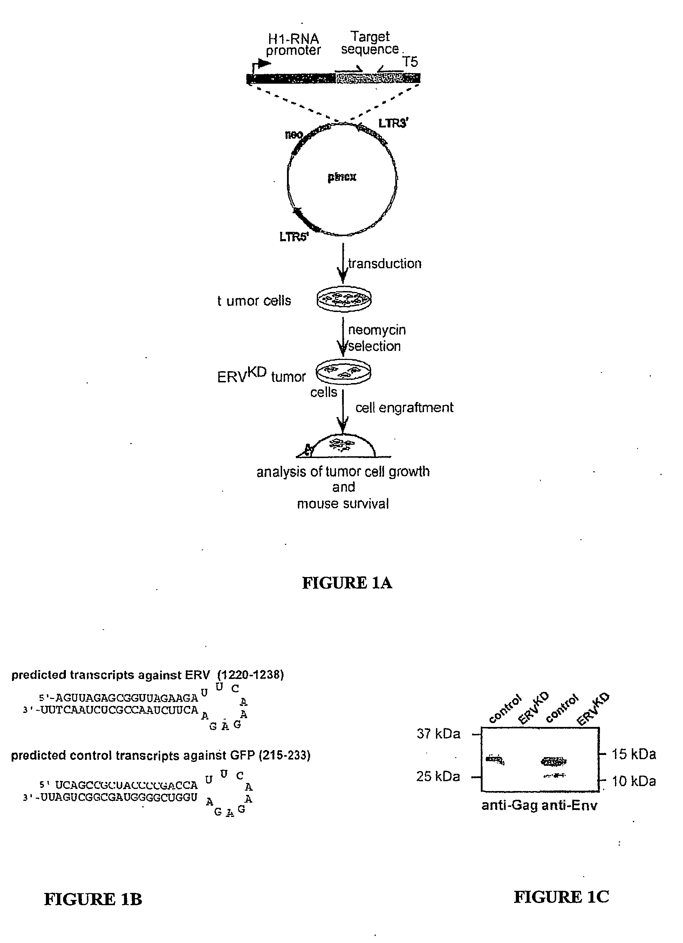

**Figures 1A, 1B and 1C****:** Knockdown procedure and rationale of the assay
Figure 1A: To knock down ERV expression, we constructed a plncx-derived vector making use of the pSUPER vector to generate, under control of the H1-RNA promoter, short double-stranded transcripts for RNA interference. B16 cells were transduced with these expression vectors, submitted to G418 selection, and the resulting ERV^{KD} and control B16 cells were injected subcutaneously into the flank of the mice, whose tumor growth was monitored.
Figure 1B: Predicted structure of the dsRNA generated by the ERV and control (GFP) vectors; numbers refer to nt positions within the respective targeted sequences (see Methods).
Figure 1C: Western blot analysis of Gag and Env expression in the supernatant of ERV-knocked down (ERV^{KD}) and control cells.
**Figures 2A and 2B****:** Knocked down cells have conserved a transformed phenotype

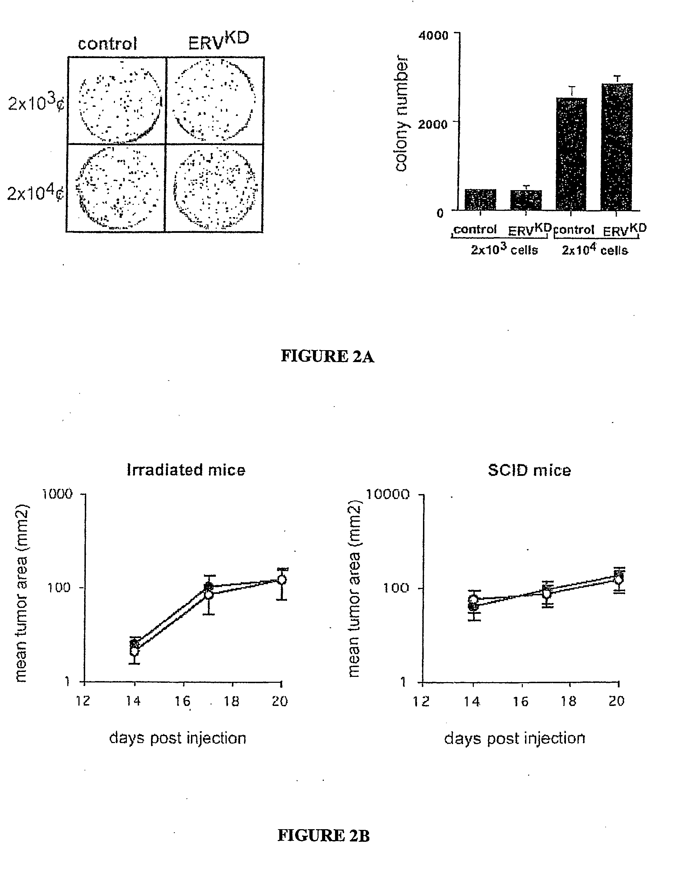

Figure 2A: *In vitro* analysis of the transformed phenotype using soft agar assay. ERV^{KD} and control B16 cells (2x10³ or 2x10⁴) were plated onto a semi-solid layer for 4 weeks, and then colonies were counted.
Figure 2B: Assay for the transformed phenotype *in vivo* using immuno-incompetent mice. ERV^{KD} and control B16 cells (2x10⁵) were injected subcutaneously into the flank of either X-irradiated (5 Gy) C57B1/6 or SCID mice (2-5 independent experiments with 5 mice per group) and tumor growth was determined by measuring tumor area.

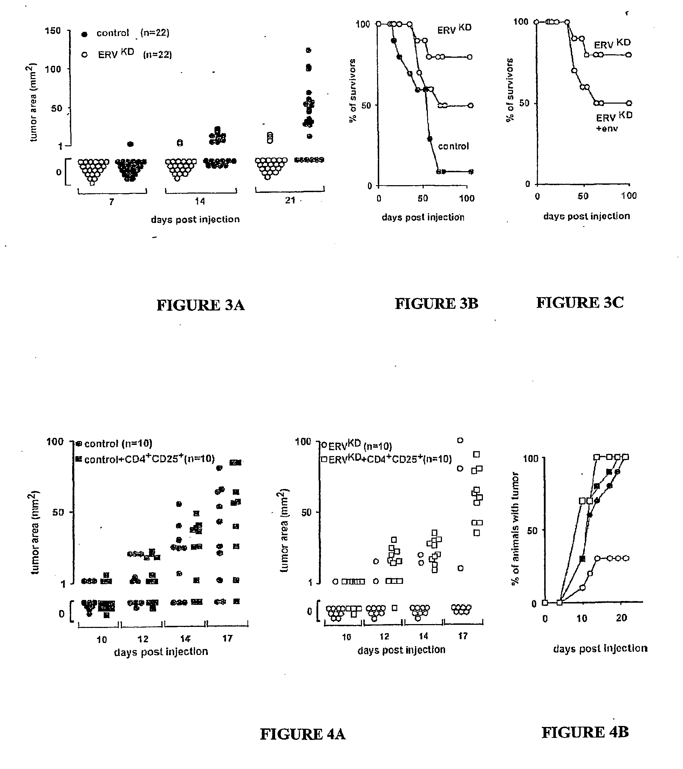

**Figures 3A, 3B and 3C****:** Inhibition of tumor cell growth and increased mouse survival upon ERV knockdown
Figure 3A: Tumor cell growth of control and ERV^{KD} B16 cells engrafted into immunocompetent C57B1/6 mice (22 mice per group; same experimental conditions as in figure 2B).
Figure 3B:Percentage of survivors among the control and ERV^{KD} B16 cells engrafted mice (10 mice per group).
Figure 3C: Percentage of survivors (10 mice per group) among Me1ARV *env* -and control- transduced ERV^{KD} B16 cells.
**Figures 4A and 4B****:** Adoptive transfer of CD4⁺CD25⁺ T cells restores ERV^{KD} B16 tumor growth
   The CD4⁺CD25⁺ T cells were purified from splenocytes of C57B1/6 mice injected 17 days before with B16 cells. Adoptive transfer was performed by intravenous injection of 0.1 ml of PBS that contained (squares) -or not (circles)- 5x10⁴ purified CD4⁺CD25⁺ cells. Control (filled symbols) and ERV^{KD} (opened symbols) B16 cells were engrafted on the same day (10 mice per group), and tumor area (figure 4A) and percentage of animals with tumor (figure 4B) were monitored.

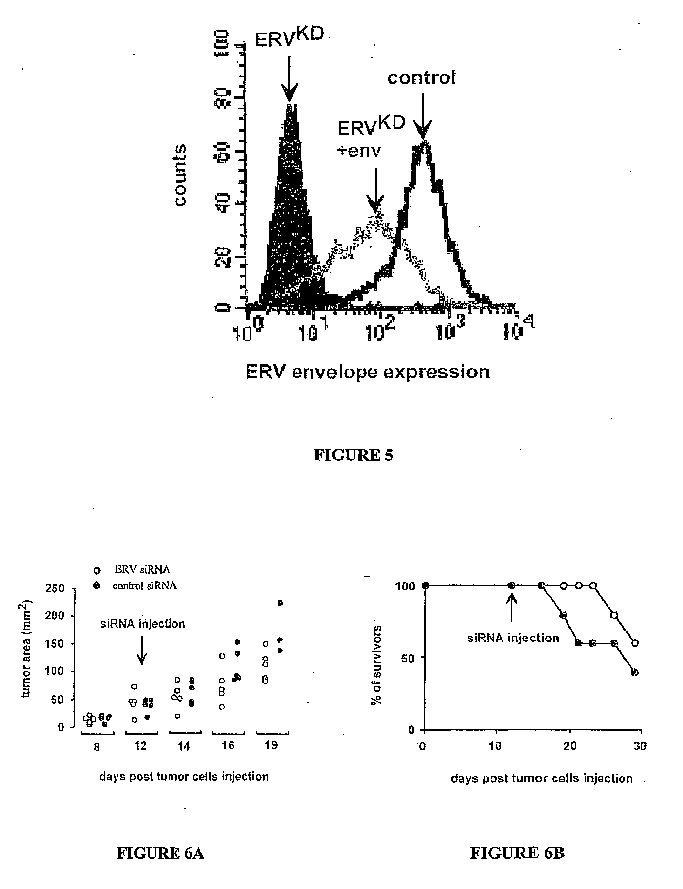

**Figure 5****:** Immunostaining for ERV envelope protein detection
   Control, ERV^{KD} , and ERV^{KD} +env B16 cells were labelled with the 9B6 antibody (directed against the MelARV envelope protein; gift from E. Gorelik, Cancer Res 1988; 48:4954-4958) revealed by a goat anti-mouse FITC antibody (Caltag, Burlingame, USA). Flow cytometry analysis was performed using a Facscalibur cytometer.
**Figures 6A and 6B****:** *In vivo* systemic administration of siRNA reduces tumor cell progression
   Synthetic siRNA targeted to the 19 nt ERV and control (GFP) sequences referred to in figure 1b and Materials and Methods were purchased from MWG Biotech. They were injected intraperitoneously (3 µg of siRNA in 50 µl of PBS), at day 12 after prior engraftement of 2x10⁵ B16 cells in the right flank of the mice. The tumor area (figure 6A) and the percentage of survivors (figure 6B) were monitored (5 mice per group in two independent experiments).

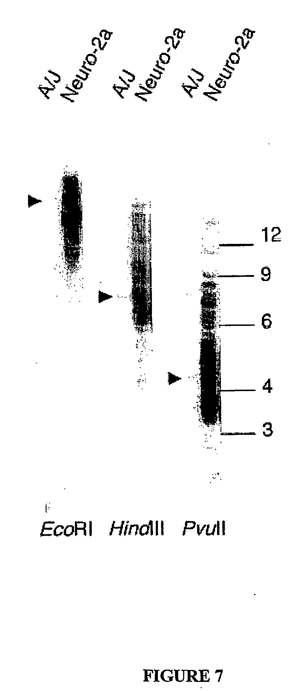

**Figure 7****:** Southern blot analysis of ecotropic MLV proviral sequences in A/J mouse normal tissue and Neuro-2a cell
   Genomic DNA was extracted from A/J mouse liver and Neuro-2a cells. DNA (30 µg) from each sample was digested by EcoRI, HindIII and PvuII and separated on a 0.7% agarose gel for 24 hours. Following transfer, membranes were hybridized with the ecotropic MLV env-specific probe. Numbers to the right refer to molecular size markers (in kilobases). Arrows indicate the Emv-1 specific band.

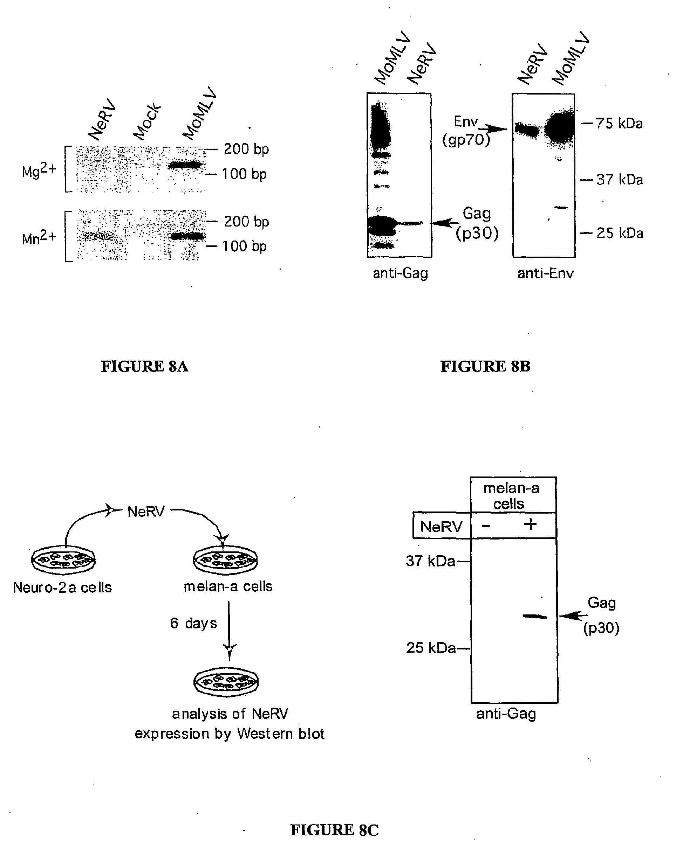

**Figures 8A to 8C****:** Characterization of Neuro-2a-derived particles
Figure 8A: Reverse transcriptase (RT) activity assay. RT activity in supernatants from Neuro-2a cells (NeRV) was assayed in the presence of Mn2+ or Mg2+ using a PCR-based method with MS2 bacteriophage RNA as a reverse transcription template, as described.13 The supernatants of NIH/3T3 cells infected (MoMLV) or not (Mock) by MoMLV were used as positive and negative controls, respectively.
Figure 8B: Viral protein immunodetection. Proteins from the particulate material harvested from Neuro-2a cell supernatants were separated by SDS-PAGE, blotted, and NeRV proteins were detected with anti-Gag and anti-Env goat antisera. Molecular weights are indicated on the right.
Figure 8C: Infectivity assay. Melan-a cells were exposed (NeRV+) or not (NeRV-) to Neuro-2a cell supernatant. Six days later, expression of retroviral Gag proteins by the target cells was analyzed by Western blotting as in Figure 8B.

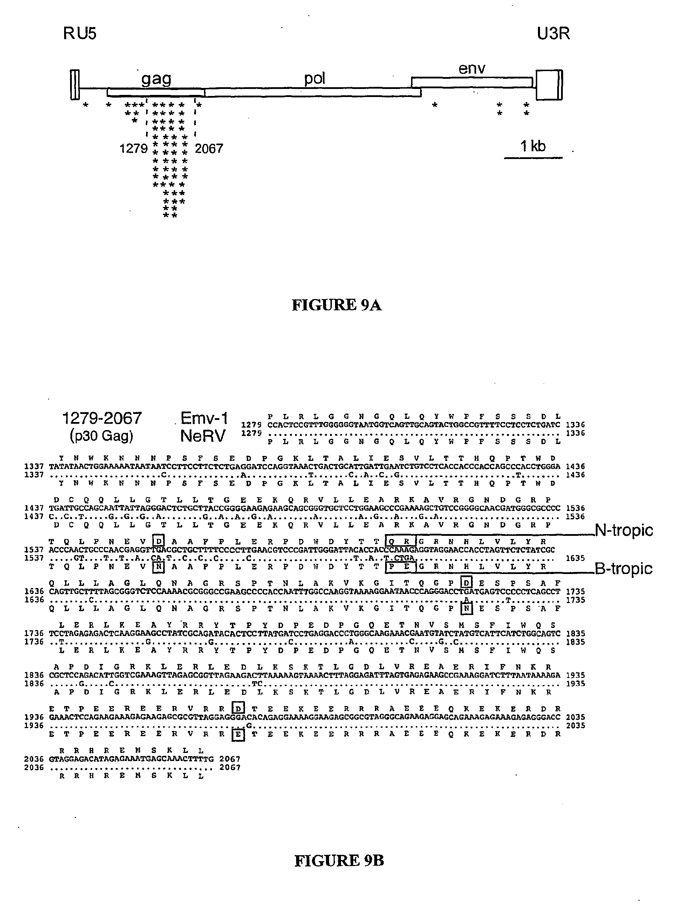

**Figures 9A and 9B**: Sequence variations between Emv-1 and NeRV
Figure 9A: gag, pol and env ORF map of the NeRV. Asterisks indicate the position of divergent nucleotides between Emv-1 and NeRV, with the position of the p30 gag ORF delineated by dotted lines.
Figure 9B:Sequence alignment of the 1279 to 2067 region of the Emv-1 and NeRV gag genes; divergent amino acids are boxed.

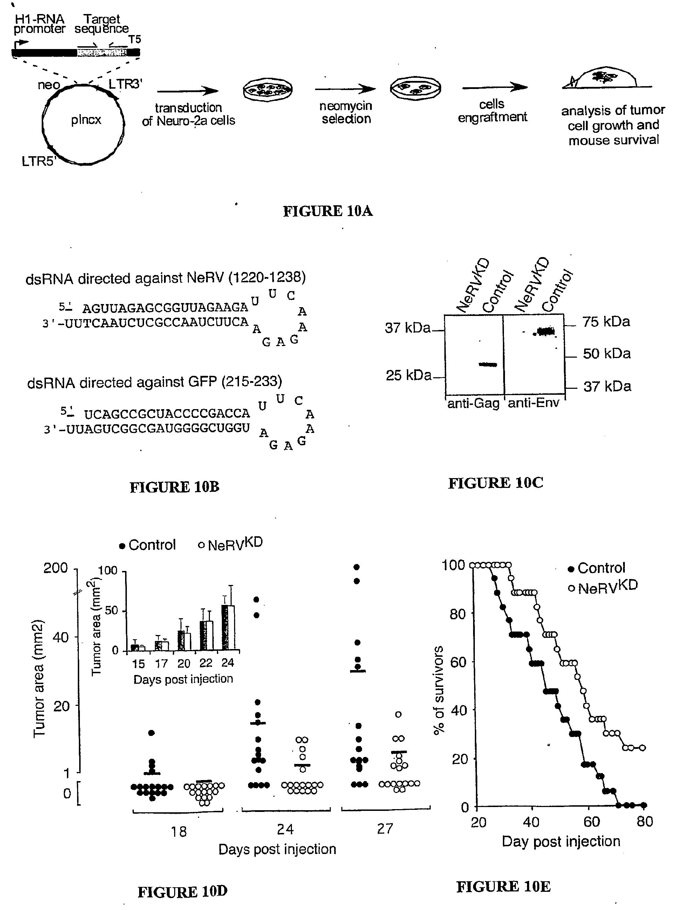

**Figures 10A to 10E**: Reduced tumor cell growth and increased mouse survival upon NeRV knockdown
Figures 10A: Knockdown procedure and rationale of the assay. To knock down NeRV expression, we constructed a plncx-derived vector making use of the pSUPER vector to generate, under control of the H1-RNA promoter, short double-stranded transcripts for RNA interference (see Fig. 10B). Neuro-2a cells were transduced with these expression vectors, submitted to neomycin selection, and the resulting NeRVKD and control Neuro-2a cells were injected subcutaneously into the flank of the mice, whose tumor growth was monitored.
Figures 10B: Sequence of the dsRNA generated by the NeRV and control (GFP) RNAi expression vectors; numbers refer to nt positions within the respective targeted sequences.
Figures 10C: Western blot analysis of Gag and Env proteins present in the particulate material harvested from the supernatant of NeRVKD and control cells.
Figures 10D: Role of NeRV on tumor cell growth *in vivo.* Control and NeRVKD Neuro-2a cells were injected subcutaneously into the flank of immunocompetent A/J mice (17 mice per group) or of X-irradiated (5 Gy) immunocompromised A/J mice (5 mice per group, inset). Tumor growth was determined by measuring tumor area (control: filled circles and bars; NeRVKD: open circles and bars; p = 0.00046).
Figures 10E: Percentage of survivors among the control and NeRVKD Neuro-2a cells engrafted mice (17 mice per group; p = 0.022).

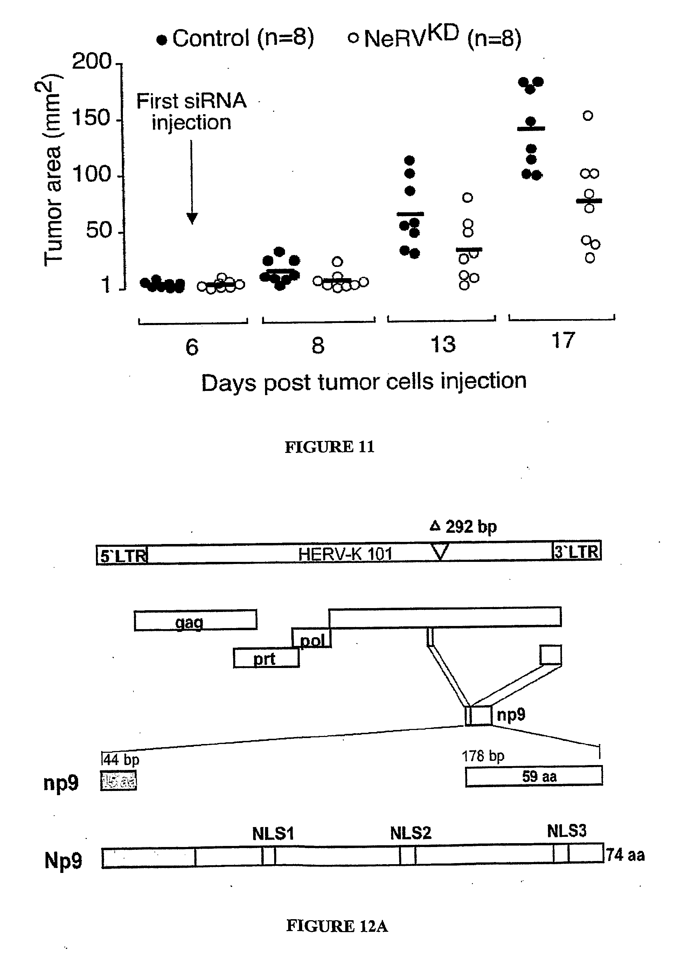

**Figure 11**: In vivo systemic administration of siRNA reduces tumor cell progression Synthetic siRNAs targeting the 19 nt NeRV and control (GFP) sequences referred to in
Fig. 10A were purchased from MWG Biotech. These synthetic siRNAs (0.2 nmol of siRNA in 500 µl of PBS) were injected intraperitoneously every 2-3 days into immunocompetent mice bearing a palpable Neuro-2a tumor (8 mice per group). The first siRNA injection was performed 6 days after engraftment of 5x10⁶ Neuro-2a cells (control: filled circles and bars; NeRVKD: open circles and bars; p = 10-6).
**Figures 12A** **and** **12B**
Figure 12A: Schematic representation of the HERV-K101 provirus.
   The open reading frames encoding the viral proteins Gag, Prt, and Pol are indicated. The env reading frame encodes Np9. The two exons of the np9 gene are highlighted. The shown provirus type fails to code for Rec, due to the type 1-specific deletion of 292 bp depicted. (B) Np9 protein. The positions of the three putative NLSs are depicted.

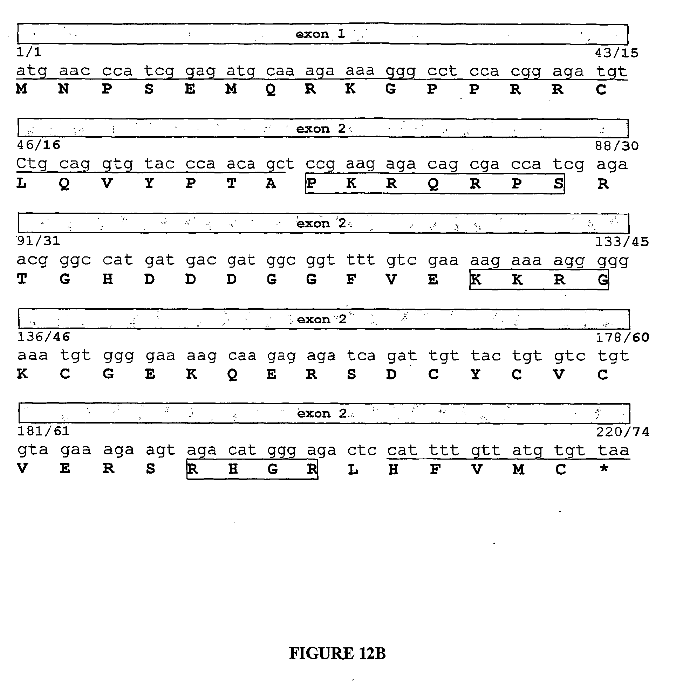

Figure 12B: Nucleotidic sequence of Np9 (SEQ ID NO: 2) was aligned, beginning with the first exon at nt position 6451 in the HERV-K (HML-2.HOM) sequence. The positions of the PCR primers forward and reverse used to construct phCMV-Np9 are underlined. The deduced amino acid sequence is shown and the putative NLSs are boxed.

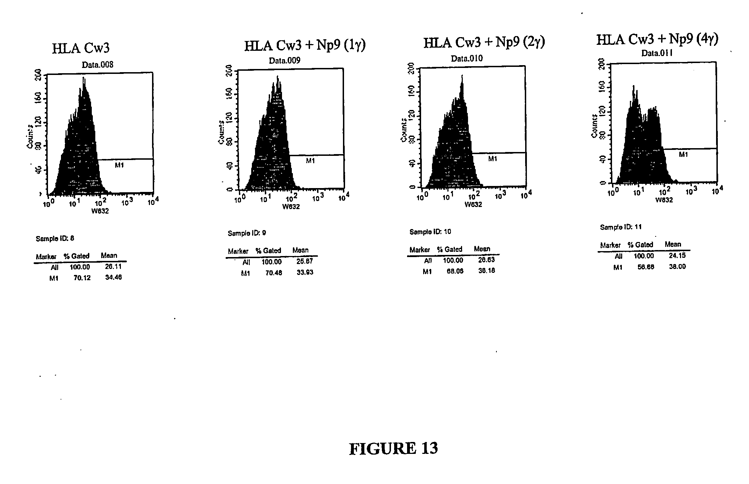

**Figure 13**: Downregulation of HLA class I expression by 293T cells cotransfected with Np9 expressing vectors
   The figure demonstrates the downregulation of HLA class I expression by 293T cells cotransfected with 5µg of Np9 expression (phCMV-Np9) vector and 3µg of HLA-A2, HLA-B27 or HLA-Cw3 (all three as EcoRI genomic fragments in pBR 328) expression vectors. HLA class I expression was measured by FACS with PE-coupled anti-human MHC-I antibody W6/32 (eBioscience) 72h post-transfection.

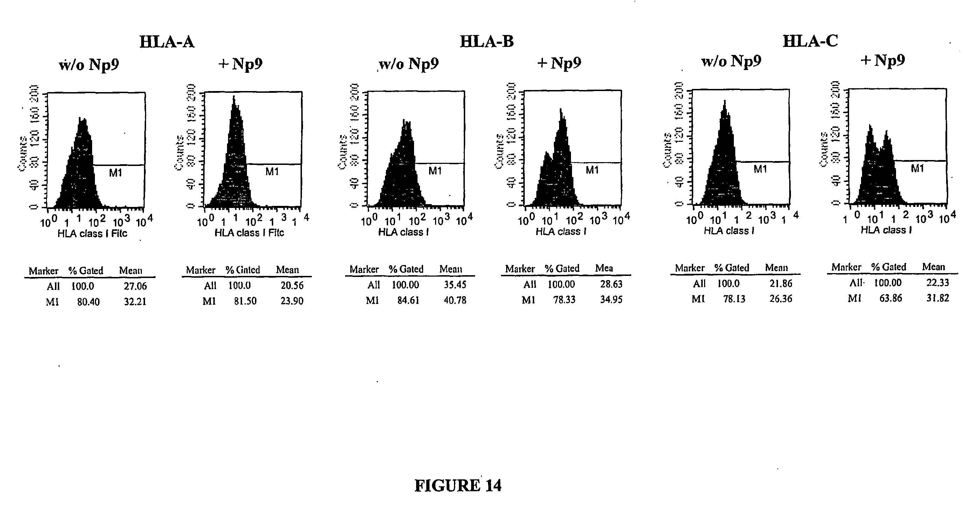

**Figure 14****:** Dose-dependent downregulation of HLA class-I expression by 293T cells cotransfected with the indicated variable amounts (in µg) of Np9 expression vector(phCMV-Np9) and a fixed amount (2µg) of HLA-Cw3 expression vector. HLA class-I expression was measured by FACS with PE-coupled anti-human HLA class-I antibody W6/32 (eBioscience) 72h post-transfection.

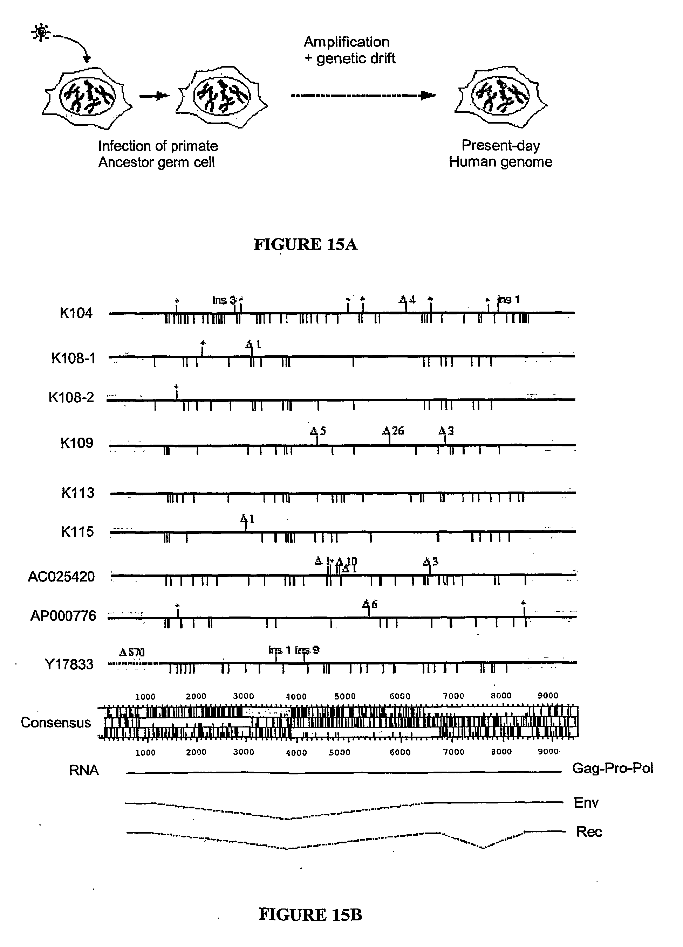

**Figures 15A and 15B**: HERV-K(HML2) "endogenization" and present-day human proviruses
Figure 15A: Evolutionary scheme for HERV-K(HML2) entry into and invasion of the genome of primates.
Figure 15B: Map of the full-length 9.4 kb-long human-specific HERV-K(HML2) proviruses and comparison with the *in silico*-engineered consensus sequence (SEQ ID No:4). Each provirus is represented by a solid dark line, with the amino acid substitutions in Gag, Pro, Pol and Env as compared with the consensus element indicated below the line, and the insertions/deletions and premature Stop codons (red stars) indicated above the line. The ORF map of the consensus provirus is shown, with gag in green, pro in pink, pol in blue, env in orange and yellow, the bipartite rec in orange, and the two LTRs as grey boxes. (Note that the first coding exon of rec belongs to the env ORF). The transcripts responsible for the expression of the viral proteins - with the corresponding spliced out domains (dotted lines)- are schematized below the ORF map.

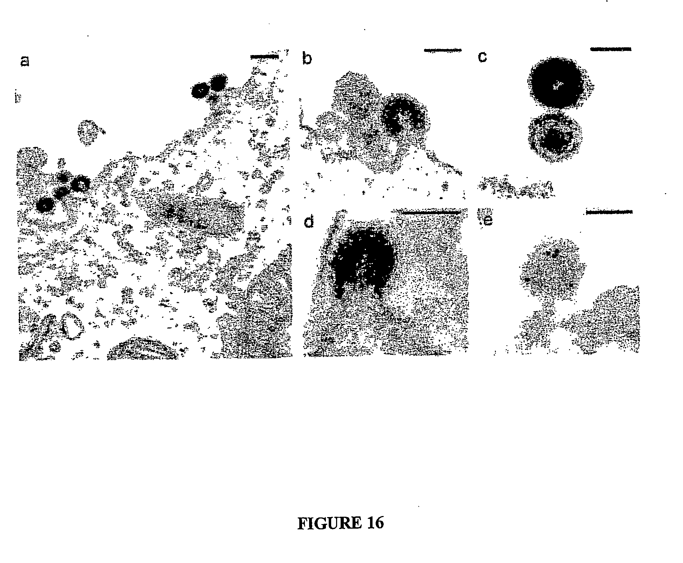

**Figures 16A to 16E:** Electron microscopy of the viral-like particles generated by the Phoenix provirus. Human 293T cells were transfected with an expression vector for Phoenix and observed 48 h post-transfection
Figure 16A: Low magnification of particles assembled at the cell membrane.
Figure 16B: Representative image of particles budding from the plasma membrane.
Figure 16C: High magnification of two particles, one of which (bottom) discloses a mature morphology with a condensed core, while the other appears to be still immature with two dark peripheral rings surrounding an electron-lucent core.
Figure 16D: High magnification of a particle with prominent spikes, corresponding to the Env protein.
Figure 16E: Image of a particle after labeling with an antibody specific for the HERV-K envelope protein, and a secondary antibody linked to gold beads, obtained by immuno-electron microscopy. Scale: (a): 200 nm, (b-e):100 nm.

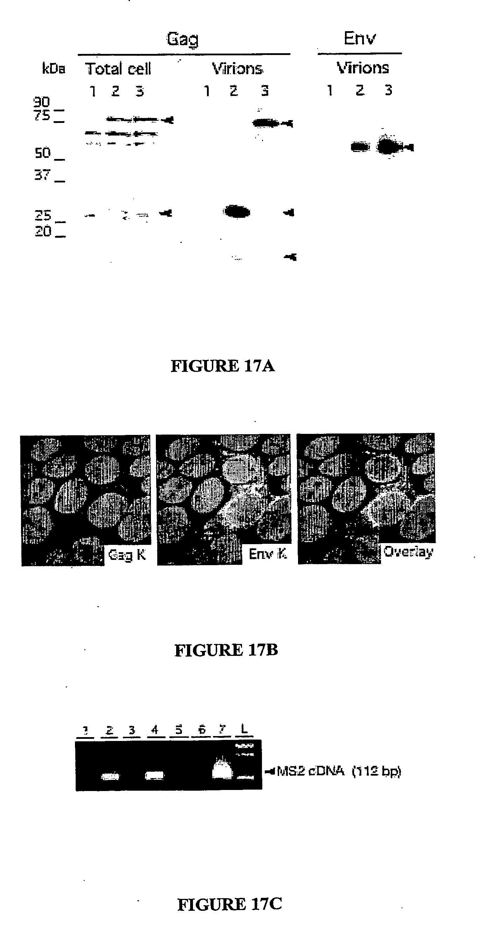

**Figures 17A to 17C****:** Characterization of Phoenix
Figure 17A: Immunoblot analysis of the Phoenix products present in the lysates of transiently transfected 293T cells, or in the concentrated supernatant. Cells were transfected with (1) a control plasmid (pCMV-β), (2) an expression vector for Phoenix or (3) an expression vector for a pro mutant. The membranes were hybridized with an antibody directed against the Gag protein (Left) or the Env protein (Right).
Figure 17B: Immunofluorescence study of 293T cells transfected with Phoenix. Gag is marked in red, Env in green and the nuclei are stained in blue. Note the co-localization of the two proteins, especially at the membrane that separates the two adjacent marked cells.
Figure 17C: Detection of RT activity in the concentrated supernatant of 293T cells transfected with Phoenix and a series of proviral and control vectors. Concentrated virions were used as a source of RT activity to reverse-transcribe synthetic MS2 RNA. Presence of cDNA was revealed via a PCR amplification using appropriate primers. (1: pCMV-β; 2: Phoenix; 3: Phoenix mutated in RT; 4: chimera K109-K115; 5: chimera K109-K113; 6: chimera K109-K108; 7: MLV core proteins).

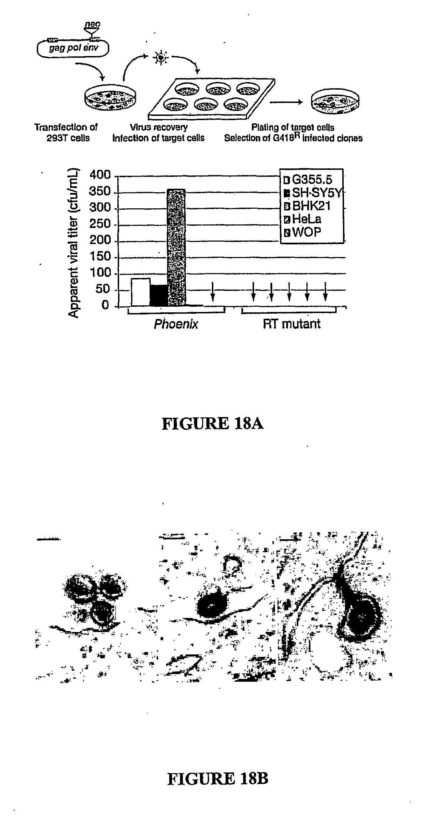

**Figures 18A and 18B****:** Infectious properties of Phoenix encoded particles
Figure 18A: Infectivity of the HERV-K retroviral particles. The apparent viral titer observed with the neo-marked HERV-K(HML2) provirus is indicated for a panel of target cell lines, together with that obtained with a mutant for the RT domain. The rationale of the assay is schematized in the upper part.
Figure 18B: Infection of cells by Phoenix particles. On the left, two mature particles appear to interact with the cell membrane of the closest cell, with a thickening of the membrane at the exact point of interaction with the particle. In the middle and on the right, two images suggestive of a cell-to-cell infection are presented, with a particle still budding from its progenitor cell already in contact with the neighboring cell.

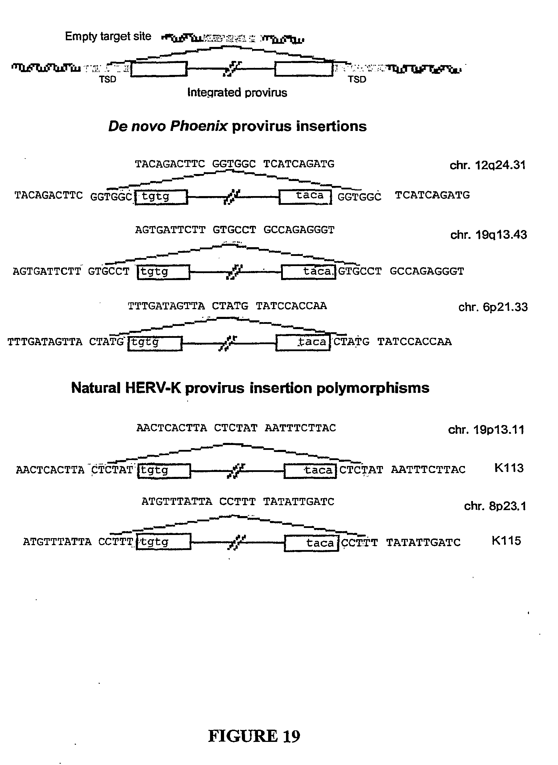

**Figure 19****:** Structure of three de novo integration sites of Phoenix provirus, and comparison with the structure of natural HERV-K provirus insertions
   The complete characterization of inserted Phoenix elements and insertion sites was performed using individual clones from human SH-SY5Y cells after infection and G418 selection. A provirus insertion and the corresponding empty site are schematized at the top. The sequences of three characterized Phoenix de novo insertions are shown below, with the flanking DNA in uppercase and the proviral sequences in lowercase; target-site duplications of 5/6 bp (TSD,) are found in all cases, associated with full-length LTRs. For comparison, the corresponding structures of two resident HERV-K proviruses with a polymorphic insertion in humans (9) are presented in the lower part.

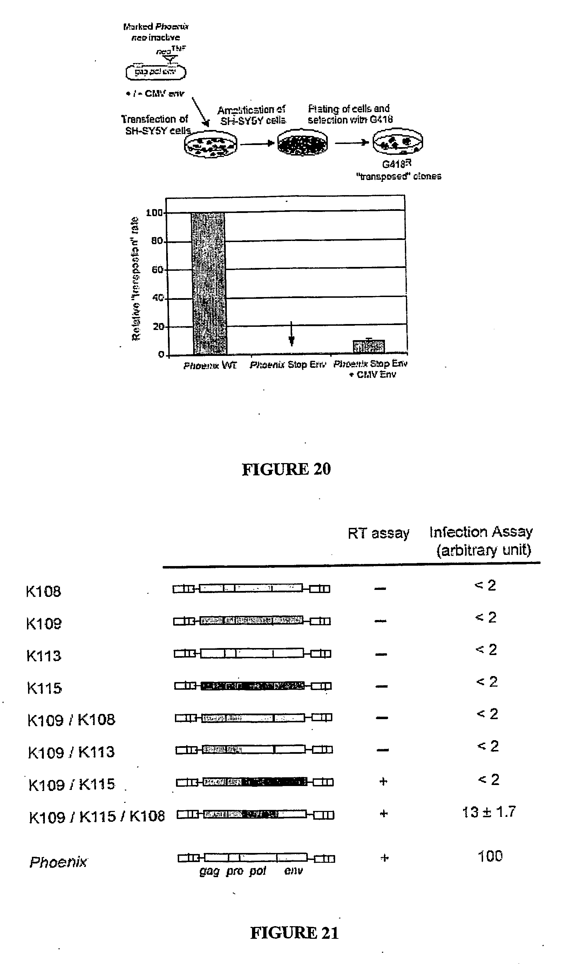

**Figure 20****:** Assay for Phoenix "retrotransposition"
   Upper part: rationale of the assay. Phoenix was marked with the neoTNF indicator gene for retrotransposition (31), in which neo becomes active only after a complete retrotransposition cycle (i.e. transcription, reverse transcription and integration), and the marked element was introduced by transfection into SH-SY5Y human cells permissive for Phoenix infection. The cells were then amplified, and subjected to G418 selection for 2 weeks; the number of G418R clones (several of which were assayed by PCR to demonstrate splicing out of the intron within the neo indicator gene) yielded the apparent rate of retrotransposition of the marked elements. Lower part: "retrotransposition" is dependent on the presence of a functional env gene, either present within the native Phoenix provirus (Phoenix WT) or added in trans to an env-mutated Phoenix provirus (Phoenix Stop Env) by cotransfection of the cells with an env expression vector (CMV Env).
**Figure 21****:** An infectious HERV-K(HML2) retrovirus can be generated by in vitro recombination from cloned HERV-K loci. Structure of the cloned HERV-K(HML2) proviruses and of the in vitro constructed chimera (see Materials and Methods), with the RT activities in the supernatant of 293T cells transfected with the corresponding plasmids measured as in Fig.3C, and the associated infection efficiencies measured by quantitative PCR on the target cell genomic DNA (BHK21 cells, see Methods).

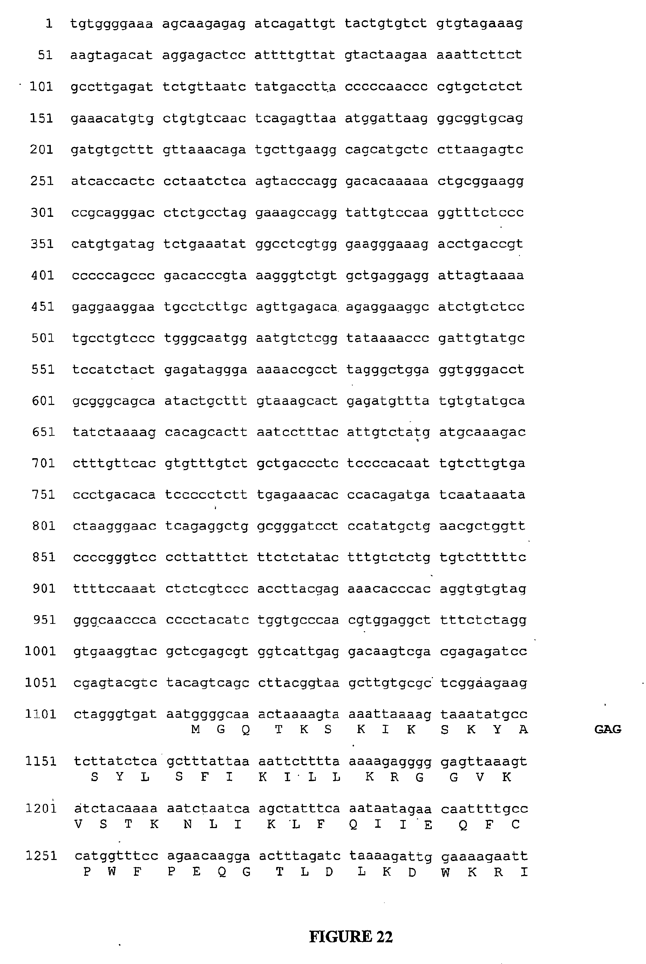

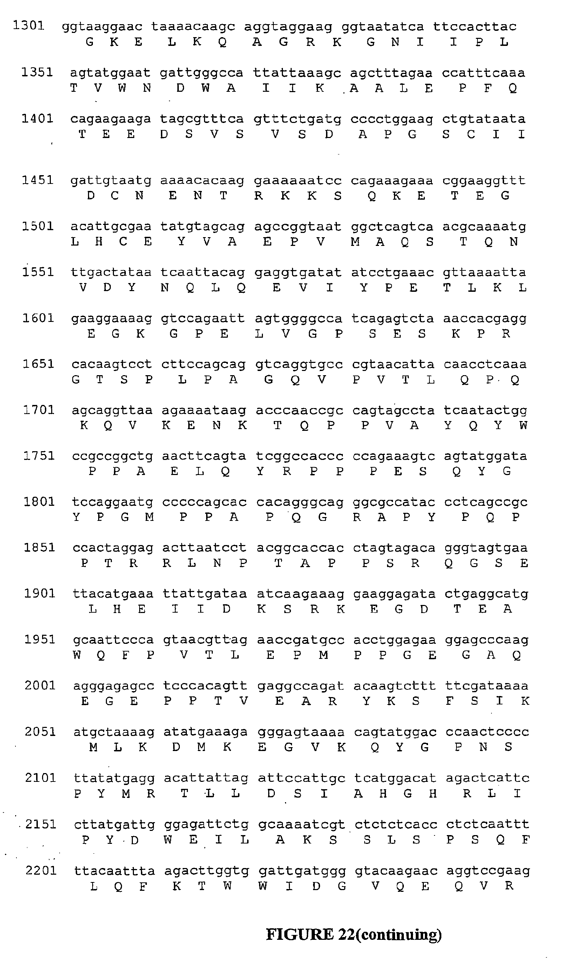

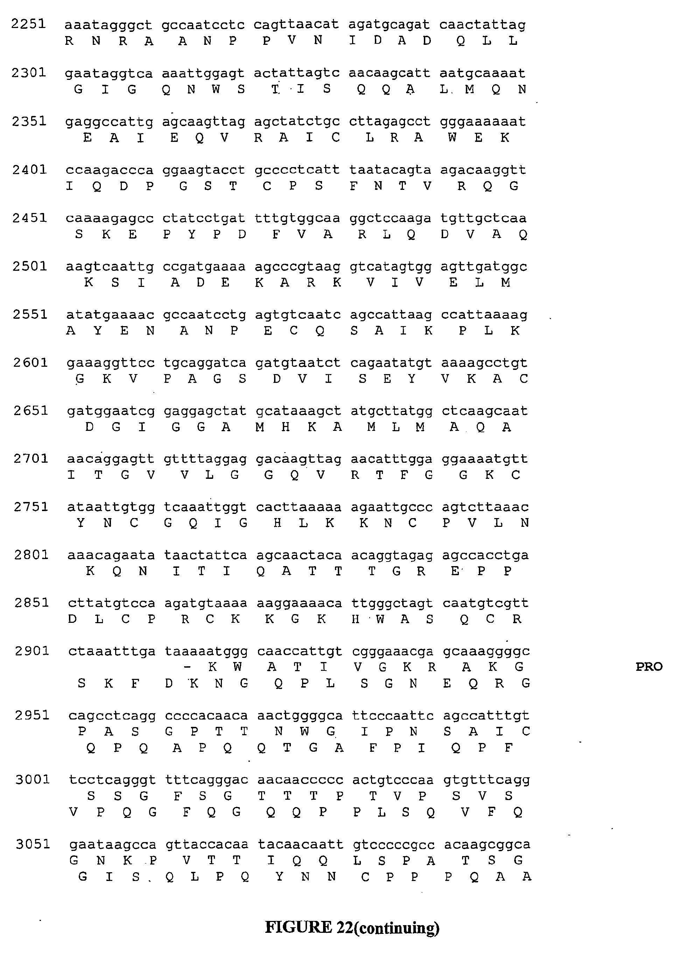

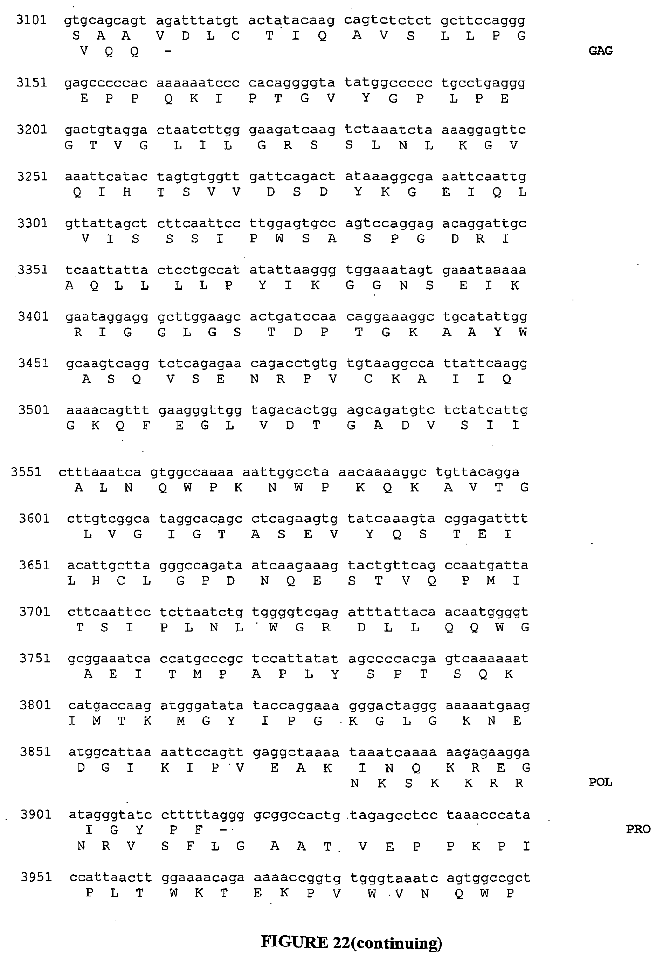

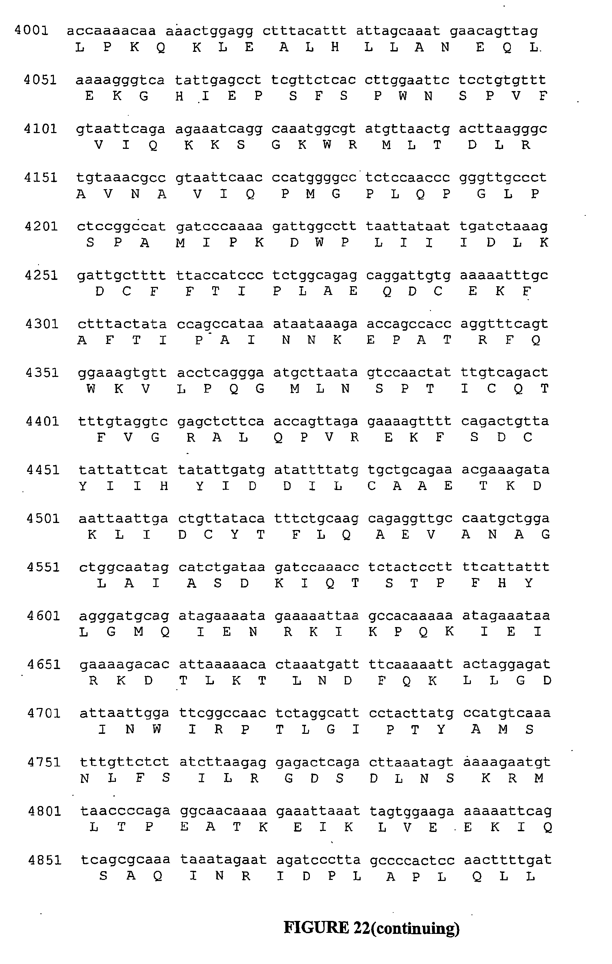

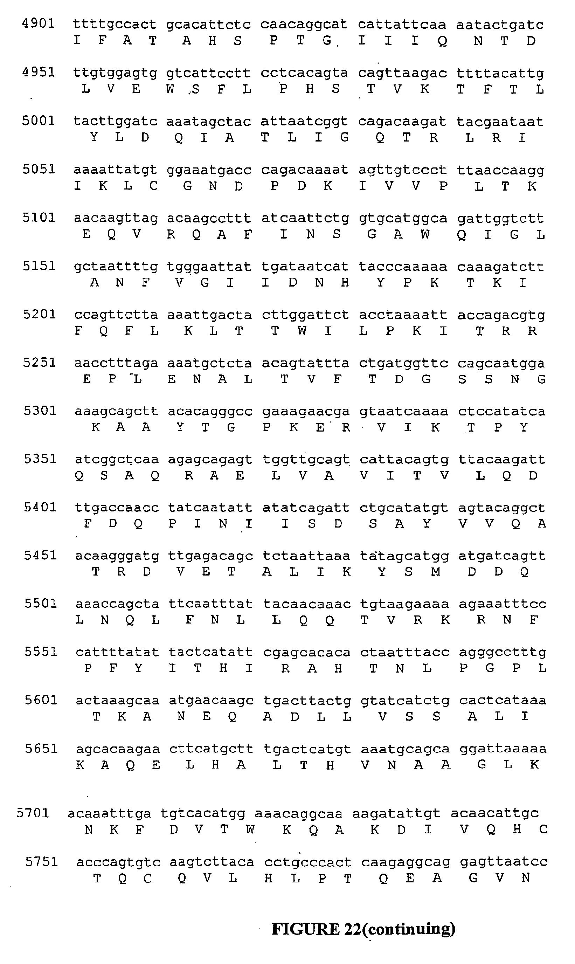

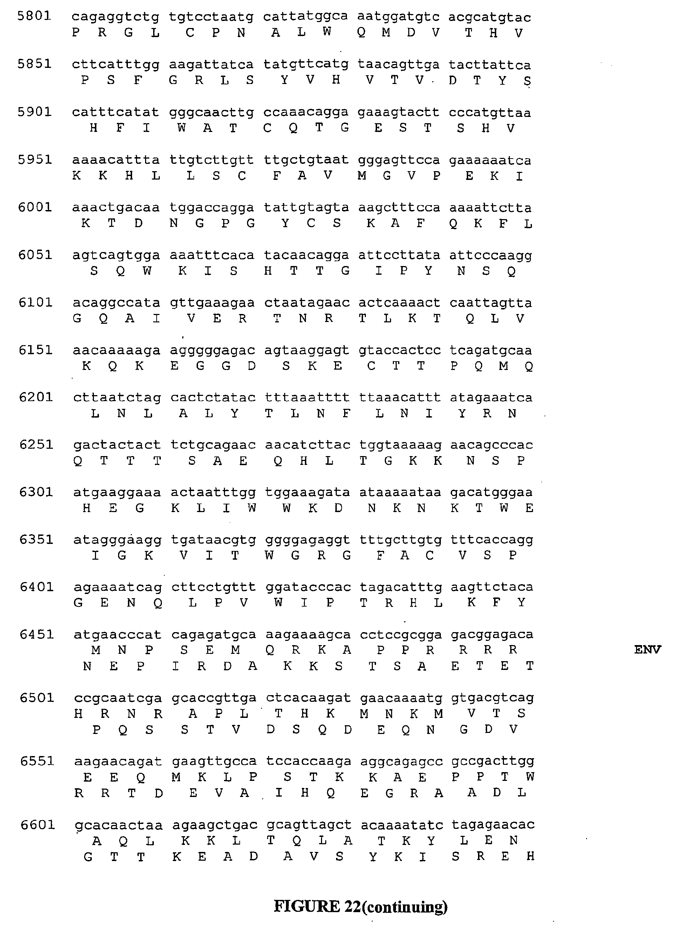

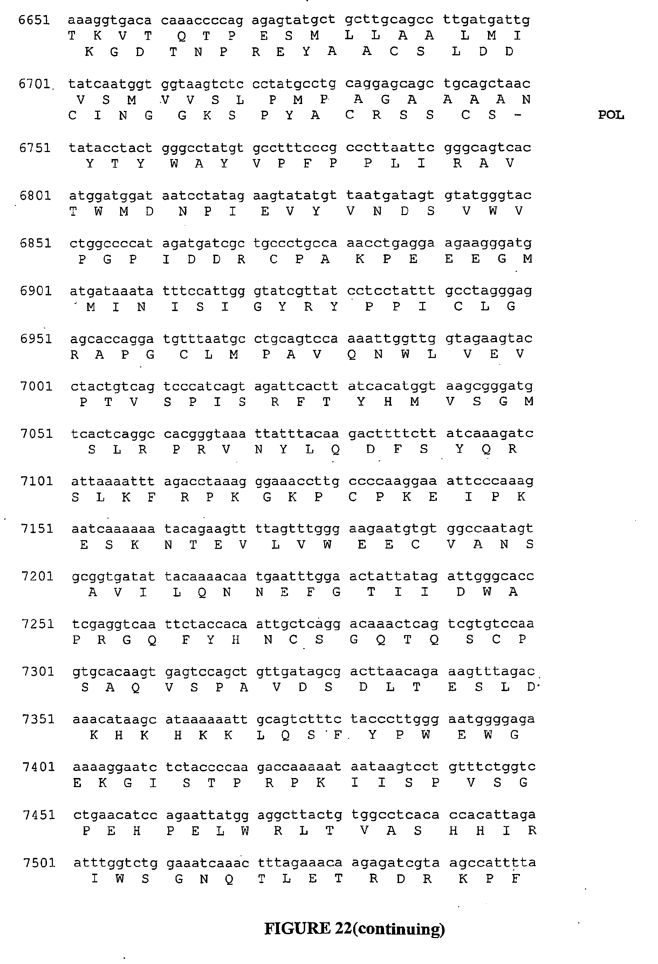

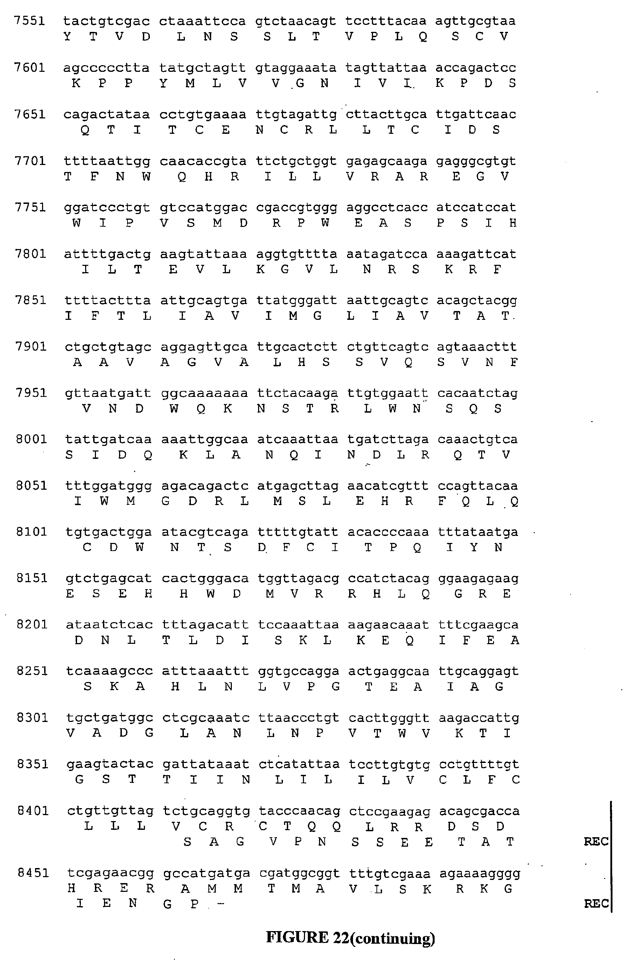

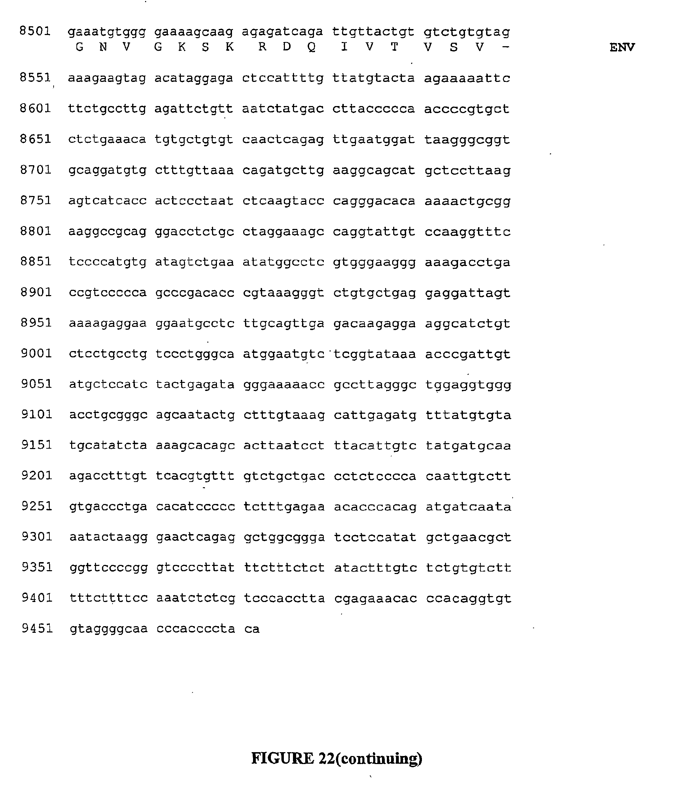

**Figure 22****:** Annoted nucleic acid (SEQ ID NO: 4) and amino acid sequences (SEQ ID NOs. 5 to 9 of the consensus Phoenix provirus.

### EXAMPLE 1: Endogenous Retrovirus expression is required for murine melanoma tumor growth in vivo

Tumors are the final outcome of a series of genetic events, which include not only cell transformation *per se* but also modifications of the interactions between the transformed cells and the host. In particular, it is now recognized that developing tumors arise from the fraction of transformed cells having acquired the capacity to escape immune surveillance (reviewed in references 1-3). The molecular bases for the acquisition of an immune-resistant phenotype are not fully understood, although several mechanisms have been described, including down regulation of MHC class I antigens (2) and up-regulation of apoptosis inhibiting factors (3). Along this line, we have previously shown that introduction of an expression vector for the envelope (*env*) gene of an infections murine retrovirus can induce escape from immune surveillance, in cells otherwise rejected after engraftment into immunocompetent mice (4). Similar effects were observed with the *env* gene of a human endogenous Retrovirus (ERV)(5). ERVs, which occupy a large fraction of mammalian genomes, are the genomic traces of ancient retroviral infections that reached the germ line, with the integrated proviruses then being transmitted in a Mendelian manner (reviewed in references 6 and 7). Most of these sequences have accumulated point mutations and deletions, but there are still hundreds of full-length proviral elements in the human -and mouse- genomes, with some of them still containing coding genes responsible, for instance, for the observation of viral-like particles in some tumors (6, 8). Accordingly, we hypothesised that expression of endogenous retroviruses could be involved in tumor progression *in vivo,* by subverting the immune response. In this example, we used a spontaneous murine melanoma tumor cell line and demonstrate that an ERV, the Melanoma Associated RetroVirus (MelARV), whose expression is spontaneously induced in melanoma of C57B1/6 origin (9), is necessary for a regulatory T cell-mediated immune escape of the tumor cells *in vivo.*

### A Materials and Methods

### Mice and cell lines

C57BL/6 and SCID mice, 8-12 weeks old, were obtained from Janvier (France). B16 (murine melanoma cell line of C57BL/6 origin, EACC 94042254) and 293T (human embryonic kidney cells, ATCC CRL11268) were maintained in DMEM supplemented with 10% heat-inactivated foetal calf serum and antibiotics.

### Constructions

PlncxHl expression vectors derived from the plncx (10) and the pSUPER (11) vectorshave been constructed to generate short transcripts directed against MelARV (targeted to the genomic transcript within the gag sequence; nt positions 1220-1238 from the start codon), or against the green fluorescent protein transcript (nt position 215-233 from the start codon) as a control. They were obtained by first inserting annealed 64-mer oligonucleotides (sequences in figure 1) into pSUPER opened at the *Bgl*II and *Hind*III sites, followed by introduction of the *BamH*I*-Hind*III fragment from these constructs into plncx opened at the corresponding sites. The expression vector for the MelARV envelope (pDFG MelARVenv) and the control (pDFG none) were constructed by introducing (or not) a RT-PCR product, generated from the MelARV viral RNA using an *AgeI*-containing primer at the envelope 5'-end and a *XhoI-*containing primer at the envelope 3'-end, into a hygromycin-containing pDPG vector (4) opened at die same sites.

### Establishment of ERV^{KD} B16 tumor cells

7.5x10⁵ 293T cells were cotransfected with the plncxH1 vector (1.75 µg) and expression vectors for the MLV proteins (0.55 µg for the amphotropic MLV envelope vector and 1.75 µg for the MLV gag and pol vector, see reference 12). Thirty six hours post-transfection, viral supernatants were collected for infection of the B16 tumor cells (2.5 ml of supernatant for 5x10⁵ cells, with 8 µg/ml polybrene). Cells were maintained in selective medium (1 mg/ml neomycin) for three weeks. In some experiments, the pDPG MelARVenv expression vector (or control pDFG none) was additionally introduced into the cells using the same protocol and infected cells were selected with 300 units/ml hygromycin.

### Expression of MelARV proteins

Analysis of MelARV expression was performed by Western 30 blot analyses. The supernatants of 10⁷ cells were collected, centrifuged for 10 min at 100xg, filtered and concentrated by ultra-centrifugation in a SW41 Beckman rotor (150,000xg, 1 hour, 4°C). Pellets were resuspended in lysis buffer, submitted to SDS-PAGE, blotted and revealed with an anti-Env mAb (13) and an anti-Gag goat serum (Viromed Biosafety Labs).

### In vitro transformation assay:

Both control- and ERV^{KD}- B16 cells were plated in soft agar to determine the efficiency of anchorage-independent growth. Cells (2x10³ or 2x10⁴ were plated in 5 ml of 0.33 % agar in DMEM with 10% foetal bovine serum overlaid onto a solid layer of 0.5 % agar in DMEM supplemented with 10 % foetal bovine serum. The culture was maintained for 4 weeks, the colonies were stained with INT solution (Sigma-Aldrich) and then counted.

### Tumor progression in vivo

For *in vivo* assays, tumor cells were washed three times with PBS, scrapped without trypsination, and subcutaneously inoculated in the shaved area of the right flank of the mice. Tumor establishment was determined by palpation and tumor area was determined by measuring perpendicular tumor diameters.

### CD4⁺CD25⁺ T cell purification and adoptive transfer in syngenic C57BL/6 mice

CD4⁺CD25⁺ cells were freshly isolated from spleens of C57BL/6 mice engrafted with 2x10⁵ B16 cells 17 days before. Cells were purified by a two step procedure of negative and positive selections, using MACS magnetic beads (mouse regulatory T cell isolation kit, Miltenyi Biotech), according to the manufacturer's instructions. Fifty thousands purified lymphocytes were transferred intravenously into naive C57BL/6 mice. Recipient mice were challenged the same clay with 2x10⁵ control- or ERV^{KD}-B 16 cells in the right flank.

### B Knocking down ERV does not modify the transformed phenotype of B16 melanoma cells

An RNA interference approach based on stable vectors producing short double-stranded RNA (dsRNA) directed against the viral genome of the MelARV element and the irrelevant *gfp* gene as a control has been used. The rationale of the procedure and the structure of the plasmids used are illustrated in figures 1A, 1B. Figure 1C clearly shows that the ERV-specific dsRNA vector almost completely abolished ERV expression in the transduced B16 cells (ERV^{KD} B16 cells), with a > 10 fold reduction in the amount of both the Env and Gag viral proteins as compared to the control transduced cells (control B16 cells). As a next step, the transformed phenotype of the ERV^{KD} and control B16 cells was assayed both *in vitro* and *in vivo*. *In vitro,* we measured the anchorage independent growth rate after plating in semi-solid media (soft agar assay). As illustrated in figure 2A, the ERV^{KD} B16 cell line gave rise to a similar number of colonies as the control B16 cells. *In vivo,* we analysed the growth rate of the two cell populations after engrafting into X-irradiated or SCID mice. As illustrated in figure 2B, both cell populations have a transformed phenotype, with similar growth rates. Altogether, these results show that knocking down the MelARV endogenous retrovirus has no effect on the transformed state of the melanoma cells.

### C Knocking down ERV inhibits B16 tumor cell growth in vivo and increases survival of immunocompetent hosts

To investigate whether tumor cells may overwhelm the antitumor response *in vivo* through an ERV-dependent mechanism, we explored the impact of the knocking down of MelARV on tumor progression by injecting C57BL/6 immunocompetent mice with the control and the ERV^{KD} B16 cells. As illustrated in figure 3A, growth of control B16 cells, as expected, led to large tumors in most of the animals, whereas the ERV^{KD} B16 cells yielded tumors of a limited size and in only a small number of engrafted mice. The difference in tumor cell growth is also clearly substantiated by the extent of animal survival (figure 3b): as soon as day 70, 90 % of the mice engrafted with the control B16 cells had been killed by their tumor, whereas 80 % of mice engrafted with ERV^{KD} B16 cells were alive and tumor-free (and still so at day 130). In an attempt to identify the MelARV genes involved in the observed effects, we introduced back into the ERV^{KD} B16 cells an expression vector (lacking the dsRNA-targeted sequence) for the sole MelARV *env* gene. The resulting double-transduced ERV^{KD} +env (or control) B16 cells were then engrafted into C57BL/6 mice. As illustrated in figure 3C, this resulted in partial reversion of the knockdown effect, with already 50 % of the mice engrafted with the Env-expressing cells dead by day 70. This reversion indicates that the env gene is -at least in part- responsible for tumor immune escape. The partial effect of the reversion is most likely explained by the lower expression (figure 5) of the Env protein when expressed by the exogenous vector.

### D Adoptive transfer of CD4⁺CD25⁺ regulatory T lymphocytes restores tumor progression of ERV^{KD} B16 cells

It has been reported that B16 tumor growth *in vivo* requires the presence of regulatory T cells (14, 15). On the other hand, murine leukaemia viruses closely related to MelARV have also been shown to induce regulatory T cells (16, 17). One can therefore hypothesize that ERV expression by the B16 melanoma is directly responsible for regulatory T cell induction, thus resulting in tumor progression. To test this issue, we attempted to complement the reduced tumor progression of the ERV^{KD} B16 cells by adoptive transfer of the CD4⁺CD25⁺ regulatory T cells isolated from C57BL/6 mice engrafted with wild-type B16 cells. As shown in figure 4, the transfer of these CD4⁺CD25⁺ cells had no effect on tumor progression of engrafted control B16 cells (figures 4A left and 4B), but enhanced ERV^{KD} B16 tumor cell growth up to levels similar to those of the control B16 cells (figures 4A right and 4B). These results therefore strongly suggest that regulatory T cells are the necessary intermediates of the ERV-mediated effect.

The present data demonstrate that tumors are able to overwhelm the immune system by expressing the envelope of an ERV. Blocking ERV expression resulted in enhanced tumor rejection, and expression of the sole ERV envelope protein partially restored the initial extent of tumor progression. We also demonstrate that regulatory T cells are sufficient to allow a tumor cell line devoid of ERV expression to progress into a tumor. More generally, the results also suggest that tumor cells are likely subject to immunoediting, a concept developed by Dunn et al. (1) and related to immunosurveillance as initially described by Burnet and Thomas (18). Indeed, we illustrate that tumor development not only involves mutations within classical proto- or anti-oncogenes, but also active processes targeted to unanticipated genes that have no effect on the transformation state *per se* - such as the presently identified MelARV gene. In combination with classical chemo- or radiotherapies, targeting such genes by any available mean -including the injection of unvectorized siRNA- may contribute to tumor regression by restoring the natural ability of the immune system to reject tumor cells, and therefore contribute to cancer treatment. Along this line, it is of interest that a first series of experiments using synthetic siRNA targeted to MelARV, and injected intraperitoneously 12 days after engraftment of B16 cells into immunocompetent mice, actually resulted in a 1/3 inhibition of tumor growth as compared to mice injected with control siRNA and, as illustrated in the figures 6, in a moderate -but reproducible-increase in survival delay. Finally, it is noteworthy that in humans the expression of ERV env genes, mainly restricted to placenta and testis in normal tissues, can be observed in several tumor types such as seminomas and melanomas (8, 19, 20). Moreover, several of the fully coding env genes that can be identified in the human genome are immunosuppressive *in vivo* in a mouse model assay (5), and thus might act as the MelARV env gene to promote tumor escape.

### Abstract

Tumor development is a multistep process in which both genetic and epigenetic events cooperate for the emergence of a malignant clone. The possibility that endogenous retroviruses promote the expansion of a neoplastic clone by subverting immune surveillance has been proposed, but remained elusive. Here we show that knocking down -by RNA interference- an endogenous retrovirus spontaneously induced in the B16 murine melanoma results in the rejection of the tumor cells in immunocompetent mice, under conditions where control melanoma cells grow into lethal tumors. The knockdown does not modify the transformed phenotype of the cells, as measured both in vitro by a Soft agar assay and *in vivo* by tumor cell proliferation in immune incompetent (X-irradiated and SCID) mice. Tumor rejection can be reverted upon adoptive transfer of regulatory T cells from control melanoma-engrafted mice, as well as upon re expression of the sole envelope gene of the endogenous retrovirus in the knocked down cells, These results demonstrate that endogenous retroviruses can be essential for a regulatory T cell mediated subversion of immune surveillance and could be relevant to human tumors where such elements -and especially their envelope gene- are induced.

### References for Example 1

1. Dunn et al. Nat Immunol 2002;3: 991-998.
2. Garcia-Lora et al. J Cell Physiol 2003;195: 346-355,
3. Smyth et al. Immunity 2003;18: 1-6.
4. Mangeney et al. Proc Natl Acad Sci USA 1998;95: 14920-14925.
5. Mangeney et al. J Gen Virol 2001 ;82: 2515-2518.
6. Lower et al. Proc Natl Acad Sci USA 1996;93: 5177-5184.
7. Stoye et al. Curr Biol 2001;11: R914-6.
8. Bieda et al. J Gen Virol 2001 ;82: 591-596.
9. Li et al. J Virol 1999;73: 9178-86.
10. Miller et al. Biotechniques 1989;7: 989-90.
11. Brummelkamp et al. Science 2002;296: 550-3.
12. Blaise et al. J Virol 2004;78: 1050-1054.
13. Cianciolo et al. J Exp Med 1984;1 S9: 964- 969.
14. Sutmuller et al. J Exp Med 2001;194:823-32.
15. Turk et al. J Exp Med 2004;200: 771-82.
16. Dittmer et al. Immunity 2004;20: 293-303.
17. He et al. J Virol 78: 11641-7, 2004.
18. Burnet et al. Brit Med J 1: 841-847,1957.
19. Muster et al. Cancer Res 63: 8735-41, 2003.
20. Schiavetti et al. Cancer Res 62: 5510-5516,2002.

### EXAMPLE 2: A recombinant endogenous retrovirus amplified in a mouse neuroblastoma is involved in tumor growth in vivo

Abbreviations: dsRNA, double-stranded RNA; gfp, green fluorescent protein; MelARV, melanoma-associated retrovirus; MLV, murine leukemia virus; NeRV, Neuro-2a-associated retrovirus; RT, reverse transcriptase; siRNA, small interfering RNA

We demonstrate that the Neuro-2a tumor -a spontaneous A/J mouse neuroblastoma- produces a retrovirus of endogenous origin that we characterize. Knocking down -by RNA interference- the expression of this retroviral element results in delayed tumor growth and prolonged animal survival, without any effect on the transformed phenotype of the cells. These results identify an immune system-dependent role of endogenous retroviruses in tumorigenesis.

### Abstract

The theory of immunoediting postulates that tumor cells exhibit a reduced immunogenicity to escape eradication by the host immune system. It has been proposed that endogenous retroviruses -provided that they are active- could play a role in this process, via the immunosuppressive domain carried by their envelope protein. Here, we demonstrate that the Neuro-2a tumor cell line -originating from a spontaneous A/J mouse neuroblastoma- produces an infectious retrovirus which most probably results from a recombination event between two mouse endogenous retroviral elements. This Neuro-2a-associated recombinant retrovirus derives from the unique ecotropic provirus located at the Emv-1 locus, but with a gag sequence conferring B-tropism, thus allowing its high-level amplification in Neuro-2a cells. We show that knocking down -by RNA interference- this endogenous retrovirus in Neuro-2a cells has no effect on the transformed phenotype of the cells, but results in delayed tumor growth and prolonged animal survival, following engraftment of the cells into immunocompetent mice. Recombination between endogenous retroviruses, amplification of the resulting element, and high-level expression of its immunosuppressive activity are therefore likely steps of an immunoediting process leading to an invading tumor.

### Introduction

Although several evidences support the notion of cancer immunosurveillance in mice and humans, (1, 2) some individuals with an intact immune system still develop cancer. This can be explained by a failure of the host immunity to eradicate specifically the transformed cells. The term "cancer immunoediting" has been coined to emphasize the interplay between the host immune system and the tumor, that sometimes lead to "tumor escape"(2, 3). The molecular and cellular basis of this process is not yet elucidated. The high incidence of spontaneous tumors in some inbred mouse strains has been related to the sustained expression of endogenous murine leukemia viruses (MLVs) early in life (4). It is now well established that these elements can play a role in the transformation process via insertional mutagenesis (5). They could also be involved not in the transformation process per se but in tumor progression in vivo, by subverting the immune response. Indeed, the envelope protein of retroviruses -including the MLVs- possesses an immunosuppressive activity, which can be revealed by an in vivo assay (6-8). Furthermore, we have recently shown that an endogenous retrovirus specifically produced by a murine melanoma is absolutely required for tumor growth in immunocompetent mice, precisely because its immunosuppressive activity subverts host immunosurveillance. Indeed, in this melanoma tumor model, knocking down -by RNA interference- the endogenous retrovirus resulted in the rejection of the engrafted tumor cells, without any alteration in the transformed phenotype of the cells (9).

We analyze another class of spontaneous mouse tumors, i.e. neuroblastoma, using the Neuro-2a cell line as a model (10), to determine whether endogenous retroviruses are similarly involved in the tumor process. We show that the Neuro-2a neuroblastoma expresses a functional endogenous retrovirus. We demonstrate -by an RNA interference approach- that this expression, as observed for the melanoma tumors, is involved in tumor growth in vivo. Furthermore, we present evidence that this endogenous retrovirus most probably results from a recombination event between the N-tropic provirus located at the Emv-1 genetic locus and a B-tropic endogenous MLV, allowing amplification of the recombinant element in cells of A/J mouse origin. These genetic events and the resulting high-level expression of endogenous retroviruses exerting an immunosuppressive activity could contribute to the immunoediting process.

### A Materials and Methods

### Mice and cell lines

A/J mite, 8-12 weeks old, were obtained from Harlan (Gannat, France). Neuro-2a (a murine neuroblastoma cell line of A/J origin; ATCC CCL-131) and NIH/3T3 (ATCC CRL-1658) cells were maintained in DMEM supplemented with 10% heat-inactivated foetal calf serum and antibiotics. Melan-a (11) melanocytes (a gift from L. Larue, Institut Curie, Orsay) were maintained in RPMI 1640 supplemented with 10 % heat-inactivated foetal calf serum, antibiotics and 12-O-tetradecanoylphorbol-13-acetate (TPA, 200 nmol per liter) in a 10 % CO₂ atmosphere.

### Southern blot analysis

Genomic DNA from A/J mouse liver and Neuro-2a cells was digested to completion with EcoRI, HindIII or PvuII (Invitrogen, Cergy Pontoise, France), and 30 µg of DNA were loaded and separated on 0.7 % agarose gels. Following transfer, membranes were hybridized overnight at 65°C in 7 % SDS, 1 mmol of EDTA per liter, and 0.5 mmol of Na₂HPO₄ (pH 7) per liter, using a ³²P-labeled env (nt 6006-6514) fragment specific for ecotropic retroviruses as described in references (12, 13) and obtained by PCR amplification using pDFGMelARVenv (9) as a template and primers pEcB4F/R (Table 1). The blots were then washed twice with 2X SSC plus 0.1% SDS at 65°C for 15 min. Labeling was first detected using a PhosphorImager (FLA-3000 scanner, Molecular Dynamics) and membranes were finally exposed to X-ray film at - 80°C for 2 weeks.

### Emv-1 provirus characterization

Identification of the Emv-1 flanking DNA was performed by inverse PCR. Five µg of genomic DNA from A/J mice were digested to completion with PvuII (Invitrogen, Cergy Pontoise, France). DNA was purified by phenol extraction, and then self-ligated in 500 µl with 1200 U of T4 DNA ligase (New England Biolabs, Hitchin, England). DNA was then used as a template for an inverse PCR with the Expand Long Template System (Roche, Penzberg, Germany) with buffer 1 or 3 according to the manufacturer's instructions. After an initial denaturation step at 93°C (2 min), we carried out 45 cycles of amplification (10 s at 93°C, 30 s at 65°C, 15 min at 68°C) with divergent primers (Table 1). The PCR product was isolated by electrophoresis on agarose gels, purified (Nucleospin extract, Macherey-Nagel, Düren, Germany) cloned into the pGEM-T easy vector (Promega, Charbonnières-les-bains, France), and sequenced using the BigDye terminator v1.1 cycle sequencing kit (Applied Biosystems, Foster City, CA) with the standard T7 and SP6 primers. The resulting sequence was mapped to A/J mouse chromosome band 5qB1 (chr5: 28664818) using the UCSC genome bioinformatic (http://genome.ucsc.edu/cgi-bin/hgGateway) web site, which allowed the design of 5' and 3' flanking primers to amplify the complete Emv-1 provirus as two overlapping fragments, using two internal primers (Table 1). The Emv-1 provirus was then entirely sequenced as described below for the cDNAs of the Neuro-2a virus.

### Isolation and sequencing of viral RNA

Viruses were obtained by ultracentrifugation of the supernatants collected from 107 Neuro-2a cells, in an SW41 Beckman (Fullerton, CA) rotor (150,000 X g, 1 hour, 4°C). Viral RNA was isolated using the RNeasy RNA isolation micro Kit (QIAGEN, Courtaboeuf, France). cDNA was obtained as described (14). Ten sets of primers (Table 1) were designed to produce overlapping amplicons. The PCR reaction was performed with the Expand Long Template System (Roche, Penzberg, Germany) with buffer 2 according to the manufacturer's instructions. After an initial denaturation step at 93°C (2 min), we carried out 55 cycles of amplification (10 s at 93°C, 30 s at 63°C, 1 min at 68°C). The PCR products were separated by electrophoresis on agarose gel, purified (Nucleospin extract, Macherey-Nagel, Düren, Germany) and directly sequenced using the BigDye terminator v1.1 cycle sequencing kit (Applied Biosystems, Foster City, CA) and the primers used for the PCR.

### Reverse transcriptase (RT) activity assay

RT activity assay was performed using genomic RNA of bacteriophage MS2 (Roche Diagnostics, Mannheim, Germany) as a reverse transcription template, and the synthetic oligonucleotide RT-1 (5'-CCATAGGTCAAACCTCCTAGGAAG-3') as a primer, as described (15). Primer/template was prepared by mixing MS2 RNA (0.28 pmol) with primer RT-1 (9 pmol), heating at 95°C for 5 min, annealing at 37°C for 30 min, and chilling the mixture on ice for 5 min. Then the test sample for measuring RT activity or 50 U of Moloney murine leukemia virus RT (Applied Biosystems, Foster City, CA) used as a positive control, was added to the reaction mixture. Reverse transcription was carried out at 37°C for 5 hours. The cDNAs obtained were then amplified by PCR with primers RT-2 (5'-TCCTGCTCAACTTCCTGTCGAG-3') and RT-1. Finally the product was analysed by electrophoresis on 2 % agarose gels.

### Neuro-2a retrovirus protein assay

Analysis of Neuro-2a viral protein expression was performed by Western blot analyses on viral preparations. After ultracentrifugation, viral pellets were resuspended in lysis buffer (62.5 mmol of Tris-HCl (pH 6.8) per liter, 715 mmol of β-mercaptoethanol per liter, 2 % SDS, 10 % glycerol and 0.01 % bromophenol blue) boiled for 10 min at 95°C, separated by SDS-PAGE, membrane-blotted and revealed with anti-Env and anti-Gag goat antisera raised against Rausher leukemia virus gp70 SU and p30 CA (Viromed Biosafety Labs, New Jersey) using the chemiluminescence kit (SuperSignal West Pico Chemiluminescent Substrate, PIERCE, Rockford).

### Neuro-2a particle infectivity assay

To study the Neuro-2a particle infectivity, 2x10⁵ melan-a cells (a melanocytic cell line of C57BL/6 mouse origin susceptible to B-tropic MLV infection) (16) were exposed to culture medium (control) or Neuro-2a supernatant filtered through 0.45 µm-pore-size membranes, in the presence of 4 µg of Polybrene/ml for 24h. The medium was then replaced with the normal melan-a culture medium. Five days later, cells were lysed with MacDougal Buffer (20 mmol Tris-HCl (pH 8) per liter, 120 mmol of NaCl per liter, 0.2mmol of EGTA per liter, 0.2 % sodium deoxycholate, 0.5 % Nonidet P-40) containing a protease inhibitor cocktail (complete mini; Roche Diagnostics, Mannheim, Germany). Expression of retroviral Gag proteins by the target cells was analyzed by Western blotting as described above.

### Establishment of NeRV KD tumor cells

We constructed plncxH1 expression vectors derived from the plncx (17) and the pSUPER (18) vectors, to generate short transcripts directed against the Neuro-2a retrovirus NeRV (targeting the genomic transcript within the gag sequence; nt positions 1220-1238 from the gag start codon), or against the green fluorescent protein (GFP) encoding gene transcript (nt positions 215-233 from the start codon) as a control. The vectors were obtained by first inserting annealed 64-mer oligonucleotides into pSUPER opened at the BgIII and HindIII sites, followed by introduction of the BamHI-HindIII fragment from these constructs into plncx opened at the corresponding sites. These vectors were then used to obtain the NeRVKD (NeRV "Knock Down") and control Neuro-2a cell populations by transfection with 2 µg of each vector using the lipofectamine transfection Kit (Invitrogen, Cergy Pontoise, France). Cells were maintained in selective medium (3 mg/ml neomycin) for three weeks.

### Tumor progression in vivo

For in vivo assays, tumor cells were detached from tissue culture flasks using trypsin-EDTA (Invitrogen, Cergy Pontoise, France), washed three times with PBS, and subcutaneously inoculated into a shaved area of the right flank of the mice. In some experiments, synthetic siRNA targeted against the 19 nt of NeRV and control (GFP) sequences referred to in Fig. 10 were purchased from MWG Biotech (Ebersberg, Germany). They were injected intraperitoneously (0.2 nmol of siRNA in 500 µl of PBS) every 2-3 days after the tumor was established. Tumor appearance was determined by palpation and tumor area was calculated by multiplying two perpendicular diameters.

### Statistical analysis

Statistics were performed using the NCSS 2004 software (Gerry Hintze, Kaysville, Utah). Comparisons for quantitative variables were performed by using repeated measure ANOVA, including siRNA targeted against NeRV or control GFP as a "between" factor and time as a "within" factor. Differences between groups according to time were tested by the interaction term. Survival analysis was performed using Kaplan-Meier curves and the logrank test.

**Table 1. Sequences and positions of the primers used**

| | Primers sequences (5'-3') |
|---|---|
| Primers for *Emv-1* 3' flanking DNA | R : 6403-TGGGGTTGCCTGGTCTGAGGTTA-6381 |
| amplification^{a} | F : 8250-TCCAATAAAGCCTTTTGCTGTTGCA-8274 |
| | |
| Primers for *Emv-1* provirus | Flanking F1 : CGCAGCACCTGGAGACAG |
| amplification^{a} | Internal R1 : 4749-GCTTGCCTTGGAAGACCC-4732 |
| | Internal F2 : 3664-TCCTAACTGCCCCCGC-3679 |
| | Flanking R2 : TGGTCCCTTCCTTCCACC |
| | |
| Primers for NeRV cDNA amplification^{b} | F1: 1-GCGCCAGTCCTCCGATA-17 |
| | R1 : 1038-GGGGGTAAGAGCAGGGTAA-1020 |
| | F2 : 836-AGGTGTTCTCTCCTGGTCCC-855 |
| | R2 : 1923-GATCCTTTCGGCTTCTCTCA-1904 |
| | F3 : 1793-CTGGGCAAGAAACGAATGTAT-1813 |
| | R3 : 2816-TCCTGTGACATGGGGTATTGT-2796 |
| | F4 : 2685-GCTTTCCGACTTTCCCCA-2702 |
| | R4 : 3775-GCCAAGGTCCCAGTTTTTG-3757 |
| | F5: 3662-TCCTAACTGCCCCCGC-3677 |
| | R5 : 4747-GCTTGCCTTGGAAGACCC-4730 |
| | F6 : 4636-ACCCCATATACGCCTGCC-4653 |
| | R6 : 5630-CGCACCCACACGGAGTC-5614 |
| | F7 : 5484-TAATAGCCCATCTCTCCAAGC-5504 |
| | R7 : 6397-GTTGCCTGGTCTGAGGTTAGA-6377 |
| | F8 : 6136-AGGAGCCCCTGACTTCATATAC-6157 |
| | R8 : 7176-CTCGTCTTTCAAATTGGTGGTA-7155 |
| | F9 : 7057-CTCCCTGTATCTCAACCACCA-7077 |
| | R9 : 8149-CAGAAGCGAGAAGCGAGC-8132 |
| | F10 : 7881-GCATGGGAAAATACCAGAGC-7900 |
| | R10 : 8273-TGCAACAGCAAAAGGCTTT-8255 |
| | R11 : 150-CCCCCAAATGAAAGAC-135 |
| | |
| Primers for ecotropic *env* probe*^{b}* | pEcB4F : 6006-CTCTGTATGTTGGCCCTCC-6024 |
| | pEcB4R: 6514-GAGACGTATAGGCGCAATC-6496 |

| | |
|---|---|
| ^{a} Primers positions are numbered taking the first position of the 5' LTR Emv-1 R domain as position 1. ^{b} Primers positions are numbered taking the first position of the 5' LTR NeRV R domain as position 1. | |

### B Characterization of a Neuro-2a-associated retrovirus

As shown in the Southern blot of Fig. 7, hybridization with an ecotropic MLV-specific sequence (nt 6006-6514, from references 12, 13) reveals a single band in the DNA of normal liver cells from A/J mice, but numerous bands in that of Neuro-2a tumor cells. This is consistent with strong amplification in Neuro-2a cells of the ecotropic provirus detectable in A/J mouse genomic DNA (Fig. 7). Such amplification most probably results from the on-going retrotransposition and/or self-infection of the cells by the identified provirus copies, which might therefore still be active. Accordingly, we assayed the supernatants from the Neuro-2a cells for ReverseTranscriptase (RT) activity. Supernatants from NIH/3T3 cells infected or not with a Moloney murine leukemia retrovirus were used as positive and negative controls, respectively. Particulate material in cell-free supernatants from 10⁷ cells was concentrated by ultracentrifugation, and RT activity was analyzed as described in Pyra et al. (15). As shown in Fig. 8A, supernatants from Neuro-2a cells contain a detectable RT activity, which is found to be Mn²⁺⁻dependent. The supernatants were then analyzed for the presence of MLV-specific Env and Gag retroviral proteins by Western blots, using anti-Env and anti-Gag goat antisera. As shown in Fig. 8B, fully processed Gag and Env proteins can be detected, consistent with their incorporation within bona fide retroviral particles. We named this retrovirus NeRV for Neuro-2a-associated RetroVirus. NeRV particles were further characterized by an infectivity assay, using murine melan-a cells as a target (see scheme in Fig. 8C). Consistently, analysis of the target cells 6 days after infection by the NeRV particles contained in the Neuro-2a cell supernatant revealed viral transcripts (by RT-PCR using primers for the ecotropic specific env sequence; data not shown) and retroviral proteins (see Western blot for Gag in Fig. 8C), both not observed in mock-infected cells. Altogether, these results show that Neuro-2a cells contain sequences that generate retroviral proteins and infectious particles. To characterize further these particles, we sequenced their RNA content. Viral RNA was reverse-transcribed in vitro, and the cDNA was PCR-amplified using a series of primers to generate overlapping amplicons (see Materials and Methods) and sequencing was performed directly, without cloning. The resulting sequence (Genbank accession number to be assigned, and see Fig. 9A) discloses open reading frames for the three gag, pol and env retroviral genes, with the canonical organization of MLV retroviruses, and the ecotropic signature of the env gene (nt 5838-6727). This result is consistent with the release of infectious NeRV particles by Neuro-2a cells.

As previously shown (Jenkins et al.(19) and Southern blot in Fig. 7), A/J mice possess a unique ecotropic provirus, named Emv-1 (19), which is therefore a likely candidate as the progenitor of the multiple ecotropic sequences found in the Neuro-2a cells and released viral particles. However, its full nucleotide sequence had not been previously determined. We therefore characterized the Emv-1 locus of the A/J strain by using an inverse PCR strategy allowing identification of the provirus integration site and determination of its complete nucleotide sequence (Genbank accession number to be assigned). Comparison of the Emv-1 and NeRV sequences shows only 0.9 % divergence at the nucleotide level (76/8275), most of the differences between the two sequences being located in the gag gene (Fig. 9). The gag gene encodes the viral capsid protein that contains the determinants for N- and B-tropism (20). Interestingly, Emv-1 contains, at codon positions 323-324, the QR amino acids characteristic of the capsid protein of N-tropic viruses, whereas NeRV instead contains the PE amino acid pair specific for B-tropic viruses (Fig. 9B). These results strongly suggest that NeRV originates from a recombination event between the whole Emv-1 sequence and a segment of the gag sequence derived from another MLV retrovirus with B-tropism. A/J mice are Fv1^{b/b} (21) and, as such, are resistant to the replication of N-tropic retroviruses but permissive for that of B-tropic retroviruses. Hence, the data suggest that the recombination event has been the molecular basis for the amplification of an active Emv-1-derived retrovirus in Neuro-2a neuroblastoma cells.

### Role of the Neuro-2a-associated retrovirus on tumor cell growth in vivo

To demonstrate the role of NeRV in tumor growth in vivo, we used an RNA interference approach based on the stable expression in Neuro-2a cells of vectors producing short double-stranded RNA (dsRNA) directed against the viral genome (or the irrelevant gfp gene as a control, Figs. 10A and 10B). Fig. 10C clearly shows that the retrovirus-specific dsRNA expression vector almost completely abolished NeRV expression in stably transfected Neuro-2a cells (named NeRVKD for NeRV "Knock Down" cells), with a >10-fold reduction in the amount of both the Env and Gag viral proteins as compared to control cells transfected with the gfp-specific dsRNA expression vector (control cells). Then, we explored the impact of NeRV knockdown on tumor progression by injecting subcutaneously A/J immunocompetent mice with control or NeRVKD cells (2x10⁵ cells per mouse). As expected, nodules started to become detectable as soon as day 18 post-injection of control Neuro-2a cells, and rapidly grew into large tumors in most of the animals with lethality occurring after day 27 (Figs. 10D and 10E). Upon injection of NeRVKD cells, tumors were also observed, but of reduced size and in a smaller fraction of the engrafted mice. Slower tumor growth was consistently accompanied by prolonged animal survival (Fig. 10E). In parallel, NeRVKD and control cells were injected into immunocompromised A/J mice. As illustrated in figure 10D (inset), both cell populations displayed similar growth rates in X-irradiated mice, thus demonstrating that expression of NeRV has no impact on the transformed phenotype per se. Altogether, the results indicate that NeRV expression is involved in tumor growth via an immune system-dependent activity, most probably related to the same immunosuppressive activity as that previously described for another endogenous retrovirus (9) namely the melanoma-associated retrovirus MelARV, (22, 23) found to be expressed at a high level in the B 16 melanoma tumor model (9, 22, 23).

Finally, to determine if the extinction of NeRV could induce tumor regression by restoring the natural ability of the immune system to reject tumor cells, we directly injected synthetic siRNAs, directed against the same sequences as those targeted by the dsRNA expression vectors described above. These synthetic siRNAs were injected into immunocompetent mice 6 days after engraftment of Neuro-2a cells, i.e. at a time when the animals had already developed detectable tumors. Injections of siRNAs were then repeated at intervals of 2-3 days. In this context, as illustrated in Fig. 11, an inhibition of tumor growth of at least 45 % could be observed with the siRNAs directed against the NeRV sequence, as compared to control siRNAs.

Using the Neuro-2a cell line as a model of spontaneous murine neuroblastoma, we show that a tumor cell-expressed endogenous retrovirus, that we fully characterize here at the molecular level, plays an essential role in the tumorigenic process by significantly reducing the antitumor immune response of the mouse. Indeed, knocking down the expression of the Neuro-2a-associated retrovirus by RNA interference, using either stable expression vectors for double-stranded RNAs or direct i.p. injection of synthetic siRNAs, both directed against the viral transcript, results in reduced tumor cell growth in vivo and enhanced survival of the mice. The knockdown effect is specific as it is not observed using irrelevant double-stranded RNAs, and it affects neither the intrinsic growth properties nor the transformed phenotype of Neuro-2a cells (identical tumor growth rates in X-irradiated mice). Consistent with the nature of the Neuro-2a-associated retrovirus (see below) whose immunosuppressive domain is 100% identical to that of MLV retroviruses previously tested (6, 9) the above effect should be mediated by the immunosuppressive activity of the NeRV envelope protein. The latter can indeed be easily detected by Western blotting in Neuro-2a cell supernatants, and is also responsible for the demonstrated infectivity of NeRV particles.

Direct involvement of an endogenous retrovirus in tumor growth in vivo is a rather unanticipated result if one considers that the observed effect is not relevant to any direct alteration of the cell genome -for instance, by insertional mutagenesis- but rather depends on epigenetic modulation of tumor-host interactions mediated by the sole expression of an immunosuppressive activity by the tumor. Actually, the present results closely parallel those that we have previously obtained for a mouse melanoma, where it could be shown that the envelope protein of an associated endogenous retrovirus (MelARV) (23) -disclosing 98.8% (680/688 Aa) identity with that of NeRV - is also involved in tumor cell growth (9). Consequently, the finding of a common role of endogenous retroviruses in two different classes of mouse tumors strongly suggests that these elements might be essential players in the tumorigenic process. This assumption is not totally unexpected if one keeps in mind that endogenous retroviruses and "classical" infectious animal retroviruses are closely related biological entities (24-26), the latter being unambiguously directly involved in the generation of pathologies, with evidences for dysfunctions of the host immune system (27). This relationship therefore most probably goes far beyond similarities in gene organization and replicative cycles, and extends to the mechanisms of their pathological effects. Along this line, it is noteworthy that endogenous retroviruses can be found in all mammals, and in particular in humans, where elements with intact env genes disclosing an immunosuppressive activity have been identified (8). It is therefore tempting to speculate that such elements might similarly be involved in tumorigenesis in humans. Futhermore, it has been shown that several human endogenous retroviruses are transcriptionnally active in some tumors, such as germline tumors and melanoma, with evidence in some cases for expression of virus-like particles (28-30).

Another important outcome of the present study concerns the molecular characterization of the NeRV virus expressed by Neuro-2a cells, which provides hints to "reconstruct" a molecular history leading to the generation of the fully transformed, invading Neuro-2a tumor. Indeed, the complete sequencing of the viral NeRV RNA shows that this element has most probably been generated by a recombination event, a process which is a rather common feature for infectious retroviruses. This process most probably involves co-packaging of two distinct viral transcripts within a single retroviral particle, and jumps of the reverse transcriptase between strands in the course of viral replication (31-33). Interestingly, in the present case we could unambiguously identify the genomic progenitor of NeRV, namely the unique ecotropic MLV copy that can be found in the A/J mouse genome -see Southern blot in Fig. 7- and which corresponds to the Emv-1 locus in reference 19. Furthermore, complete sequencing of this element allowed us to map the recombination event for the generation of the NeRV virus within the gag gene, with the acquisition of part of the gag sequence from a B-tropic MLV. This molecular event has most probably been of major importance, since it converted a copy of the unique endogenous ecotropic retrovirus of the A/J mouse genome from an N-tropic to a B-tropic retrovirus, thus allowing retrotransposition and/or re-infection within the N-tropic-restricted A/J mouse cells. Accordingly, Neuro-2a cells now both disclose a high load of ecotropic MLV copies (see Fig. 7) and express at a detectable level active MLV particles (Fig. 8). Although the primary tumor is not available -and consequently it remains difficult to unambiguously assess whether amplification took place within the primary tumor and not during in vitro cultivation-, the above-mentioned molecular events are likely to have played a major role in the tumor history. Along these lines, it is of note that in the parallel study mentioned above that was carried out on a C57BL/6 mouse melanoma -where the B-tropic ecotropic MLV, MelARV, was also found to be expressed at a high level (22, 23) a change in virus tropism has also been observed, associated with its amplification and acquisition of an envelope protein-associated immunosuppressive activity by the tumor (9).

In conclusion, the present example both emphasizes the role of endogenous retroviruses in tumorigenesis, via an unanticipated effect of their immunosuppressive activity acting on the host immune system, and dissects important molecular events that have precisely made it possible for this effect to fully develop. In this respect, the Neuro-2a neuroblastoma may constitute a valuable model of "immunoediting", and opens important perspectives for the study of human tumors, for which there is already evidence that at least the expression of defined endogenous retroviruses is induced in some primary tumors in situ (reviewed in references 24 to 26).

### References for Example 2

1. Smyth et al. 2001;2:293-9.
2. Dunn et al. Immunity 2004;21:137-48.
3. Dunn et al. Nat Immunol 2002;3:991-8.
4. Massey et al. J Virol 1994;68:3773-83.
5. Mikkers et al. Adv Cancer Res 2003;88:53-99.
6. Mangeney et al. Proc Natl Acad Sci USA 1998;95:14920-5.
7. Blaise et al. J Gen Virol 2001;82(7):1597-600.
8. Mangeney et al. J Gen Virol 2001;82(10):2515-8.
9. Mangeney et al. Cancer Res 2005;65:2588-91.
10. Sandler et al. Cancer Res 2003;63(2):394-9.
11. Bennett et al. Int J Cancer 1987;39(3):414-8.
12. Chan et al. Proc Natl Acad Sci USA 1980;77(10):5779-83.
13. Chattopadhyay et al. Proc Natl Acad Sci USA 1980;77(10):5774-8.
14. de Parseval et al. J Virol 2003;77(19):10414-22.
15. Pyra et al. Proc Natl Acad Sci USA 1994;91:1544-8.
16. Li et al. Int J Cancer 1998;76(3):430-6.
17. Miller et al. biotechniques 1989;7(9):989-90.
18. Brummelkamp et al. Science 2002;296(5567):550-3.
19. Jenkins et al. J Virol 1982;43(1):26-36.
20. Goff et al. Mol Cell 2004;16:849-59.
21. Pincus et al. J Exp Med 1971;133(6):1219-33.
22. Li et al. Cancer Res 1996;56(19):4464-74.
23. Li et al. J Virol 1999;73(11):9178-86.
24. Lower et al.. Proc Natl Acad Sci USA 1996;93(11):5177-84.
25. Bannert et al. Proc Natl Acad Sci USA 2004;13:14572-9.
26. de Parseval et al. Cytogenet Genome Res 2005;110(1-4):318-32.
27. Dittmer et al. infection. Immunity 2004;20(3):293-303.
28. Bieda et al. J Gen Virol 2001;82:591-6.
29. Muster et al. Cancer Res 2003;63(24):8735-41.
30. Buscher et al. Cancer Res 2005;65:4172-80.
31. Hu et al.. Science 1990;250(4985):1227-33.
32. Zhang et al.. J Virol 2000;74(5):2313-22.
33. Telesnitsky et al. New York: Cold Spring Harbor Laboratory Press, 1997:121-60.

### EXAMPLE 3: Immunosuppressive activity of the env gene encoded Np9 protein of HERV-K 101 provirus

### A Cloning of the genes encoding Np9

Np9 was PCR-amplified with high-fidelity Pfx Platinum polymerase (Invitrogen), using phCMV K108-Env as template (Dewannieux et al. 2005 (J Virol. 2005;79(24):15573-7) and the following primers:
5'- ata cat ctc gag atg aac cca tcg gag atg caa aga aaa ggg cct cca cgg aga tgt ctg cag gtg tac cca aca gc-3' and
5'-atg tat acg cgt tta aca cat aac aaa atg -3'

The amplification product was purifed, digested with Xho-I and Mlu-I and ligated into the phCMV vector (Blaise et al. 2003, PNAS, 100(22): 13013-18) opened with the same enzymes.
phCMV-Np9 sequence (SEQ ID NO: 1), with Np9 ORF underlined

### B Downregulation of HLA class-I expression induced by by 293T cells cotransfected Np9

As it can be seen on figures 13 and 14, HLA class-I expression is downregulated by 293T cells cotransfected with Np9 expressing vectors and HLA-A2, -B27, -Cw3 expression vectors.

The figure demonstrates the downregulation of HLA class I expression by 293T cells cotransfected with 5 µg of Np9 expression (phCMV-Np9) vector and 3 µg of HLA-A2, HLA-B27 or HLA-Cw3 (all three as EcoRI genomic fragments in pBR 328) expression vectors. HLA class I expression was measured by FACS with PE-coupled anti-human MHC-I antibody W6/32 (eBioscience) 72h post-transfection.

So, these results demonstrate that the expression of cnv gene HERV-K encoded Np9 protein induces down regulation of MHC class I antigens, a characteristic feature of tumor cells allowing them to escape immune surveillance.

### EXAMPLE 4: Phoenix, an infectious progenitor for the mobile HERV-K(HML2) human endogenous retroviruses

### INTRODUCTION

Nearly 8 % of the human genome is composed of sequences of retroviral origin. Most of them are degenerate, either due to recombination between the two provirus LTRs, or to mutations interrupting the retroviral Open Reading Frames (ORFs). The env gene seems to be best conserved -with 18 intact genes retaining a full coding capacity in the human genome (1-3)-, possibly because of its potential roles in human physiology. The HERV-K(HML2) family of endogenous retroviruses is an exception to this general rule since some copies still contain complete ORFs for the other retroviral genes (4, 5). This family includes the most recently amplified endogenous retroviruses, most of which have integrated into the genome less than 5 million years ago, with a few insertions showing polymorphism within the human population (6-11). Some of these recently integrated proviruses are responsible for the synthesis of retroviral particles that can be observed in teratocarcinoma and melanoma-derived cell lines (12-16), and possibly in human placenta (17-19). Because of this "activity", the HERV-K(HML2) family has been the subject of numerous studies in the past years, with the description of alleles with nearly intact proviruses and complete coding capacity (9, 20, 21). Despite these efforts, no functional provirus able to produce infectious particles has yet been described. Even if several loci containing complete ORFs have been identified and in spite of the availability of the complete sequence of the human genome, the search for a functional provirus is hampered by the significant polymorphism that can be detected at each HERV-K(HML2) locus within the human population. To overcome this limitation, we used a reverse strategy and generated a consensus HERV-K(HML2) provirus, thus "resuscitating" Phoenix, the likely progenitor of the last, human-specific HERV-K(HML2) amplification burst.

### A Materials and Methods

### In silico engineering and in vitro reconstruction of the HERV-K(HML2) consensus provirus

To generate the consensus HERV-K(HML2) provirus sequence, we took the nucleotide sequences of the 9 full-length, human-specific, 9.4-kb class proviruses that can be found in sequence databases and publications, and aligned them using ClustalW to generate the consensus sequence (see Figure 22, SEQ ID NO: 4 and Supporting Table 1). To obtain a backbone for the Phoenix provirus expression vector, we subcloned the K108 and K109 proviruses by digesting commercial BAC clones RP11-33P21 and RP11-152J15 (BACPAC Resources), and generated chimeric constructs to get closer to the consensus sequence. Fragments from these chimeric constructs were placed in pALTER-1 plasmid (Promega) and subjected to successive rounds of site-directed mutagenesis using the Altered Sites II in vitro Mutagenesis System (Promega) and appropriate primers. When all expected mutations had been introduced, the Phoenix provirus was re-assembled in the pBluescript plasmid, and we checked the whole construct by sequencing. To generate the CMV expression vector, the CMV promoter region was isolated from pCMVβ (Clonetech, EcoRI - SacI fragment) and used to replace the U3 part of the 5' LTR -using PCR- so as to keep the transcription start site unchanged.

### Supporting Table 1

| Name | GenBank | FirstPosition | Last position | Orientation |
|---|---|---|---|---|
| | Accession Number | | | |
| K104 | AC116309 | 123,567 | 114,122 | - |
| K108-1 | AC072054 | 47,417 | 37,947 | - |
| K108-2 | AC072054 | 38,914 | 29,443 | - |
| K109 | AC055116 | 139,321 | 148,740 | + |
| K113 | AY037928 | 1 | 9,472 | + |
| K115 | AY037929 | 1 | 9,463 | + |
| | Y178333 | 1 | 8,629 | + |
| | AP000776 | 101,084 | 110,549 | + |
| | AC025420 | 37,159 | 46,615 | + |

This supporting table 1 provides the GenBank coordinates of the human endogenous HERV-K copies used to generate the *Phoenix* provirus.

Point mutations within the protease and RT motifs (from LVDTGA to LVaaaA and from YIDD to aIaa, respectively) were introduced using the Altered Sites II in vitro Mutagenesis System (Promega) and appropriate primers. Inactivation of the gag gene was achieved via an in-phase deletion between the SfoI and PshAI unique sites, and the env gene was disrupted by inserting a small synthetic linker containing Stop codons in all 6 frames at the unique StuI site.

### Construction of chimeric HERV-K proviruses

To generate the chimeric HERV-K proviruses, we first constructed an expression vector for the HERV-K109 copy by replacing the U3 part of its 5' LTR by the CMV promoter region. The 3' part of this provirus was replaced by the corresponding sequences of K113 and K115 proviruses (from the unique SexAI site to the end) or K108-1 (from the SacI site to the end). For the triple chimera K109-K115-K108, the 3' domain of K109-K115 was replaced by the corresponding part of the K108-1 provirus (from the XbaI site to the end).

### Production of antibodies - Western Blot analysis and immunofluorescence studies

We developed a polyclonal anti-HERV-K Gag antiserum by immunizing rabbits with a mix of purified recombinant proteins produced in bacteria, covering the complete HERV-K113 Gag protein. The resulting sera were checked by Immunoblot analyses and affinity-purified before use (Agrobio, France). The monoclonal antibody directed against the SU subunit of the HERV-K envelope protein has already been described (26).

For analysis of retroviral protein expression and cleavage, 293T cells transiently transfected with the provirus expression vectors (using Lipofectamine and +Reagent; Invitrogen) were lysed in Laemmli buffer 48h post-transfection and lysate proteins directly separated by SDS-PAGE, followed by transfer onto nitrocellulose membranes (Schleicher and Schuell) and incubation with the appropriate antibody and with a secondary antibody linked to Horseradish peroxidase. For analysis of cell supernatants, 293T cells were transfected by the Calcium Phosphate method using the MBS commercial kit (Stratagene). Particulate material in the medium was concentrated by ultracentrifugation on a 20 % sucrose cushion 48 h post-transfection, and the virions were lysed in Laemmli buffer before viral protein analysis by SDS-PAGE and immunoblotting.

Immunofluorescence analysis of the intracellular localization of Gag and Env was carried out on 293T cells transiently transfected with the Phoenix expression vector. Cells were fixed 30 h post transfection, permeabilized and stained with a rabbit polyclonal anti-Gag serum and a mouse monoclonal anti-Env antibody. Alexa 546-conjugated goat anti-rabbit IgG, and Alexa 488-conjugated goat anti-mouse IgG were used as secondary antibodies (Molecular probes); nuclei were stained with Dapi and Topro3 (Molecular probes).

### Electron microscopy

For morphological studies, cells were fixed in phosphate buffer pH 7.2, 3 % glutaraldehyde for 1 h, and post-fixed in 0.1 M cacodylate buffer, 1 % osmium tetroxyde for 2 h. After being rinsed for 5 min in water and 15 min in 0.1 M cacodylate buffer, cells were transferred to 0.2 M cacodylate buffer for 30 min. Cells were washed in 30 % methanol for 10 min, stained in 2 % uranyl acetate in 0.1 M cacodylate buffer - 30 % methanol for 1 h, and washed in 30 % methanol. Cells were then dehydrated through a graded ethanol-propylene oxide series and embedded in Epon 812.

For immuno-electron microscopy experiments, transfected cells were fixed for 1 h at 4°C with either 4 % formaldehyde or 1.6 % glutaraldehyde in 0.1 M phosphate buffer (pH 7.3). During fixation, the cells were scraped from the plates and centrifuged. Pellets were dehydrated in increasing concentrations of methanol and embedded in Lowicryl K4M at low temperature. Polymerization was carried out for 5 days at -30°C under long-wavelength UV light. Ultrathin sections of Lowicryl-embedded material were collected on formvar-carbon-coated gold grids (200-mesh) and used for labeling with a rabbit antiserum specific for the SU subunit of the HERV-K envelope protein followed by incubation with a goat anti-rabbit IgG conjugated to gold particles, 10 nm in diameter.

For observation, sections were contrasted with 4 % uranyl acetate and lead citrate and examined with a Zeiss 902 microscope at 80 Kv.

### RT assay

Assays for the presence of RT activity in cell supernatants were performed using transiently transfected 293T cells. At day 2 post-transfection, the medium was harvested, filtered through 0.45 µm pore-sized membranes and particulate material concentrated by ultracentrifugation on a 20 % sucrose cushion. The virions were lysed in Buffer A (25 mM Tris, 0.25 mM EDTA, 50 mM KCl, 5 mM DTT, 0.025 % Triton X-100, 50 % glycerol) and used as an RT source for reverse-transcription of commercial MS2 RNA. cDNA synthesis was then assayed by PCR (PERT assay, see ref. 24). In these assays, expression vectors for the MLV and HIV core proteins were used as positive controls, and CMV-β (Clonetech) as a negative control.

### Infection and retrotransposition assays

For infection assays, 293T cells were used as producer cells, following transient transfection with the consensus provirus, using the Calcium Phosphate method (Stratagene MBS commercial kit). Target cell lines (SH-SY5Y, BHK21, G355.5, HeLa and WOP) were seeded the day before infection in 6-well plates. The day of infection, the supernatants of 293T cells were harvested, filtered through 0.45 µm pore-sized membranes and added to target cells (1 ml per well, with 4 µg/ml polybrene). Target hamster BHK21 cells are infected with the sample to be tested in the presence of polybrene (4 µg/ml), subjected to spinoculation (centrifugation 2h30, 1200 g, 25°C) and to an overnight incubation before the medium is changed. At day 2 post infection, BHK21 cells are washed with DNaseI (20 min 37°C), splitted, and the genomic DNA is recovered at day 6 post infection using standard phenol-chloroform extraction protocole. It is then assayed for integrated HERV-K proviruses by Taqman analysis. For this, 500 ng of genomic DNA are used as a template in each reaction, using the following primers (5'-GCCTTTCCCGCCCTTAATT-3' and 5'-GCAGGGCAGCGATCATCTA-3') at a final concentration of 1 µM and a MGB Taqman probe (Applied Biosystems, 6FAM-ATAGTGTATGGGTACCTGGC-MGBNFQ) at a final concentration of 0.25 µM.

For retrotransposition assays, 7.5x10⁵ SH-SY5Y cells were seeded in 60 mm dishes the day prior to transfection. Transfection was performed using 8 µl Plus Reagent (Invitrogen), 12 µL Lipofectamine (Invitrogen) and 3 µg DNA (2.5 µg of Phoenix-derived expression vector and 0.5 µg of env expression plasmid or control plasmid).

Cells were grown for one week before plating on 10 cm plates and subsequent selection with G418. The results are given as relative transposition frequencies as compared to neoTNF-marked Phoenix (whose absolute transposition frequency is 10-5 clone per seeded cell under these conditions).

### Characterization of HERV-K(HML2) de novo insertion sites

Integration sites were analyzed by inverse PCR using individual G418R clones essentially as described (30). Briefly, 5 µg of DNA from each clone was digested with PvuII and either (i) DraI, or (ii) SspI, or (iii) with a mix of XbaI, AvrII and Spel, or (iv) with ApoI alone. After enzyme removal, DNA was diluted and self-ligated overnight. It was ethanol-precipitated, dissolved in 20 µl Tris 10 mM, and subjected to one (or in some cases two) round of PCR with sets of divergent primers located within the neo gene and designed for the amplification of the 5' or 3' end and flanking sequences of the newly inserted proviruses. The amplified DNAs were gel-purified, cloned and sequenced, thus yielding the target site sequences. Primer sequences are available on request.

### Safety precautions

All experiments involving virus production and manipulation were performed in laminar-flow biological safety cabinets under BL2 containment using BL3 practices.

### B Regeneration of Phoenix, the ancestral HERV-K (HML2) retrovirus

To construct a consensus HERV-K(HML2) provirus, we assembled all the complete copies of the 9.4 kb-provirus that are human-specific (excluding those with the 292 nt deletion at the beginning of the env gene) and aligned their nucleotide sequence to generate the consensus *in silico*, taking for each position the most frequent nucleotide. The resulting provirus sequence contains, as expected, ORFs for all the HERV-K(HML2)-encoded proteins (Gag, Pro, Pol, Env and the accessory Rec protein), as well as the expected translational signals, with canonical frameshift motifs between gag, pro and pol. Noteworthily, this consensus provirus is distinct from each of the sequences used to generate it, with at least 20 amino acid changes on the overall sequences (Fig. 15).

Based on this in silico reconstruction, we then generated a molecular clone corresponding to the consensus DNA proviral sequence that we named Phoenix, using the related K108 and K109 proviruses (that we had previously cloned from a commercial human BAC library) as a backbone, and a commercial kit to introduce single nucleotide mutations at each position required to match the consensus. As the HERV-K(HML2) LTRs are not functional in every cell line (22, 23), we also replaced the U3 part of the 5' LTR by the CMV promoter, with its start site positioned so as to conserve the expected nucleotide sequence of the native retroviral transcript. We introduced also a cloning site downstream of the env gene into the noncoding U3 region of the 3' LTR, to possibly tag (with the neo gene) the retroviral transcript without altering its coding capacity.

### C Phoenix encodes for bona fide retroviral particles

In a first assay, we introduced the Phoenix expression vector into human 293T cells, and looked for retroviral particles by transmission electron microscopy. As illustrated in Fig. 16A and 16B, this resulted in the synthesis of particles budding from the plasma membrane (not observed with a control vector), with a C-type morphology. Two classes of particles could be observed, either with a hollow interior surrounded by a dense ring of stain corresponding to immature particles, or with a condensed hexagonal core corresponding most probably to the mature form of the virus (Fig. 16C), in which the polyproteins are cleaved into the final products. Consistently, a provirus with a mutation introduced into the active site of the protease (Pro) also produces particles, but all of them disclose the immature morphology (not shown). In all cases, the particles are surrounded by prominent spikes (Fig. 16D) that can be marked in immuno-electron microscopy experiments with an antibody directed against the SU subunit of the HERV-K(HML2) envelope protein (Fig. 16E). Altogether these features suggest that Phoenix can direct the synthesis of complete retroviral particles.

As a confirmation, we performed immunoblot analyses of the 293T cells transfected with the proviral vector. As illustrated in Fig. 17A, Phoenix -as well as its pro mutant- promotes the synthesis of a Gag precursor of approximately 80 kDa in cell lysates, as well as some cleaved products (not observed with the pro mutant). Cleavage products could also be observed in the supernatant of the transfected cells, except for the pro mutant that only expresses the Gag precursor. Similarly, using an antibody directed against the SU component of the HERV-K(HML2) envelope protein, we detected, in the supernatants, a protein of a size (55 kDa) consistent with that of the processed SU subunit, but we were unable to detect the Env protein in cell lysates, most probably because of a too low expression level. Notwithstanding, expression of the envelope protein could be observed by immunofluorescence analysis using HeLa and 293T cells transiently transfected with Phoenix (Fig. 17B). In these cells, it can further be observed that Gag and Env co-localize to some common subcellular domains, including, as expected, the plasma membrane.

### D Phoenix is an infectious retrovirus

To get insight into the functionality of this ancestral virus, we then looked for a reverse-transcriptase activity in the supernatant of the transfected cells. Using the previously described sensitive product-enhanced reverse transcriptase (PERT) assay (24), an RT activity could indeed be detected in the supernatant of cells transfected with Phoenix, not observed with a provirus in which we had introduced a mutation within the catalytic site of the RT domain (Fig. 17C). To study further the infectivity potential of Phoenix-derived particles, we then infected a panel of mammalian cell lines with the supernatant of 293T cells transfected with a neo-marked Phoenix provirus, and subjected them to G418 selection. G418R clones were obtained for several target cells, including hamster BHK21 cells, feline G355.5 cells and, noteworthily, the human SH-SY5Y and 293 cells, indicating that Phoenix is fully functional and infectious (Fig. 18A). In the same assay, the human HeLa cells and the murine WOP cells seemed to be resistant to infection, most probably due to the absence of the appropriate receptor for the Env protein at the cell surface. We then ensured that the G418R clones that were obtained are the result of bona fide infection. Their occurrence actually depends on the presence of intact gag, pro, pol and env genes since the inactivation of any of the encoded proteins renders the provirus-derived particles noninfectious (Fig. 18A and data not shown). Even for the cell lines permissive for infection, the apparent titer of Phoenix is quite low. A quantitative assay by real-time PCR on the genomic DNA from BHK21 cells infected with particles generated by Phoenix, marked -or not- by the neo gene, indicated that the presence of neo within the provirus is, at least in part, responsible for this low titer. Another possible explanation is that infection with Phoenix occurs more efficiently via cell-cell interaction, as observed for HTLV and in some cases HIV, consistent with some images obtained by electron microscopy where budding particles appear to be directly captured by a recipient cell (Fig. 18B).

Then, we characterized further the G418R clones that we isolated, and determined the provirus insertion sites using an inverse PCR strategy. In all cases, we found complete LTRs and 5/6 bp target-site duplications (TSD) bordering the proviruses newly inserted in human SH-SY5Y cells (Fig. 19), thus confirming that the infection process is canonical, and consistent with the structures observed for the HERV-K(HML2) endogenous copies in the human genome.

### E HERV-K (HML2) provirus amplification via re-infection

Next, to get an insight into the possible physiological mechanism responsible for the amplification of the HERV-K(HML2) family of endogenous retroviruses, we analyzed the relative efficiency of intracellular retrotransposition versus re-infection by Phoenix. To do so, we marked Phoenix with a specific neo indicator gene (neoTNF) in which neo becomes active only after the marked element has been transcribed, reverse-transcribed and integrated. The marked element was introduced by transfection into various cell lines, permissive -or not- for infection. Under these conditions, we reproductively obtained G418R clones only with the cell lines permissive for infection (i.e. the feline G355.5 cells, the hamster BHK21 cells and the human SH-SY5Y cells; Fig. 20 and data not shown). This suggests that the amplification process must, at some stage, get the envelope protein receptor involved, and thus should occur via an extracellular re-infection process. To confirm this hypothesis, we performed the same experiment as above, but with a provirus defective for the env gene, using SH-SY5Y permissive cells. Under these conditions, no amplification of the marked mutant provirus was observed. As a control, addition of an expression vector for the envelope protein restored -at least partially- the mobility of the env-defective provirus (Fig. 20). Altogether the results strongly support the hypothesis that HERV-K(HML2) amplification in the human genome took place via a re-infection process -possibly in the germline of the developing embryo-, a process consistent both with evolutionary data indicating that the HERV-K(HML2) env gene has been subjected to the same purifying selection as the other retroviral genes during amplification of this family (25), and with the presence of retroviral particles in human placenta and in germline tumors, morphologically identical to those produced by Phoenix (13, 14, 17-19).

### F "Recombination" of human endogenous HERV-K (HML2) loci can generate an infectious retrovirus

Finally, taking advantage of our present knowledge on the "ancestor" of the HERV-K(HML2) family, we asked whether an "average human genome" would still be able to generate fully infectious retroviral particles. With the proviruses that were used to assemble the consensus sequence, we constructed a set of chimeric elements that could spontaneously emerge via recombination between definite human alleles, and able to express all the retroviral genes. As described in Supporting Materials and Methods, the 5' part was "borrowed" from the HERV-K109 element, and the 3' end from K108, K113 or K115 (Fig. 21). We assayed these three proviruses for a virion-associated RT activity in the supernatant of human 293T cells following their transient transfection, and, remarkably, found that the K109/K115 chimera was positive (Figs. 17C and 21). However, this construct is not infectious on its own, most probably because of defects in the HERV-K115 env gene (which is noninfectious in pseudotype experiments, at variance with the K108 env gene, ref. 26). Therefore we generated a "triple chimera" by replacing the env region in the above construct by that from K108, and assayed it for infection. With this element, specific integration of the provirus could be detected in the BHK21 cells by quantitative PCR (not observed with Phoenix mutated in the RT domain, used as a control), at a level only 5-10 fold lower than that observed with Phoenix (Fig. 21). This indicates that, as observed in AKR mice where a multi-step recombination process reproductively generates new infectious retroviruses (27, 28), some individuals could reconstitute functional HERV-K(HML2) proviruses, either via a three-fragment recombination event, or more likely via a single recombination event between 2 HERV-K copies (e.g. K109 and K115) with complementation in trans by an intact envelope protein (e.g. K108). Recombination events have indeed occurred frequently between HERV-K(HML2) proviruses (29). By this way, or even using functional (but still uncharacterized) alleles that might be present in some individuals, the HERV-K(HML2) family could repeat its life cycle, and regenerate functional elements that could, in turn, be the initiators of amplification events to come.

The present investigation, with the re-construction of a functional HERV-K(HML2) provirus that we demonstrate to be a bona fide infectious retrovirus i) provides a molecular scenario that can account for the observed amplification of these elements in primate genomes, ii) gives access de facto to an "ancestral" active retrovirus of primates, thanks to the fact that HERVs have in a way been "frozen" by their "endogenization", which transformed their viral status into that of a host genomic iocus (thus dramatically reducing their evolutionary rate), and iii) provides an active viral element for appraisal of the role of such elements in a series of human pathologies - such as germline tumors and melanoma- where they become transcriptionally active.

Human endogenous retroviruses are the genomic traces of infections of primate ancestors by bona fide retroviruses that have been "endogenized" and transmitted in a Mendelian manner. Among the numerous families of proviral copies present in primates, the HERV-K(HML2) family is one of the most recently amplified, with evidence for polymorphism of provirus insertions among humans. Yet, none of the presently known proviral copies can generate a functional retrovirus. To identify the ancestral, "progenitor" retrovirus, we derived a consensus element *in silico*, and then constructed it. Here we show that the resulting provirus yields a genuine retrovirus, producing type C viral particles with reverse transcriptase activity and infectious for several human and non-human target cells. This ancestral retrovirus generates integrated copies in the infected cells with the signature observed for the multiple HERV-K copies currently present in the human genome, consistent with rounds of re-infections in the course of primate evolution. Moreover, we show that the distance between the consensus and these multiple copies is sufficiently small for an in vitro recombination between cloned endogenous elements to generate a provirus which also proves to be infectious. Altogether, the present results lead to the notion that human cells still have the potential to produce retroviruses, and further provide a molecular clone -Phoenix- to recapitulate the genetic events that have led to its amplification in the course of primate evolution, and to appraise its possible role in human pathologies -such as germline tumors and melanoma- where some of these elements are transcriptionally active.

### References for Example 4

1. Benit et al. (2001) J Virol 75, 11709-19.
2. de Parseval et al. (2003) J. Virol. 77, 10414-10422.
3. Villesen et al. (1996) Proc. Natl. Acad. Sci. USA 93, 5177-5184.
5. Bannert et al. (2004) Proc. Natl. Acad. Sci. U S A 13, 14572-14579.
6. Steinhuber et al. (1995) Hum Genet 96, 188-92.
7. Medstrand et al., (1998) J. Virol. 72, 9782-9787.
8. Barbulescu et al.J. (1999) Curr. Biol. 9, 861-868.
9. Turner et al., J. (2001) Curr. Biol. 11, 1531-1535.
10. Hughes et al. (2004) Proc. Natl. Acad. Sci. U S A 101, 1668-1672.
11. Belshaw et al. (2005) J Virol 79, 12507-14.
12. Bieda, K et al. (2001) J. Gen. Virol. 82, 591-596.
13. Boller et al. (1993) Virology 196, 349-353.
14. Löwer et al.. (1993) Proc. Natl. Acad. Sci. USA 90, 4480-4484.
15. Muster et al. (2003) Cancer Res. 63, 8735-8741.
16. Buscher et al. (2005) Cancer Res 65, 4172-80.
17. Kalter et al. (1973) J. Natl. Cancer Inst. 50, 1081-1084.
18. Dirksen et al. (1977) J. Nat. Cancer Inst. 59, 1187-1192.
19. Wilkinson et al. (1994) in The retroviridae, ed. Levy, J. A. (Plenum Press, New York), Vol. 3, pp. 465-535.
20. Mayer et al. (1999) Nat. Genet. 21, 257-258.
21. Reus et al. (2001) Genomics 72, 314-320.
22. Ruda et al. (2004) Virus Res 104, 11-6.
23. Lavie et al. (2005) J. Virol. 79, 876-83.
24. Pyra et al. (1994) Proc Natl Acad Sci U S A 91, 1544-8,
25. Belshaw et al. (2004) Proc Natl Acad Sci U S A 25, 25.
26. Dewannieux et al. (2005) J Virol 79, 15573-15577.
27. Stoye et al. (1991) J. Virol. 65, 1273-85.
28. Boeke et al.. (1997) in Retroviruses, eds. Coffin, J. M., Hughes, S. H. & Varmus, H. E. (Cold Spring Harbor Laboratory Press, pp. 343-435.
29. Hughes et al. (2005) Genetics 12, 12.
30. Dewannieux et al. (2003) Nat. Genet. 35, 41-48.
31. Esnault et al. (2002) Nucleic Acids Res. 30, e49.

## Claims

1. Use of a composition comprising:
- a env gene endogenous retrovirus encoded immunosuppressive protein, mutated or not, or a fragment thereof comprising an immunosuppressive domain of said env gene endogenous retrovirus encoded protein, or
- a nucleic acid encoding said env gene endogenous retrovirus encoded protein, or fragment of said env gene endogenous retrovirus encoded protein, or
- a ligand such as antibodies, said ligand being capable of specifically binding to said env gene encoded immunosuppressive protein, or
- a double stranded RNA, siRNA, shRNA, miRNA or an antisense nucleic acid targeting nucleic acid sequences of the endogenous retrovirus and/or of a protein expressed by said endogenous retrovirus,
wherein said endogenous retrovirus is a human endogenous retrovirus HERV-K
said composition
a. inhibiting the expression of said endogenous retrovirus and/or
b. inhibiting the expression, and/or lowering the concentration, and/or inhibiting the activity of said expressed protein, and/or
c. stimulating an immune response against said env gene endogenous retrovirus encoded protein
for the preparation of a drug intended for the prevention and/or the treatment of cancer in a mammal, wherein the cancerous cells of said mammal express said env gene endogenous retrovirus encoded protein.

2. Use according to claim 1, wherein HERV-K is a HERV-K sub-type **characterized in that** the open reading frame encoding the ENV viral protein encodes a Np9 protein.

3. Use to claim 1 or 2, wherein HERV-K is a HERV-K sub-type **characterized in that** said HERV-K sub-type is selected from the group consisting of the HERV-K sub-type HERV-K1 01 , HERV-K1 02, HERV-K1 07 and HERV-K(II).

4. Use according to anyone of claims 1 to 3 , wherein HERV-K is a HERV-K sub-type **characterized in that** the open reading frame encodes a Np9 protein having an amino acid sequence selected from the group consisting of:
a. the amino acid sequence SEQ ID NO: 3 or of a HERV-K sub-type Np9 protein as depicted in Genbank (GenPept) under the Accession Number, P61580, P61581, P61582 and P61583; and
b. an amino acid sequence having at least 75 % identity with an amino acid sequence of a) and having an immunosuppressive activity.

5. Use according to anyone of claims 1 to 4, wherein the expression of said endogenous retrovirus and/or of said endogenous retrovirus immunosuppressive protein, is inhibited by a double stranded RNA, a siRNA, a shRNA, a miRNA or an antisense nucleic acid targeting nucleic acid sequences of said endogenous retrovirus and/or of said endogenous retrovirus immunosuppressive protein.

6. Use according to anyone of claims 1 to 5 , wherein said protein is :
a. said immunosuppressive endogenous retrovirus protein, or a fragment thereof, comprising its immunosuppressive domain, or
b. a mutated form of said immunosuppressive endogenous retrovirus protein lacking immunosuppressive activity, or fragments thereof comprising a mutated immunosuppressive domain of said immunosuppressive endogenous retrovirus protein.

7. Use according to claim 6, wherein said mutated form of an env gene HERV-K encoded immunosuppressive protein deprived of immunosuppressive activity is capable of eliciting an immune response, cellular and/or humoral immune response
- against cells expressing the non mutated form of said env gene HERV encoded protein (cellular immune response) or
- against the non mutated form of said env gene HERV encoded protein (humoral immune response).

8. Use according to claim 1, wherein said ligand, such as antibody, is capable of specifically binding to said endogenous retrovirus env gene encoded immunosuppressive protein.

9. Use according to claim 8, wherein said ligand is an antibody, in particular monoclonal antibody, antibody fragment, such as Fab or F(ab)'₂ fragments, scFv polypeptides, chimeric antibodies, humanized antibodies, aptamers or binding peptides.

10. Pharmaceutical composition comprising as active substance a double stranded RNA, siRNA, shRNA, miRNA or an antisense nucleic acid targeting nucleic acid sequences of an endogenous retrovirus and/or of an immunosuppressive protein expressed by an endogenous retrovirus, in association with a pharmaceutically acceptable vehicle,
said endogenous retrovirus is a human endogenous retrovirus HERV-K

11. Pharmaceutical composition according to claim 10, wherein the protein is an env gene HERV encoded immunosuppressive protein, such as ENV protein or a Np9 protein.

12. Pharmaceutical composition comprising as active substance an immunosuppressive protein expressed by an endogenous retrovirus, or a fragment thereof,
said endogenous retrovirus is a human endogenous retrovirus HERV-K

13. Pharmaceutical composition according to claim 12, comprising as active substance an immunosuppressive protein of an env gene HERV-K encoded protein, mutated or not, or a fragment thereof comprising the immunosuppressive domain of said env gene HERV-K encoded protein, or nucleic acid encoding said active substance, in association with a pharmaceutically acceptable vehicle.

14. A pharmaceutical composition according to claim 13, wherein the endogenous retrovirus is a HERV-K sub-type **characterized in that** the open reading frame encoding the env viral protein encodes a Np9 protein.

15. A pharmaceutical composition according to claim 13 or 14, wherein HERV-K is a HERV-K sub-type selected from the group consisting of the HERV-K sub-type HERV-K101, HERV-K1 02, HERV-K1 07 and HERV-K(II).

16. A pharmaceutical composition according to claim 15, wherein HERV-K is a HERV-K sub-type **characterized in that** the open reading frame encoding the env viral protein encodes a Np9 protein having an amino acid sequence selected from the group consisting of
a. the amino acid sequence SEQ ID NO: 3 or of a HERV-K sub-type Np9 protein as depicted in Genbank (GenPept) under the Accession Number, P61580, P61581, P61582 and P61583; and
b. an amino acid sequence having at least 75 % identity with an amino acid sequence of a) and having an immunosuppressive activity.

17. A pharmaceutical composition according to anyone of claims 12 to 16, **characterized in that** said composition further comprises as active substance another HERV protein encoded by the nucleic sequence of said provirus HERV, or a nucleic acid encoding said another HERV protein.

18. A pharmaceutical composition according to anyone of claims 12 to 17, **characterized in that** said composition further comprises as active substance at least one protein, preferably at least two, three or all the proteins, encoded by the nucleic sequence of the consensus HERV-K(HML2) provirus ("Phoenix"), said nucleic sequence of the consensus HERV-K(HML2) provirus having the sequence SEQ ID NO: 4, or a nucleic acid encoding said at least one protein.

## Patentansprüche

1. Verwendung einer Zusammensetzung, umfassend:
- ein vom env-Gen eines endogenen Retrovirus kodiertes, immunsuppressives Protein, mutiert oder nicht, oder ein Fragment davon, das eine immunsuppressive Domäne des vom env-Gen eines endogenen Retrovirus kodierten Proteins umfasst, oder
- eine Nukleinsäure, die für das vom env-Gen eines endogenen Retrovirus kodierte Protein oder ein Fragment des vom env-Gen eines endogenen Retrovirus kodierten Proteins kodiert, oder
- einen Liganden, wie Antikörper, wobei der Ligand imstande ist, spezifisch an das vom env-Gen kodierte immunsuppressive Protein zu binden
- eine doppelsträngige RNA, eine siRNA, eine shRNA, eine miRNA oder eine Antisense-Nukleinsäure, die auf Nukleinsäuresequenzen des endogenen Retrovirus und/oder eines Proteins, das von dem endogenen Retrovirus exprimiert wird, gerichtet ist,
wobei das endogene Retrovirus ein humanes endogenes Retrovirus HERV-K ist,
wobei die Zusammensetzung
a. die Expression des endogenen Retrovirus hemmt und/oder
b. die Expression des exprimierten Proteins hemmt und/oder dessen Konzentration senkt und/oder dessen Aktivität hemmt, und/oder
c. eine Immunantwort gegen das vom env-Gen eines endogenen Retrovirus kodierte Protein stimuliert,
für die Herstellung eines Arzneimittels, das zur Vorbeugung und/oder Behandlung von Krebs in einem Säuger bestimmt ist, wobei die kanzerösen Zellen des Säugers das vom env-Gen eines endogenen Retrovirus kodierte Protein exprimieren.

2. Verwendung nach Anspruch 1, wobei HERV-K ein HERV-K-Subtyp ist, **dadurch gekennzeichnet, dass** der offene Leserahmen, der für das virale ENV-Protein kodiert, für ein Np9-Protein kodiert.

3. Verwendung nach Anspruch 1 oder 2, wobei HERV-K ein HERV-K-Subtyp ist, **dadurch gekennzeichnet, dass** der HERV-K-Subtyp ausgewählt ist aus der Gruppe bestehend aus dem HERV-K-Subtyp HERV-K1 01, HERV-K1 02, HERV-K1 07 und HERV-K(II).

4. Verwendung nach einem der Ansprüche 1 bis 3, wobei HERV-K ein HERV-K-Subtyp ist, **dadurch gekennzeichnet, dass** der offene Leserahmen für ein Np9-Protein mit einer Aminosäuresequenz kodiert, die ausgewählt ist aus der Gruppe bestehend aus:
a. der Aminosäuresequenz SEQ ID NR: 3 oder eines HERV-K-Subtyp Np9-Proteins, wie in der Genbank (GenPept) unter der Zugangsnummer P61580, P61581, P61582 und P61583 dargestellt; und
b. einer Aminosäuresequenz mit zumindest 75% Identität mit einer Aminosäuresequenz von a) und mit immunsuppressiver Aktivität.

5. Verwendung nach einem der Ansprüche 1 bis 4, wobei die Expression des endogenen Retrovirus und/oder des immunsuppressiven endogenen Retrovirusproteins durch eine doppelsträngige RNA, eine siRNA, eine shRNA, eine miRNA oder eine Antisense-Nukleinsäure gehemmt wird, die auf Nukleinsäuresequenzen des endogenen Retrovirus und/oder des immunsuppresiven endogenen Retrovirusproteins gerichtet ist.

6. Verwendung nach einem der Ansprüche 1 bis 5, wobei das Protein:
a. das immunsuppressive endogene Retrovirusprotein oder ein Fragment davon ist, das dessen immunsuppressive Domäne umfasst, oder
b. eine mutierte Form des immunsuppressiven endogenen Retrovirusproteins ist, dem es an immunsuppressiver Aktivität fehlt, oder Fragmente davon, die eine mutierte immunsuppressive Domäne des immunsuppressiven endogenen Retrovirusproteins umfassen.

7. Verwendung nach Anspruch 6, wobei die mutierte Form eines vom env-Gen des HERV-K kodierten immunsuppressiven Proteins, dem es an immunsuppressiver Aktivität mangelt, imstande ist, eine Immunantwort, eine zelluläre und/oder humorale Immunantwort
- gegen Zellen, die die nicht mutierte Form des vom env-Gen des HERV kodierten Proteins exprimieren, (zelluläre Immunantwort), oder
- gegen die nicht mutierte Form des vom env-Gen des HERV kodierten Proteins (humorale Immunantwort) hervorzurufen.

8. Verwendung nach Anspruch 1, wobei der Ligand, wie ein Antikörper, imstande ist, spezifisch an das vom env-Gen des endogenen Retrovirus kodierte immunsuppressive Protein zu binden.

9. Verwendung nach Anspruch 8, wobei der Ligand ein Antikörper, insbesondere ein monoklonare Antikörper, ein Antikörperfragment, wie Fab- oder F(ab) '₂-Fragmente, scFv-Polypeptide, chimäre Antikörper, humanisierte Antikörper, Aptamere oder Bindungspeptide ist.

10. Pharmazeutische Zusammensetzung, umfassend als aktive Substanz eine doppelsträngige RNA, eine siRNA, eine shRNA, eine miRNA oder eine Antisense-Nukleinsäure, die auf Nukleinsäuresequenzen eines endogenen Retrovirus und/oder eines immunsuppressiven Proteins gerichtet ist, das von einem endogenen Retrovirus exprimiert wird, in Verbindung mit einem pharmazeutisch annehmbaren Träger, wobei das endogene Retrovirus ein humanes endogenes Retrovirus HERV-K ist.

11. Pharmazeutische Zusammensetzung nach Anspruch 10, wobei das Protein ein vom env-Gen des HERV kodiertes immunsuppressives Protein, wie ENV-Protein oder ein Np9-Protein ist.

12. Pharmazeutische Zusammensetzung, umfassend als aktive Substanz ein immunsuppressives Protein, das von einem endogenen Retrovirus exprimiert wird, oder ein Fragment davon, wobei das endogene Retrovirus ein humanes endogenes Retrovirus HERV-K ist.

13. Pharmazeutische Zusammensetzung nach Anspruch 12, umfassend als aktive Substanz ein immunsuppressives Protein eines vom env-Gen des HERV-K kodierten immunsuppressives Proteins, mutiert oder nicht, oder ein Fragment davon, das die immunsuppressive Domäne des vom env-Gen des HERV-K kodierten immunsuppressiven
Proteins umfasst, oder eine Nukleinsäure, die für die aktive Substanz kodiert, in Verbindung mit einem pharmazeutisch annehmbaren Träger.

14. Pharmazeutische Zusammensetzung nach Anspruch 13, wobei das endogene Retrovirus ein HERV-K-Subtyp ist, **dadurch gekennzeichnet, dass** der offene Leserahmen, der für das virale env-Protein kodiert, für ein Np9-Protein kodiert.

15. Pharmazeutische Zusammensetzung nach Anspruch 13 oder 14, wobei HERV-K ein HERV-K-Subtyp ist, der ausgewählt ist aus der Gruppe bestehend aus dem HERV-K-Subtyp HERV-K1 01, HERV-K1 02, HERV-K1 07 und HERV-K(II).

16. Pharmazeutische Zusammensetzung nach Anspruch 15, wobei HERV-K ein HERV-K-Subtyp ist, **dadurch gekennzeichnet, dass** der offene Leserahmen, der für das virale env-Protein kodiert, für ein Np9-Protein mit einer Aminosäuresequenz kodiert, die ausgewählt ist aus der Gruppe bestehend aus:
a. der Aminosäuresequenz SEQ ID NR: 3 oder eines HERV-K-Subtyp Np9-Proteins, wie in der Genbank (GenPept) unter der Zugangsnummer P61580, P61581, P61582 und P61583 dargestellt; und
b. einer Aminosäuresequenz mit zumindest 75% Identität mit einer Aminosäuresequenz von a) und mit immunsuppressiver Aktivität.

17. Pharmazeutische Zusammensetzung nach einem der Ansprüche 12 bis 16, **dadurch gekennzeichnet, dass** die Zusammensetzung des Weiteren als aktive Substanz ein anderes HERV-Protein, für das die Nukleinsequenz des Provirus HERV kodiert, oder eine Nukleinsäure, die für das andere HERV-Protein kodiert, umfasst.

18. Pharmazeutische Zusammensetzung nach einem der Ansprüche 12 bis 17, **dadurch gekennzeichnet, dass** die Zusammensetzung des Weiteren als aktive Substanz zumindest ein Protein, vorzugsweise zumindest zwei, drei oder alle Proteine, für das bzw. die die Nukleinsequenz des Konsensus-HERV-K(HML2)-Provirus ("Phoenix") kodiert, wobei die Nukleinsequenz des Konsensus-HERV-K(HML2)-Provirus die Sequenz SEQ ID NR: 4 hat, oder eine Nukleinsäure, die das zumindest eine Protein kodiert, umfasst.

## Revendications

1. Utilisation d'une composition comprenant :
- une protéine immunosuppressive codée par le gène *env* d'un rétrovirus endogène, muté ou non, ou un fragment de celle-ci comprenant un domaine immunosuppresseur de ladite protéine immunosuppressive codée par le gène *env* d'un rétrovirus endogène, ou
- un acide nucléique codant ladite protéine immunosuppressive codée par le gène *env* d'un rétrovirus endogène, ou codant un fragment de ladite protéine immunosuppressive codée par le gène *env* d'un rétrovirus endogène, ou
- un ligand tel que des anticorps, ledit ligand étant capable de se lier spécifiquement à ladite protéine immunosuppressive codée par un gène *env* d'un rétrovirus endogène, ou
- un ARN double brin, un siRNA, un shRNA, un miRNA, ou un acide nucléique antisens ciblant les séquences d'acides nucléiques du rétrovirus endogène et/ou d'une protéine exprimée par ledit rétrovirus endogène,
où ledit retrovirus endogène est le rétrovirus endogène humain HERV-K
ladite composition :
a. inhibant l'expression dudit rétrovirus endogène, et/ou
b. inhibant l'expression, et/ou diminuant la concentration, et/ou inhibant l'activité de ladite protéine exprimée, et/ou
c. stimulant une réponse immune contre ladite protéine immunosuppressive codée par un gène *env* d'un rétrovirus endogène
pour la préparation d'un médicament pour la prévention et/ou le traitement du cancer chez un mammifère, où les cellules cancéreuses dudit mammifère expriment ladite protéine immunosuppressive codée par le gène *env* d'un rétrovirus endogène.

2. Utilisation selon la revendication 1, où HERV-K est un sous-type HERV-K **caractérisé en ce que** la phase ouverte de lecture codant la protéine virale ENV code une protéine Np9.

3. Utilisation selon la revendication 1 ou 2, où HERV-K est un sous-type HERV-K **caractérisé en ce que** ledit sous-type HERV-K est choisi dans le groupe constitué du sous-type HERV-K HERV-K1 01, HERV-K1 02, HERV-K1 07 et HERV-K(II).

4. Utilisation selon l'une quelconque des revendications 1 à 3, où HERV-K est un sous-type HERV-K **caractérisé en ce que** la phase ouverte de lecture code une protéine Np9 ayant une séquence d'acides aminés choisie dans le groupe constitué de :
a. la séquence d'acides aminés SEQ ID NO : 3 ou de la protéine NP9 du sous type HERV-K tel que décrite dans Genbank (GenPept) sous le numéro d'accession, P61580, P61581, P61582 et P61583 ; et
b. une séquence d'acides aminés ayant au moins 75% d'identité avec une séquence d'acides aminés de a) et ayant une activité immunosuppressive.

5. Utilisation selon l'une quelconque des revendications 1 à 4, où l'expression dudit rétrovirus endogène et/ou de la protéine immunosuppressive du rétrovirus endogène, est inhibée par un ARN double brin, un siRNA, un shRNA, un miRNA, ou un acide nucléique antisens ciblant les séquences d'acides nucléiques du rétrovirus endogène et/ou d'une protéine exprimée par ledit rétrovirus endogène.

6. Utilisation selon l'une quelconque des revendications 1 à 5, où ladite protéine est :
a. ladite protéine immunosuppressive d'un rétrovirus endogène, ou un fragment de celle-ci, comprenant son domaine immunosuppresseur, ou
b. une forme mutée de ladite protéine immunosuppressive d'un rétrovirus endogène dépourvue d'activité immunosuppressive, ou des fragments de celle-ci, comprenant un domaine immunosuppresseur muté de ladite protéine immunosuppressive d'un rétrovirus endogène.

7. Utilisation selon la revendication 6, où ladite forme mutée dépourvue d'activité immunosuppressive de la protéine immunosuppressive codée par le gène env de HERV-K est capable d'induire une réponse immune, une réponse immune cellulaire et/ou humorale
- contre les cellules exprimant la forme non mutée de ladite protéine codée par le gène *env* de HERV-K (réponse immune cellulaire) ou
- contre la forme non mutée de la de ladite protéine codée par le gène *env* de HERV-K (réponse immune humorale).

8. Utilisation selon la revendication 1, où ledit ligand, tel que des anticorps, est capable de se lier spécifiquement à ladite protéine immunosuppressive codée par le gène *env* d'un rétrovirus endogène.

9. Utilisation selon la revendication 8, où ledit ligand est un anticorps, en particulier un anticorps monoclonal, un fragment d'anticorps, tel que des fragments Fab ou F(ab)'₂, des peptides scFv, des anticorps chimériques, des anticorps humanisés, des aptamères ou des peptides de liaison.

10. Composition pharmaceutique comprenant en tant que substance active un ARN double brin, un siRNA, un shRNA, un miRNA, ou un acide nucléique antisens ciblant les séquences d'acides nucléiques du rétrovirus endogène et/ou d'une protéine exprimée par ledit rétrovirus endogène, en association avec un véhicule pharmaceutiquement acceptable,
ledit rétrovirus endogène étant le rétrovirus endogène humain HERV-K.

11. Composition pharmaceutique selon la revendication 10, où la protéine est une protéine immunosuppressive codée par le gène *env* de HERV, telle que la protéine ENV ou une protéine Np9.

12. Composition pharmaceutique comprenant en tant que substance active une protéine immunosuppressive exprimée par un rétrovirus endogène, ou un fragment de celle-ci ledit rétrovirus endogène étant le rétrovirus endogène humain HERV-K.

13. Composition pharmaceutique selon la revendication 12, comprenant en tant que substance active une protéine immunosuppressive d'une protéine codée par un gène *env* de HERV-K, mutée ou non, ou un fragment de celle-ci comprenant le domaine immunosuppresseur de la dite protéine codée par un gène *env* de HERV-K, ou un acide nucléique codant ladite substance active, en association avec un véhicule pharmaceutiquement acceptable.

14. Composition pharmaceutique selon la revendication 13, où le rétrovirus endogène est un sous type de HERV-K **caractérisé en ce que** la phase ouverte de lecture codant la protéine virale ENV code une protéine Np9.

15. Composition pharmaceutique selon la revendication 13 ou 14, où HERV-K est un sous-type HERV-K choisi dans le group constitué du sous type HERV-K HERV-K1 01, HERV-K1 02, HERV-K1 07 et HERV-K(II).

16. Composition pharmaceutique selon la revendication 15, où HERV-K est un sous-type HERV-K **caractérisé en ce que** la phase ouverte de lecture de la protéine virale ENV code une protéine Np9 ayant une séquence d'acides aminés choisie dans le groupe constitué de
a. la séquence d'acides aminés SEQ ID NO : 3 ou de la protéine NP9 du sous type HERV-K tel que décrite dans Genbank (GenPept) sous le numéro d'accession, P61580, P61581, P61582 et P61583 ; et
b. une séquence d'acides aminés ayant au moins 75% d'identité avec la séquence d'acides aminés de a) et ayant une activité immunosuppressive.

17. Composition pharmaceutique selon l'une quelconque des revendications 12 à 16, **caractérisée en ce que** ladite composition comprend de plus en tant que substance active une autre protéine de HERV codée par la séquence d'acide nucléique dudit provirus HERV, ou un acide nucléique codant ladite autre protéine HERV.

18. Composition pharmaceutique selon l'une quelconque des revendications 12 à 17, **caractérisée en ce que** ladite composition comprend de plus en tant que substance active, au moins une protéine, préférentiellement au moins deux, trois ou toutes les protéines, codées par la séquence nucléique du consensus du provirus HERV-K(HML2) (« Phoenix »), ladite séquence consensus du provirus HERV-K (HML2) ayant la séquence SEQ ID NO : 4, ou un acide nucléique codant ladite au moins une protéine.


### SEQUENCE LISTING

<110> CENTRE NATIONAL DE LA RECHERCHE SCIENTIFIQUE (CNRS)
   UNIVERSITE PARIS SUD
   INSTITUT GUSTAVE-ROUSSY
   Marianne MANGENEY
   Martial RENARD
   Thierry HEIDMANN
   Julien POTHLICHET
   Marie DEWANNIEUX
<120> Endogenous Retrovirus and proteins encoded by env gene as a target for cancer treatment
<130> D24324
<150> US 60/666,178
   <151> 2005-03-30
<160> 9
<170> PatentIn version 3.3
<210> 1
   <211> 5035
   <212> DNA
   <213> artificial sequence
<220>
   <223> phCMV-Np9 plasmid nucleic sequence
<400> 1
   gcggccgctc tagagagctt ggcccattgc atacgttgta tccatatcat aatatgtaca 60
   tttatattgg ctcatgtcca acattaccgc catgttgaca ttgattattg actagttatt 120
   aatagtaatc aattacgggg tcattagttc atagcccata tatggagttc cgcgttacat 180
   aacttacggt aaatggcccg cctggctgac cgcccaacga cccccgccca ttgacgtcaa 240
   taatgacgta tgttcccata gtaacgccaa tagggacttt ccattgacgt caatgggtgg 300
   agtatttacg gtaaactgcc cacttggcag tacatcaagt gtatcatatg ccaagtacgc 360
   cccctattga cgtcaatgac ggtaaatggc ccgcctggca ttatgcccag tacatgacct 420
   tatgggactt tcctacttgg cagtacatct acgtattagt catcgctatt accatggtga 480
   tgcggttttg gcagtacatc aatgggcgtg gatagcggtt tgactcacgg ggatttccaa 540
   gtctccaccc cattgacgtc aatgggagtt tgttttggca ccaaaatcaa cgggactttc 600
   caaaatgtcg taacaactcc gccccattga cgcaaatggg cggtaggcgt gtacggtggg 660
   aggtctatat aagcagagct cgtttagtga accgtcagat cgcctggaga cgccatccac 720
   gctgttttga cctccataga agacaccggg accgatccag cctccggtcg accgatcctg 780
   agaacttcag ggtgagtttg gggacccttg attgttcttt ctttttcgct attgtaaaat 840
   tcatgttata tggagggggc aaagttttca gggtgttgtt tagaatggga agatgtccct 900
   tgtatcacca tggaccctca tgataatttt gtttctttca ctttctactc tgttgacaac 960
   cattgtctcc tcttattttc ttttcatttt ctgtaacttt ttcgttaaac tttagcttgc 1020
   atttgtaacg aatttttaaa ttcacttttg tttatttgtc agattgtaag tactttctct 1080
   aatcactttt ttttcaaggc aatcagggta tattatattg tacttcagca cagttttaga 1140
   gaacaattgt tataattaaa tgataaggta gaatatttct gcatataaat tctggctggc 1200
   gtggaaatat tcttattggt agaaacaact acaccctggt catcatcctg cctttctctt 1260
   tatggttaca atgatataca ctgtttgaga tgaggataaa atactctgag tccaaaccgg 1320
   gcccctctgc taaccatgtt catgccttct tctctttcct acagctcctg ggcaacgtgc 1380
   tggttgttgt gctgtctcat cattttggca aagaattcct cgacggatcc ctcgagatga 1440
   acccatcgga gatgcaaaga aaagggcctc cacggagatg tctgcaggtg tacccaacag 1500
   ctccgaagag acagcgacca tcgagaacgg gccatgatga cgatggcggt tttgtcgaaa 1560
   agaaaagggg gaaatgtggg gaaaagcaag agagatcaga ttgttactgt gtctgtgtag 1620
   aaagaagtag acatgggaga ctccatttgg ttatgtgtta aacgcgtcag gtgcaggctg 1680
   cctatcagaa ggtggtggct ggtgtggcca atgccctggc tcacaaatac cactgagatc   1740
   tttttccctc tgccaaaaat tatggggaca tcatgaagcc ccttgagcat ctgacttctg   1800
   gctaataaag gaaatttatt ttcattgcaa tagtgtgttg gaattttttg tgtctctcac   1860
   tcggaaggac atatgggagg gcaaatcatt taaaacatca gaatgagtat ttggtttaga   1920
   gtttggcaac atatgcccat atgctggctg ccatgaacaa aggttggcta taaagaggtc   1980
   atcagtatat gaaacagccc cctgctgtcc attccttatt ccatagaaaa gccttgactt   2040
   gaggttagat tttttttata ttttgttttg tgttattttt ttctttaaca tccctaaaat   2100
   tttccttaca tgttttacta gccagatttt tcctcctctc ctgactactc ccagtcatag   2160
   ctgtccctct tctcttatgg agatccctcg acggatcggc cgcaattcgt aatcatgtca   2220
   tagctgtttc ctgtgtgaaa ttgttatccg ctcacaattc cacacaacat acgagccgga   2280
   agcataaagt gtaaagcctg gggtgcctaa tgagtgagct aactcacatt aattgcgttg   2340
   cgctcactgc ccgctttcca gtcgggaaac ctgtcgtgcc agctgcatta atgaatcggc   2400
   caacgcgcgg ggagaggcgg tttgcgtatt gggcgctctt ccgcttcctc gctcactgac   2460
   tcgctgcgct cggtcgttcg gctgcggcga gcggtatcag ctcactcaaa ggcggtaata   2520
   cggttatcca cagaatcagg ggataacgca ggaaagaaca tgtgagcaaa aggccagcaa   2580
   aaggccagga accgtaaaaa ggccgcgttg ctggcgtttt tccataggct ccgcccccct   2640
   gacgagcatc acaaaaatcg acgctcaagt cagaggtggc gaaacccgac aggactataa   2700
   agataccagg cgtttccccc tggaagctcc ctcgtgcgct ctcctgttcc gaccctgccg   2760
   cttaccggat acctgtccgc ctttctccct tcgggaagcg tggcgctttc tcatagctca   2820
   cgctgtaggt atctcagttc ggtgtaggtc gttcgctcca agctgggctg tgtgcacgaa   2880
   ccccccgttc agcccgaccg ctgcgcctta tccggtaact atcgtcttga gtccaacccg   2940
   gtaagacacg acttatcgcc actggcagca gccactggta acaggattag cagagcgagg   3000
   tatgtaggcg gtgctacaga gttcttgaag tggtggccta actacggcta cactagaaga   3060
   acagtatttg gtatctgcgc tctgctgaag ccagttacct tcggaaaaag agttggtagc   3120
   tcttgatccg gcaaacaaac caccgctggt agcggtggtt tttttgtttg caagcagcag   3180
   attacgcgca gaaaaaaagg atctcaagaa gatcctttga tcttttctac ggggtctgac   3240
   gctcagtgga acgaaaactc acgttaaggg attttggtca tgagattatc aaaaaggatc   3300
   ttcacctaga tccttttaaa ttaaaaatga agttttaaat caatctaaag tatatatgag   3360
   taaacttggt ctgacagtta ccaatgctta atcagtgagg cacctatctc agcgatctgt   3420
   ctatttcgtt catccatagt tgcctgactc cccgtcgtgt agataactac gatacgggag   3480
   ggcttaccat ctggccccag tgctgcaatg ataccgcgag acccacgctc accggctcca   3540
   gatttatcag caataaacca gccagccgga agggccgagc gcagaagtgg tcctgcaact   3600
   ttatccgcct ccatccagtc tattaattgt tgccgggaag ctagagtaag tagttcgcca   3660
   gttaatagtt tgcgcaacgt tgttgccatt gctacaggca tcgtggtgtc acgctcgtcg   3720
   tttggtatgg cttcattcag ctccggttcc caacgatcaa ggcgagttac atgatccccc   3780
   atgttgtgca aaaaagcggg ttagctcctt cggtcctccg atcgttgtca gaagtaagtt   3840
   ggccgcagtg ttatcactca tggttatggc agcactgcat aattctctta ctgtcatgcc   3900
   atccgtaaga tgcttttctg tgactggtga gtactcaacc aagtcattct gagaatagtg   3960
   tatgcggcga ccgagttgct cttgcccggc gtcaatacgg gataataccg cgccacatag   4020
   cagaacttta aaagtgctca tcattggaaa acgttcttcg gggcgaaaac tctcaaggat   4080
   cttaccgctg ttgagatcca gttcgatgta acccactcgt gcacccaact gatcttcagc   4140
   atcttttact ttcaccagcg tttctgggtg agcaaaaaca ggaaggcaaa atgccgcaaa   4200
   aaagggaata agggcgacac ggaaatgttg aatactcata ctcttccttt ttcaatatta   4260
   ttgaagcatt tatcagggtt attgtctcat gagcggatac atatttgaat gtatttagaa   4320
   aaataaacaa ataggggttc cgcgcacatt tccccgaaaa gtgccaccta aattgtaagc   4380
   gttaatattt tgttaaaatt cgcgttaaat ttttgttaaa tcagctcatt ttttaaccaa   4440
   taggccgaaa tcggcaaaat cccttataaa tcaaaagaat agaccgagat agggttgagt   4500
   gttgttccag tttggaacaa gagtccacta ttaaagaacg tggactccaa cgtcaaaggg   4560
   cgaaaaaccg tctatcaggg cgatggccca ctacgtgaac catcacccta atcaagtttt   4620
   ttggggtcga ggtgccgtaa agcactaaat cggaacccta aagggagccc ccgatttaga   4680
   gcttgacggg gaaagccggc gaacgtggcg agaaaggaag ggaagaaagc gaaaggagcg   4740
   ggcgctaggg cgctggcaag tgtagcggtc acgctgcgcg taaccaccac acccgccgcg   4800
   cttaatgcgc cgctacaggg cgcgtcccat tcgccattca ggctgcgcaa ctgttgggaa   4860
   gggcgatcgg tgcgggcctc ttcgctatta cgccagctgg cgaaaggggg atgtgctgca   4920
   aggcgattaa gttgggtaac gccagggttt tcccagtcac gacgttgtaa aacgacggcc   4980
   agtgagcgcg cgtaatacga ctcactatag ggcgaattgg agctccaccg cggtg        5035
<210> 2
   <211> 225
   <212> DNA
   <213> artificial sequence
<220>
   <223> nucleic sequence encoding HERV-K (HML-2.HOM) Np9
<400> 2
   atgaacccat cggagatgca aagaaaaggg cctccacgga gatgtctgca ggtgtaccca    60
   acagctccga agagacagcg accatcgaga acgggccatg atgacgatgg cggttttgtc   120
   gaaaagaaaa gggggaaatg tggggaaaag caagagagat cagattgtta ctgtgtctgt   180
   gtagaaagaa gtagacatgg gagactccat tttgttatgt gttaa                   225
<210> 3
   <211> 74
   <212> PRT
   <213> artificial sequence
<220>
   <223> Np9 amino acid sequence from HERV-K (HML-2.HOM)
<400> 3
   Met Asn Pro Ser Glu Met Gln Arg Lys Gly Pro Pro Arg Arg Cys Leu
   1               5                   10                  15
   Gln Val Tyr Pro Thr Ala Pro Lys Arg Gln Arg Pro Ser Arg Thr Gly
               20                  25                  30
   His Asp Asp Asp Gly Gly Phe Val Glu Lys Lys Arg Gly Lys Cys Gly
           35                  40                  45
   Glu Lys Gln Glu Arg Ser Asp Cys Tyr Cys Val Cys Val Glu Arg Ser
       50                  55                  60
   Arg His Gly Arg Leu His Phe Val Met Cys
   65                  70
<210> 4
   <211> 9472
   <212> DNA
   <213> Artificial sequence
<220>
   <223> consensus nucleic sequence of the Phoenix provirus
<400> 4
   tgtggggaaa agcaagagag atcagattgt tactgtgtct gtgtagaaag aagtagacat     60
   aggagactcc attttgttat gtactaagaa aaattcttct gccttgagat tctgttaatc    120
   tatgacctta cccccaaccc cgtgctctct gaaacatgtg ctgtgtcaac tcagagttaa    180
   atggattaag ggcggtgcag gatgtgcttt gttaaacaga tgcttgaagg cagcatgctc    240
   cttaagagtc atcaccactc cctaatctca agtacccagg gacacaaaaa ctgcggaagg    300
   ccgcagggac ctctgcctag gaaagccagg tattgtccaa ggtttctccc catgtgatag    360
   tctgaaatat ggcctcgtgg gaagggaaag acctgaccgt cccccagccc gacacccgta    420
   aagggtctgt gctgaggagg attagtaaaa gaggaaggaa tgcctcttgc agttgagaca    480
   agaggaaggc atctgtctcc tgcctgtccc tgggcaatgg aatgtctcgg tataaaaccc    540
   gattgtatgc tccatctact gagataggga aaaaccgcct tagggctgga ggtgggacct    600
   gcgggcagca atactgcttt gtaaagcact gagatgttta tgtgtatgca tatctaaaag    660
   cacagcactt aatcctttac attgtctatg atgcaaagac ctttgttcac gtgtttgtct    720
   gctgaccctc tccccacaat tgtcttgtga ccctgacaca tccccctctt tgagaaacac    780
   ccacagatga tcaataaata ctaagggaac tcagaggctg gcgggatcct ccatatgctg    840
   aacgctggtt ccccgggtcc ccttatttct ttctctatac tttgtctctg tgtctttttc    900
   ttttccaaat ctctcgtccc accttacgag aaacacccac aggtgtgtag gggcaaccca    960
   cccctacatc tggtgcccaa cgtggaggct tttctctagg gtgaaggtac gctcgagcgt   1020
   ggtcattgag gacaagtcga cgagagatcc cgagtacgtc tacagtcagc cttacggtaa   1080
   gcttgtgcgc tcggaagaag ctagggtgat aatggggcaa actaaaagta aaattaaaag   1140
   taaatatgcc tcttatctca gctttattaa aattctttta aaaagagggg gagttaaagt   1200
   atctacaaaa aatctaatca agctatttca aataatagaa caattttgcc catggtttcc   1260
   agaacaagga actttagatc taaaagattg gaaaagaatt ggtaaggaac taaaacaagc   1320
   aggtaggaag ggtaatatca ttccacttac agtatggaat gattgggcca ttattaaagc   1380
   agctttagaa ccatttcaaa cagaagaaga tagcgtttca gtttctgatg cccctggaag   1440
   ctgtataata gattgtaatg aaaacacaag gaaaaaatcc cagaaagaaa cggaaggttt   1500
   acattgcgaa tatgtagcag agccggtaat ggctcagtca acgcaaaatg ttgactataa   1560
   tcaattacag gaggtgatat atcctgaaac gttaaaatta gaaggaaaag gtccagaatt   1620
   agtggggcca tcagagtcta aaccacgagg cacaagtcct cttccagcag gtcaggtgcc   1680
   cgtaacatta caacctcaaa agcaggttaa agaaaataag acccaaccgc cagtagccta   1740
   tcaatactgg ccgccggctg aacttcagta tcggccaccc ccagaaagtc agtatggata   1800
   tccaggaatg cccccagcac cacagggcag ggcgccatac cctcagccgc ccactaggag   1860
   acttaatcct acggcaccac ctagtagaca gggtagtgaa ttacatgaaa ttattgataa   1920
   atcaagaaag gaaggagata ctgaggcatg gcaattccca gtaacgttag aaccgatgcc   1980
   acctggagaa ggagcccaag agggagagcc tcccacagtt gaggccagat acaagtcttt   2040
   ttcgataaaa atgctaaaag atatgaaaga gggagtaaaa cagtatggac ccaactcccc   2100
   ttatatgagg acattattag attccattgc tcatggacat agactcattc cttatgattg   2160
   ggagattctg gcaaaatcgt ctctctcacc ctctcaattt ttacaattta agacttggtg   2220
   gattgatggg gtacaagaac aggtccgaag aaatagggct gccaatcctc cagttaacat   2280
   agatgcagat caactattag gaataggtca aaattggagt actattagtc aacaagcatt   2340
   aatgcaaaat gaggccattg agcaagttag agctatctgc cttagagcct gggaaaaaat   2400
   ccaagaccca ggaagtacct gcccctcatt taatacagta agacaaggtt caaaagagcc   2460
   ctatcctgat tttgtggcaa ggctccaaga tgttgctcaa aagtcaattg ccgatgaaaa   2520
   agcccgtaag gtcatagtgg agttgatggc atatgaaaac gccaatcctg agtgtcaatc   2580
   agccattaag ccattaaaag gaaaggttcc tgcaggatca gatgtaatct cagaatatgt   2640
   aaaagcctgt gatggaatcg gaggagctat gcataaagct atgcttatgg ctcaagcaat   2700
   aacaggagtt gttttaggag gacaagttag aacatttgga ggaaaatgtt ataattgtgg   2760
   tcaaattggt cacttaaaaa agaattgccc agtcttaaac aaacagaata taactattca   2820
   agcaactaca acaggtagag agccacctga cttatgtcca agatgtaaaa aaggaaaaca   2880
   ttgggctagt caatgtcgtt ctaaatttga taaaaatggg caaccattgt cgggaaacga   2940
   gcaaaggggc cagcctcagg ccccacaaca aactggggca ttcccaattc agccatttgt   3000
   tcctcagggt tttcagggac aacaaccccc actgtcccaa gtgtttcagg gaataagcca   3060
   gttaccacaa tacaacaatt gtcccccgcc acaagcggca gtgcagcagt agatttatgt   3120
   actatacaag cagtctctct gcttccaggg gagcccccac aaaaaatccc cacaggggta   3180
   tatggccccc tgcctgaggg gactgtagga ctaatcttgg gaagatcaag tctaaatcta   3240
   aaaggagttc aaattcatac tagtgtggtt gattcagact ataaaggcga aattcaattg   3300
   gttattagct cttcaattcc ttggagtgcc agtccaggag acaggattgc tcaattatta   3360
   ctcctgccat atattaaggg tggaaatagt gaaataaaaa gaataggagg gcttggaagc   3420
   actgatccaa caggaaaggc tgcatattgg gcaagtcagg tctcagagaa cagacctgtg   3480
   tgtaaggcca ttattcaagg aaaacagttt gaagggttgg tagacactgg agcagatgtc   3540
   tctatcattg ctttaaatca gtggccaaaa aattggccta aacaaaaggc tgttacagga   3600
   cttgtcggca taggcacagc ctcagaagtg tatcaaagta cggagatttt acattgctta   3660
   gggccagata atcaagaaag tactgttcag ccaatgatta cttcaattcc tcttaatctg   3720
   tggggtcgag atttattaca acaatggggt gcggaaatca ccatgcccgc tccattatat   3780
   agccccacga gtcaaaaaat catgaccaag atgggatata taccaggaaa gggactaggg   3840
   aaaaatgaag atggcattaa aattccagtt gaggctaaaa taaatcaaaa aagagaagga   3900
   atagggtatc ctttttaggg gcggccactg tagagcctcc taaacccata ccattaactt   3960
   ggaaaacaga aaaaccggtg tgggtaaatc agtggccgct accaaaacaa aaactggagg   4020
   ctttacattt attagcaaat gaacagttag aaaagggtca tattgagcct tcgttctcac   4080
   cttggaattc tcctgtgttt gtaattcaga agaaatcagg caaatggcgt atgttaactg   4140
   acttaagggc tgtaaacgcc gtaattcaac ccatggggcc tctccaaccc gggttgccct   4200
   ctccggccat gatcccaaaa gattggcctt taattataat tgatctaaag gattgctttt   4260
   ttaccatccc tctggcagag caggattgtg aaaaatttgc ctttactata ccagccataa   4320
   ataataaaga accagccacc aggtttcagt ggaaagtgtt acctcaggga atgcttaata   4380
   gtccaactat ttgtcagact tttgtaggtc gagctcttca accagttaga gaaaagtttt   4440
   cagactgtta tattattcat tatattgatg atattttatg tgctgcagaa acgaaagata   4500
   aattaattga ctgttataca tttctgcaag cagaggttgc caatgctgga ctggcaatag 4560
   catctgataa gatccaaacc tctactcctt ttcattattt agggatgcag atagaaaata 4620
   gaaaaattaa gccacaaaaa atagaaataa gaaaagacac attaaaaaca ctaaatgatt 4680
   ttcaaaaatt actaggagat attaattgga ttcggccaac tctaggcatt cctacttatg 4740
   ccatgtcaaa tttgttctct atcttaagag gagactcaga cttaaatagt aaaagaatgt 4800
   taaccccaga ggcaacaaaa gaaattaaat tagtggaaga aaaaattcag tcagcgcaaa 4860
   taaatagaat agatccctta gccccactcc aacttttgat ttttgccact gcacattctc 4920
   caacaggcat cattattcaa aatactgatc ttgtggagtg gtcattcctt cctcacagta 4980
   cagttaagac ttttacattg tacttggatc aaatagctac attaatcggt cagacaagat 5040
   tacgaataat aaaattatgt ggaaatgacc cagacaaaat agttgtccct ttaaccaagg 5100
   aacaagttag acaagccttt atcaattctg gtgcatggca gattggtctt gctaattttg 5160
   tgggaattat tgataatcat tacccaaaaa caaagatctt ccagttctta aaattgacta 5220
   cttggattct acctaaaatt accagacgtg aacctttaga aaatgctcta acagtattta 5280
   ctgatggttc cagcaatgga aaagcagctt acacagggcc gaaagaacga gtaatcaaaa 5340
   ctccatatca atcggctcaa agagcagagt tggttgcagt cattacagtg ttacaagatt 5400
   ttgaccaacc tatcaatatt atatcagatt ctgcatatgt agtacaggct acaagggatg 5460
   ttgagacagc tctaattaaa tatagcatgg atgatcagtt aaaccagcta ttcaatttat 5520
   tacaacaaac tgtaagaaaa agaaatttcc cattttatat tactcatatt cgagcacaca 5580
   ctaatttacc agggcctttg actaaagcaa atgaacaagc tgacttactg gtatcatctg 5640
   cactcataaa agcacaagaa cttcatgctt tgactcatgt aaatgcagca ggattaaaaa 5700
   acaaatttga tgtcacatgg aaacaggcaa aagatattgt acaacattgc acccagtgtc 5760
   aagtcttaca cctgcccact caagaggcag gagttaatcc cagaggtctg tgtcctaatg 5820
   cattatggca aatggatgtc acgcatgtac cttcatttgg aagattatca tatgttcatg 5880
   taacagttga tacttattca catttcatat gggcaacttg ccaaacagga gaaagtactt 5940
   cccatgttaa aaaacattta ttgtcttgtt ttgctgtaat gggagttcca gaaaaaatca 6000
   aaactgacaa tggaccagga tattgtagta aagctttcca aaaattctta agtcagtgga 6060
   aaatttcaca tacaacagga attccttata attcccaagg acaggccata gttgaaagaa 6120
   ctaatagaac actcaaaact caattagtta aacaaaaaga agggggagac agtaaggagt 6180
   gtaccactcc tcagatgcaa cttaatctag cactctatac tttaaatttt ttaaacattt 6240
   atagaaatca gactactact tctgcagaac aacatcttac tggtaaaaag aacagcccac 6300
   atgaaggaaa actaatttgg tggaaagata ataaaaataa gacatgggaa atagggaagg 6360
   tgataacgtg ggggagaggt tttgcttgtg tttcaccagg agaaaatcag cttcctgttt 6420
   ggatacccac tagacatttg aagttctaca atgaacccat cagagatgca aagaaaagca 6480
   cctccgcgga gacggagaca ccgcaatcga gcaccgttga ctcacaagat gaacaaaatg 6540
   gtgacgtcag aagaacagat gaagttgcca tccaccaaga aggcagagcc gccgacttgg 6600
   gcacaactaa agaagctgac gcagttagct acaaaatatc tagagaacac aaaggtgaca 6660
   caaaccccag agagtatgct gcttgcagcc ttgatgattg tatcaatggt ggtaagtctc 6720
   cctatgcctg caggagcagc tgcagctaac tatacctact gggcctatgt gcctttcccg 6780
   cccttaattc gggcagtcac atggatggat aatcctatag aagtatatgt taatgatagt 6840
   gtatgggtac ctggccccat agatgatcgc tgccctgcca aacctgagga agaagggatg 6900
   atgataaata tttccattgg gtatcgttat cctcctattt gcctagggag agcaccagga 6960
   tgtttaatgc ctgcagtcca aaattggttg gtagaagtac ctactgtcag tcccatcagt 7020
   agattcactt atcacatggt aagcgggatg tcactcaggc cacgggtaaa ttatttacaa 7080
   gacttttctt atcaaagatc attaaaattt agacctaaag ggaaaccttg ccccaaggaa 7140
   attcccaaag aatcaaaaaa tacagaagtt ttagtttggg aagaatgtgt ggccaatagt 7200
   gcggtgatat tacaaaacaa tgaatttgga actattatag attgggcacc tcgaggtcaa 7260
   ttctaccaca attgctcagg acaaactcag tcgtgtccaa gtgcacaagt gagtccagct 7320
   gttgatagcg acttaacaga aagtttagac aaacataagc ataaaaaatt gcagtctttc 7380
   tacccttggg aatggggaga aaaaggaatc tctaccccaa gaccaaaaat aataagtcct 7440
   gtttctggtc ctgaacatcc agaattatgg aggcttactg tggcctcaca ccacattaga 7500
   atttggtctg gaaatcaaac tttagaaaca agagatcgta agccatttta tactgtcgac 7560
   ctaaattcca gtctaacagt tcctttacaa agttgcgtaa agccccctta tatgctagtt 7620
   gtaggaaata tagttattaa accagactcc cagactataa cctgtgaaaa ttgtagattg 7680
   cttacttgca ttgattcaac ttttaattgg caacaccgta ttctgctggt gagagcaaga 7740
   gagggcgtgt ggatccctgt gtccatggac cgaccgtggg aggcctcacc atccatccat 7800
   attttgactg aagtattaaa aggtgtttta aatagatcca aaagattcat ttttacttta 7860
   attgcagtga ttatgggatt aattgcagtc acagctacgg ctgctgtagc aggagttgca 7920
   ttgcactctt ctgttcagtc agtaaacttt gttaatgatt ggcaaaaaaa ttctacaaga 7980
   ttgtggaatt cacaatctag tattgatcaa aaattggcaa atcaaattaa tgatcttaga 8040
   caaactgtca tttggatggg agacagactc atgagcttag aacatcgttt ccagttacaa 8100
   tgtgactgga atacgtcaga tttttgtatt acaccccaaa tttataatga gtctgagcat 8160
   cactgggaca tggttagacg ccatctacag ggaagagaag ataatctcac tttagacatt 8220
   tccaaattaa aagaacaaat tttcgaagca tcaaaagccc atttaaattt ggtgccagga 8280
   actgaggcaa ttgcaggagt tgctgatggc ctcgcaaatc ttaaccctgt cacttgggtt 8340
   aagaccattg gaagtactac gattataaat ctcatattaa tccttgtgtg cctgttttgt 8400
   ctgttgttag tctgcaggtg tacccaacag ctccgaagag acagcgacca tcgagaacgg 8460
   gccatgatga cgatggcggt tttgtcgaaa agaaaagggg gaaatgtggg gaaaagcaag 8520
   agagatcaga ttgttactgt gtctgtgtag aaagaagtag acataggaga ctccattttg 8580
   ttatgtacta agaaaaattc ttctgccttg agattctgtt aatctatgac cttaccccca 8640
   accccgtgct ctctgaaaca tgtgctgtgt caactcagag ttgaatggat taagggcggt 8700
   gcaggatgtg ctttgttaaa cagatgcttg aaggcagcat gctccttaag agtcatcacc 8760
   actccctaat ctcaagtacc cagggacaca aaaactgcgg aaggccgcag ggacctctgc 8820
   ctaggaaagc caggtattgt ccaaggtttc tccccatgtg atagtctgaa atatggcctc 8880
   gtgggaaggg aaagacctga ccgtccccca gcccgacacc cgtaaagggt ctgtgctgag 8940
   gaggattagt aaaagaggaa ggaatgcctc ttgcagttga gacaagagga aggcatctgt 9000
   ctcctgcctg tccctgggca atggaatgtc tcggtataaa acccgattgt atgctccatc 9060
   tactgagata gggaaaaacc gccttagggc tggaggtggg acctgcgggc agcaatactg 9120
   ctttgtaaag cattgagatg tttatgtgta tgcatatcta aaagcacagc acttaatcct 9180
   ttacattgtc tatgatgcaa agacctttgt tcacgtgttt gtctgctgac cctctcccca 9240
   caattgtctt gtgaccctga cacatccccc tctttgagaa acacccacag atgatcaata 9300
   aatactaagg gaactcagag gctggcggga tcctccatat gctgaacgct ggttccccgg 9360
   gtccccttat ttctttctct atactttgtc tctgtgtctt tttcttttcc aaatctctcg 9420
   tcccacctta cgagaaacac ccacaggtgt gtaggggcaa cccaccccta ca 9472
<210> 5
   <211> 666
   <212> PRT
   <213> artificial sequence
<220>
   <223> Gag protein encoded by the consensus nucleic sequence of the Phoenix provirus
<400> 5
   Met Gly Gln Thr Lys Ser Lys Ile Lys Ser Lys Tyr Ala Ser Tyr Leu
   1               5                   10                  15
   Ser Phe Ile Lys Ile Leu Leu Lys Arg Gly Gly Val Lys Val Ser Thr
               20                  25                  30
   Lys Asn Leu Ile Lys Leu Phe Gln Ile Ile Glu Gln Phe Cys Pro Trp
           35                  40                  45
   Phe Pro Glu Gln Gly Thr Leu Asp Leu Lys Asp Trp Lys Arg Ile Gly
       50                  55                  60
   Lys Glu Leu Lys Gln Ala Gly Arg Lys Gly Asn Ile Ile Pro Leu Thr
   65                  70                  75                  80
   Val Trp Asn Asp Trp Ala Ile Ile Lys Ala Ala Leu Glu Pro Phe Gln
                   85                  90                  95
   Thr Glu Glu Asp Ser Val Ser Val Ser Asp Ala Pro Gly Ser Cys Ile
               100                 105                 110
   Ile Asp Cys Asn Glu Asn Thr Arg Lys Lys Ser Gln Lys Glu Thr Glu
           115                 120                 125
   Gly Leu His Cys Glu Tyr Val Ala Glu Pro Val Met Ala Gln Ser Thr
       130                 135                 140
   Gln Asn Val Asp Tyr Asn Gln Leu Gln Glu Val Ile Tyr Pro Glu Thr
   145                 150                 155                 160
   Leu Lys Leu Glu Gly Lys Gly Pro Glu Leu Val Gly Pro Ser Glu Ser
                   165                 170                 175
   Lys Pro Arg Gly Thr Ser Pro Leu Pro Ala Gly Gln Val Pro Val Thr
               180                 185                 190
   Leu Gln Pro Gln Lys Gln Val Lys Glu Asn Lys Thr Gln Pro Pro Val
           195                 200                 205
   Ala Tyr Gln Tyr Trp Pro Pro Ala Glu Leu Gln Tyr Arg Pro Pro Pro
       210                 215                 220
   Glu Ser Gln Tyr Gly Tyr Pro Gly Met Pro Pro Ala Pro Gln Gly Arg
   225                 230                 235                 240
   Ala Pro Tyr Pro Gln Pro Pro Thr Arg Arg Leu Asn Pro Thr Ala Pro
                   245                 250                 255
   Pro Ser Arg Gln Gly Ser Glu Leu His Glu Ile Ile Asp Lys Ser Arg
               260                 265                 270
   Lys Glu Gly Asp Thr Glu Ala Trp Gln Phe Pro Val Thr Leu Glu Pro
           275                 280                 285
   Met Pro Pro Gly Glu Gly Ala Gln Glu Gly Glu Pro Pro Thr Val Glu
       290                 295                 300
   Ala Arg Tyr Lys Ser Phe Ser Ile Lys Met Leu Lys Asp Met Lys Glu
   305                 310                 315                 320
   Gly Val Lys Gln Tyr Gly Pro Asn Ser Pro Tyr Met Arg Thr Leu Leu
                   325                 330                 335
   Asp Ser Ile Ala His Gly His Arg Leu Ile Pro Tyr Asp Trp Glu Ile
               340                 345                 350
   Leu Ala Lys Ser Ser Leu Ser Pro Ser Gln Phe Leu Gln Phe Lys Thr
           355                 360                 365
   Trp Trp Ile Asp Gly Val Gln Glu Gln Val Arg Arg Asn Arg Ala Ala
       370                 375                 380
   Asn Pro Pro Val Asn Ile Asp Ala Asp Gln Leu Leu Gly Ile Gly Gln
   385                 390                 395                 400
   Asn Trp Ser Thr Ile Ser Gln Gln Ala Leu Met Gln Asn Glu Ala Ile
                   405                 410                 415
   Glu Gln Val Arg Ala Ile Cys Leu Arg Ala Trp Glu Lys Ile Gln Asp
               420                 425                 430
   Pro Gly Ser Thr Cys Pro Ser Phe Asn Thr Val Arg Gln Gly Ser Lys
           435                 440                 445
   Glu Pro Tyr Pro Asp Phe Val Ala Arg Leu Gln Asp Val Ala Gln Lys
       450                 455                 460
   Ser Ile Ala Asp Glu Lys Ala Arg Lys Val Ile Val Glu Leu Met Ala
   465 470 475 480
   Tyr Glu Asn Ala Asn Pro Glu Cys Gln Ser Ala Ile Lys Pro Leu Lys
   485 490 495
   Gly Lys Val Pro Ala Gly Ser Asp Val Ile Ser Glu Tyr Val Lys Ala
   500 505 510
   Cys Asp Gly Ile Gly Gly Ala Met His Lys Ala Met Leu Met Ala Gln
   515 520 525
   Ala Ile Thr Gly Val Val Leu Gly Gly Gln Val Arg Thr Phe Gly Gly
   530 535 540
   Lys Cys Tyr Asn Cys Gly Gln Ile Gly His Leu Lys Lys Asn Cys Pro
   545 550 555 560
   Val Leu Asn Lys Gln Asn Ile Thr Ile Gln Ala Thr Thr Thr Gly Arg
   565 570 575
   Glu Pro Pro Asp Leu Cys Pro Arg Cys Lys Lys Gly Lys His Trp Ala
   580 585 590
   Ser Gln Cys Arg Ser Lys Phe Asp Lys Asn Gly Gln Pro Leu Ser Gly
   595 600 605
   Asn Glu Gln Arg Gly Gln Pro Gln Ala Pro Gln Gln Thr Gly Ala Phe
   610 615 620
   Pro Ile Gln Pro Phe Val Pro Gln Gly Phe Gln Gly Gln Gln Pro Pro
   625 630 635 640
   Leu Ser Gln Val Phe Gln Gly Ile Ser Gln Leu Pro Gln Tyr Asn Asn
   645 650 655
   Cys Pro Pro Pro Gln Ala Ala Val Gln Gln
   660 665
<210> 6
   <211> 334
   <212> PRT
   <213> artificial sequence
<220>
   <223> Pro protein encoded by the consensus nucleic sequence of the Phoenix provirus
<400> 6
   Lys Trp Ala Thr Ile Val Gly Lys Arg Ala Lys Gly Pro Ala Ser Gly
   1               5                   10                  15
   Pro Thr Thr Asn Trp Gly Ile Pro Asn Ser Ala Ile Cys Ser Ser Gly
               20                  25                  30
   Phe Ser Gly Thr Thr Thr Pro Thr Val Pro Ser Val Ser Gly Asn Lys
           35                  40                  45
   Pro Val Thr Thr Ile Gln Gln Leu Ser Pro Ala Thr Ser Gly Ser Ala
   50                  55                  60
   Ala Val Asp Leu Cys Thr Ile Gln Ala Val Ser Leu Leu Pro Gly Glu
   65                  70                  75                  80
   Pro Pro Gln Lys Ile Pro Thr Gly Val Tyr Gly Pro Leu Pro Glu Gly
                   85                  90                  95
   Thr Val Gly Leu Ile Leu Gly Arg Ser Ser Leu Asn Leu Lys Gly Val
               100                 105                 110
   Gln Ile His Thr Ser Val Val Asp Ser Asp Tyr Lys Gly Glu Ile Gln
           115                 120                 125
   Leu Val Ile Ser Ser Ser Ile Pro Trp Ser Ala Ser Pro Gly Asp Arg
       130                 135                 140
   Ile Ala Gln Leu Leu Leu Leu Pro Tyr Ile Lys Gly Gly Asn Ser Glu
   145                 150                 155                 160
   Ile Lys Arg Ile Gly Gly Leu Gly Ser Thr Asp Pro Thr Gly Lys Ala
                   165                 170                 175
   Ala Tyr Trp Ala Ser Gln Val Ser Glu Asn Arg Pro Val Cys Lys Ala
               180                 185                 190
   Ile Ile Gln Gly Lys Gln Phe Glu Gly Leu Val Asp Thr Gly Ala Asp
           195                 200                 205
   Val Ser Ile Ile Ala Leu Asn Gln Trp Pro Lys Asn Trp Pro Lys Gln
       210                 215                 220
   Lys Ala Val Thr Gly Leu Val Gly Ile Gly Thr Ala Ser Glu Val Tyr
   225                 230                 235                 240
   Gln Ser Thr Glu Ile Leu His Cys Leu Gly Pro Asp Asn Gln Glu Ser
                   245                 250                 255
   Thr Val Gln Pro Met Ile Thr Ser Ile Pro Leu Asn Leu Trp Gly Arg
               260                 265                 270
   Asp Leu Leu Gln Gln Trp Gly Ala Glu Ile Thr Met Pro Ala Pro Leu
           275                 280                 285
   Tyr Ser Pro Thr Ser Gln Lys Ile Met Thr Lys Met Gly Tyr Ile Pro
       290                 295                 300
   Gly Lys Gly Leu Gly Lys Asn Glu Asp Gly Ile Lys Ile Pro Val Glu
   305                 310                 315                 320
   Ala Lys Ile Asn Gln Lys Arg Glu Gly Ile Gly Tyr Pro Phe
                   325                 330
<210> 7
   <211> 956
   <212> PRT
   <213> artificial sequence
<220>
   <223> Pol protein encoded by the consensus nucleic sequence of the Phoenix provirus
<400> 7
   Asn Lys Ser Lys Lys Arg Arg Asn Arg Val Ser Phe Leu Gly Ala Ala
   1               5                   10                  15
   Thr Val Glu Pro Pro Lys Pro Ile Pro Leu Thr Trp Lys Thr Glu Lys
               20                  25                  30
   Pro Val Trp Val Asn Gln Trp Pro Leu Pro Lys Gln Lys Leu Glu Ala
           35                  40                  45
   Leu His Leu Leu Ala Asn Glu Gln Leu Glu Lys Gly His Ile Glu Pro
       50                  55                  60
   Ser Phe Ser Pro Trp Asn Ser Pro Val Phe Val Ile Gln Lys Lys Ser
   65                  70                  75                  80
   Gly Lys Trp Arg Met Leu Thr Asp Leu Arg Ala Val Asn Ala Val Ile
                   85                  90                  95
   Gln Pro Met Gly Pro Leu Gln Pro Gly Leu Pro Ser Pro Ala Met Ile
               100                 105                 110
   Pro Lys Asp Trp Pro Leu Ile Ile Ile Asp Leu Lys Asp Cys Phe Phe
           115                 120                 125
   Thr Ile Pro Leu Ala Glu Gln Asp Cys Glu Lys Phe Ala Phe Thr Ile
       130                 135                 140
   Pro Ala Ile Asn Asn Lys Glu Pro Ala Thr Arg Phe Gln Trp Lys Val
   145                 150                 155                 160
   Leu Pro Gln Gly Met Leu Asn Ser Pro Thr Ile Cys Gln Thr Phe Val
                   165                 170                 175
   Gly Arg Ala Leu Gln Pro Val Arg Glu Lys Phe Ser Asp Cys Tyr Ile
               180                 185                 190
   Ile His Tyr Ile Asp Asp Ile Leu Cys Ala Ala Glu Thr Lys Asp Lys
           195                 200                 205
   Leu Ile Asp Cys Tyr Thr Phe Leu Gln Ala Glu Val Ala Asn Ala Gly
       210                 215                 220
   Leu Ala Ile Ala Ser Asp Lys Ile Gln Thr Ser Thr Pro Phe His Tyr
   225                 230                 235                 240
   Leu Gly Met Gln Ile Glu Asn Arg Lys Ile Lys Pro Gln Lys Ile Glu
                   245                 250                 255
   Ile Arg Lys Asp Thr Leu Lys Thr Leu Asn Asp Phe Gln Lys Leu Leu
               260                 265                 270
   Gly Asp Ile Asn Trp Ile Arg Pro Thr Leu Gly Ile Pro Thr Tyr Ala
           275                 280                 285
   Met Ser Asn Leu Phe Ser Ile Leu Arg Gly Asp Ser Asp Leu Asn Ser
       290                 295                 300
   Lys Arg Met Leu Thr Pro Glu Ala Thr Lys Glu Ile Lys Leu Val Glu
   305                 310                 315                 320
   Glu Lys Ile Gln Ser Ala Gln Ile Asn Arg Ile Asp Pro Leu Ala Pro
                   325                 330                 335
   Leu Gln Leu Leu Ile Phe Ala Thr Ala His Ser Pro Thr Gly Ile Ile
               340                 345                 350
   Ile Gln Asn Thr Asp Leu Val Glu Trp Ser Phe Leu Pro His Ser Thr
           355                 360                 365
   Val Lys Thr Phe Thr Leu Tyr Leu Asp Gln Ile Ala Thr Leu Ile Gly
       370                 375                 380
   Gln Thr Arg Leu Arg Ile Ile Lys Leu Cys Gly Asn Asp Pro Asp Lys
   385                 390                 395                 400
   Ile Val Val Pro Leu Thr Lys Glu Gln Val Arg Gln Ala Phe Ile Asn
                   405                 410                 415
   Ser Gly Ala Trp Gln Ile Gly Leu Ala Asn Phe Val Gly Ile Ile Asp
               420                 425                 430
   Asn His Tyr Pro Lys Thr Lys Ile Phe Gln Phe Leu Lys Leu Thr Thr
           435                 440                 445
   Trp Ile Leu Pro Lys Ile Thr Arg Arg Glu Pro Leu Glu Asn Ala Leu
       450                 455                 460
   Thr Val Phe Thr Asp Gly Ser Ser Asn Gly Lys Ala Ala Tyr Thr Gly
   465                 470                 475                 480
   Pro Lys Glu Arg Val Ile Lys Thr Pro Tyr Gln Ser Ala Gln Arg Ala
                   485                 490                 495
   Glu Leu Val Ala Val Ile Thr Val Leu Gln Asp Phe Asp Gln Pro Ile
               500                 505                 510
   Asn Ile Ile Ser Asp Ser Ala Tyr Val Val Gln Ala Thr Arg Asp Val
           515                 520                 525
   Glu Thr Ala Leu Ile Lys Tyr Ser Met Asp Asp Gln Leu Asn Gln Leu
       530                 535                 540
   Phe Asn Leu Leu Gln Gln Thr Val Arg Lys Arg Asn Phe Pro Phe Tyr
   545                 550                 555                 560
   Ile Thr His Ile Arg Ala His Thr Asn Leu Pro Gly Pro Leu Thr Lys
                   565                 570                 575
   Ala Asn Glu Gln Ala Asp Leu Leu Val Ser Ser Ala Leu Ile Lys Ala
               580                 585                 590
   Gln Glu Leu His Ala Leu Thr His Val Asn Ala Ala Gly Leu Lys Asn
           595                 600                 605
   Lys Phe Asp Val Thr Trp Lys Gln Ala Lys Asp Ile Val Gln His Cys
       610                 615                 620
   Thr Gln Cys Gln Val Leu His Leu Pro Thr Gln Glu Ala Gly Val Asn
   625                 630                 635                 640
   Pro Arg Gly Leu Cys Pro Asn Ala Leu Trp Gln Met Asp Val Thr His
                   645                 650                 655
   Val Pro Ser Phe Gly Arg Leu Ser Tyr Val His Val Thr Val Asp Thr
               660                 665                 670
   Tyr Ser His Phe Ile Trp Ala Thr Cys Gln Thr Gly Glu Ser Thr Ser
           675                 680                 685
   His Val Lys Lys His Leu Leu Ser Cys Phe Ala Val Met Gly Val Pro
       690                 695                 700
   Glu Lys Ile Lys Thr Asp Asn Gly Pro Gly Tyr Cys Ser Lys Ala Phe
   705                 710                 715                 720
   Gln Lys Phe Leu Ser Gln Trp Lys Ile Ser His Thr Thr Gly Ile Pro
                   725                 730                 735
   Tyr Asn Ser Gln Gly Gln Ala Ile Val Glu Arg Thr Asn Arg Thr Leu
               740                 745                 750
   Lys Thr Gln Leu Val Lys Gln Lys Glu Gly Gly Asp Ser Lys Glu Cys
           755                 760                 765
   Thr Thr Pro Gln Met Gln Leu Asn Leu Ala Leu Tyr Thr Leu Asn Phe
       770                 775                 780
   Leu Asn Ile Tyr Arg Asn Gln Thr Thr Thr Ser Ala Glu Gln His Leu
   785                 790                 795                 800
   Thr Gly Lys Lys Asn Ser Pro His Glu Gly Lys Leu Ile Trp Trp Lys
                   805                 810                 815
   Asp Asn Lys Asn Lys Thr Trp Glu Ile Gly Lys Val Ile Thr Trp Gly
               820                 825                 830
   Arg Gly Phe Ala Cys Val Ser Pro Gly Glu Asn Gln Leu Pro Val Trp
           835                 840                 845
   Ile Pro Thr Arg His Leu Lys Phe Tyr Asn Glu Pro Ile Arg Asp Ala
       850                 855                 860
   Lys Lys Ser Thr Ser Ala Glu Thr Glu Thr Pro Gln Ser Ser Thr Val
   865                 870                 875                 880
   Asp Ser Gln Asp Glu Gln Asn Gly Asp Val Arg Arg Thr Asp Glu Val
                   885                 890                 895
   Ala Ile His Gln Glu Gly Arg Ala Ala Asp Leu Gly Thr Thr Lys Glu
               900                 905                 910
   Ala Asp Ala Val Ser Tyr Lys Ile Ser Arg Glu His Lys Gly Asp Thr
           915                 920                 925
   Asn Pro Arg Glu Tyr Ala Ala Cys Ser Leu Asp Asp Cys Ile Asn Gly
       930                 935                 940
   Gly Lys Ser Pro Tyr Ala Cys Arg Ser Ser Cys Ser
   945                 950                 955
<210> 8
   <211> 699
   <212> PRT
   <213> artificial sequence
<220>
   <223> Env protein encoded by the consensus nucleic sequence of the Phoenix provirus
<400> 8
   Met Asn Pro Ser Glu Met Gln Arg Lys Ala Pro Pro Arg Arg Arg Arg
   1               5                   10                  15
   His Arg Asn Arg Ala Pro Leu Thr His Lys Met Asn Lys Met Val Thr
               20                  25                  30
   Ser Glu Glu Gln Met Lys Leu Pro Ser Thr Lys Lys Ala Glu Pro Pro
           35                  40                  45
   Thr Trp Ala Gln Leu Lys Lys Leu Thr Gln Leu Ala Thr Lys Tyr Leu
       50                  55                  60
   Glu Asn Thr Lys Val Thr Gln Thr Pro Glu Ser Met Leu Leu Ala Ala
   65                  70                  75                  80
   Leu Met Ile Val Ser Met Val Val Ser Leu Pro Met Pro Ala Gly Ala
                   85                  90                  95
   Ala Ala Ala Asn Tyr Thr Tyr Trp Ala Tyr Val Pro Phe Pro Pro Leu
               100                 105                 110
   Ile Arg Ala Val Thr Trp Met Asp Asn Pro Ile Glu Val Tyr Val Asn
           115                 120                 125
   Asp Ser Val Trp Val Pro Gly Pro Ile Asp Asp Arg Cys Pro Ala Lys
       130                 135                 140
   Pro Glu Glu Glu Gly Met Met Ile Asn Ile Ser Ile Gly Tyr Arg Tyr
   145                 150                 155                 160
   Pro Pro Ile Cys Leu Gly Arg Ala Pro Gly Cys Leu Met Pro Ala Val
                   165                 170                 175
   Gln Asn Trp Leu Val Glu Val Pro Thr Val Ser Pro Ile Ser Arg Phe
               180                 185                 190
   Thr Tyr His Met Val Ser Gly Met Ser Leu Arg Pro Arg Val Asn Tyr
           195                 200                 205
   Leu Gln Asp Phe Ser Tyr Gln Arg Ser Leu Lys Phe Arg Pro Lys Gly
       210                 215                 220
   Lys Pro Cys Pro Lys Glu Ile Pro Lys Glu Ser Lys Asn Thr Glu Val
   225                 230                 235                 240
   Leu Val Trp Glu Glu Cys Val Ala Asn Ser Ala Val Ile Leu Gln Asn
                   245                 250                 255
   Asn Glu Phe Gly Thr Ile Ile Asp Trp Ala Pro Arg Gly Gln Phe Tyr
   260 265 270
   His Asn Cys Ser Gly Gln Thr Gln Ser Cys Pro Ser Ala Gln Val Ser
   275 280 285
   Pro Ala Val Asp Ser Asp Leu Thr Glu Ser Leu Asp Lys His Lys His
   290 295 300
   Lys Lys Leu Gln Ser Phe Tyr Pro Trp Glu Trp Gly Glu Lys Gly Ile
   305 310 315 320
   Ser Thr Pro Arg Pro Lys Ile Ile Ser Pro Val Ser Gly Pro Glu His
   325 330 335
   Pro Glu Leu Trp Arg Leu Thr Val Ala Ser His His Ile Arg Ile Trp
   340 345 350
   Ser Gly Asn Gln Thr Leu Glu Thr Arg Asp Arg Lys Pro Phe Tyr Thr
   355 360 365
   Val Asp Leu Asn Ser Ser Leu Thr Val Pro Leu Gln Ser Cys Val Lys
   370 375 380
   Pro Pro Tyr Met Leu Val Val Gly Asn Ile Val Ile Lys Pro Asp Ser
   385 390 395 400
   Gln Thr Ile Thr Cys Glu Asn Cys Arg Leu Leu Thr Cys Ile Asp Ser
   405 410 415
   Thr Phe Asn Trp Gln His Arg Ile Leu Leu Val Arg Ala Arg Glu Gly
   420 425 430
   Val Trp Ile Pro Val Ser Met Asp Arg Pro Trp Glu Ala Ser Pro Ser
   435 440 445
   Ile His Ile Leu Thr Glu Val Leu Lys Gly Val Leu Asn Arg Ser Lys
   450 455 460
   Arg Phe Ile Phe Thr Leu Ile Ala Val Ile Met Gly Leu Ile Ala Val
   465 470 475 480
   Thr Ala Thr Ala Ala Val Ala Gly Val Ala Leu His Ser Ser Val Gln
   485 490 495
   Ser Val Asn Phe Val Asn Asp Trp Gln Lys Asn Ser Thr Arg Leu Trp
   500 505 510
   Asn Ser Gln Ser Ser Ile Asp Gln Lys Leu Ala Asn Gln Ile Asn Asp
   515 520 525
   Leu Arg Gln Thr Val Ile Trp Met Gly Asp Arg Leu Met Ser Leu Glu
   530 535 540
   His Arg Phe Gln Leu Gln Cys Asp Trp Asn Thr Ser Asp Phe Cys Ile
   545 550 555 560
   Thr Pro Gln Ile Tyr Asn Glu Ser Glu His His Trp Asp Met Val Arg
   565 570 575
   Arg His Leu Gln Gly Arg Glu Asp Asn Leu Thr Leu Asp Ile Ser Lys
   580 585 590
   Leu Lys Glu Gln Ile Phe Glu Ala Ser Lys Ala His Leu Asn Leu Val
   595 600 605
   Pro Gly Thr Glu Ala Ile Ala Gly Val Ala Asp Gly Leu Ala Asn Leu
   610 615 620
   Asn Pro Val Thr Trp Val Lys Thr Ile Gly Ser Thr Thr Ile Ile Asn
   625 630 635 640
   Leu Ile Leu Ile Leu Val Cys Leu Phe Cys Leu Leu Leu Val Cys Arg
   645 650 655
   Cys Thr Gln Gln Leu Arg Arg Asp Ser Asp His Arg Glu Arg Ala Met
   660 665 670
   Met Thr Met Ala Val Leu Ser Lys Arg Lys Gly Gly Asn Val Gly Lys
   675 680 685
   Ser Lys Arg Asp Gln Ile Val Thr Val Ser Val
   690 695
<210> 9
   <211> 105
   <212> PRT
   <213> artificial sequence
<220>
   <223> Rec protein encoded by the consensus nucleic sequence of the Phoenix provirus
<400> 9
   Met Asn Pro Ser Glu Met Gln Arg Lys Ala Pro Pro Arg Arg Arg Arg
   1 5 10 15
   His Arg Asn Arg Ala Pro Leu Thr His Lys Met Asn Lys Met Val Thr
   20 25 30
   Ser Glu Glu Gln Met Lys Leu Pro Ser Thr Lys Lys Ala Glu Pro Pro
   35 40 45
   Thr Trp Ala Gln Leu Lys Lys Leu Thr Gln Leu Ala Thr Lys Tyr Leu
   50 55 60
   Glu Asn Thr Lys Val Thr Gln Thr Pro Glu Ser Met Leu Leu Ala Ala
   65 70 75 80
   Leu Met Ile Val Ser Met Val Ser Ala Gly Val Pro Asn Ser Ser Glu
   85 90 95
   Glu Thr Ala Thr Ile Glu Asn Gly Pro
   100 105